(19) **Europäisches Patentamt**
**European Patent Office**
**Office européen des brevets**

(11) **EP 4 541 902 A1**

(12) **EUROPEAN PATENT APPLICATION**
published in accordance with Art. 153(4) EPC

(43) Date of publication:
**23.04.2025 Bulletin 2025/17**

(21) Application number: **23831594.9**

(22) Date of filing: **29.06.2023**

(51) International Patent Classification (IPC):
*C12P 13/00* (2006.01)      *C12N 5/10* (2006.01)
*C12N 1/15* (2006.01)       *C12N 1/19* (2006.01)
*C12N 1/21* (2006.01)       *C12N 15/53* (2006.01)
*C12N 15/54* (2006.01)      *C12N 9/10* (2006.01)

(52) Cooperative Patent Classification (CPC):
**C12N 5/10; C12N 9/0004; C12N 9/10; C12N 15/74;
C12N 15/80; C12N 15/81; C12P 13/00**

(86) International application number:
**PCT/JP2023/024236**

(87) International publication number:
**WO 2024/005155 (04.01.2024 Gazette 2024/01)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **30.06.2022 JP 2022106294**

(71) Applicants:
• **KIRIN HOLDINGS KABUSHIKI KAISHA
Nakano-ku, Tokyo 164-0001 (JP)**
• **Kyowa Hakko Bio Co., Ltd.
Tokyo 100-8185 (JP)**

(72) Inventors:
• **GODA, Asuka
Tokyo 164-0001 (JP)**

• **YAMASHITA, Makoto
Tokyo 164-0001 (JP)**
• **KOUKETSU, Kento
Tokyo 164-0001 (JP)**
• **AOKI YAMAMOTO, Yuriko
Tokyo 100-8185 (JP)**
• **WATANABE, Taro
Tokyo 164-0001 (JP)**

(74) Representative: **Vossius & Partner
Patentanwälte Rechtsanwälte mbB
Siebertstrasse 3
81675 München (DE)**

Remarks:
The complete document including Reference
Table(s) and the Sequence Listing(s) can be
downloaded from the EPO website

(54) **METHOD FOR PRODUCING 4-(AMINOMETHYL)CYCLOHEXANE-1-CARBOXYLIC ACID**

(57)   A production method according to one embodiment is a method for producing 4-(aminomethyl)cyclohexane-1-carboxylic acid from 1,4-bis(aminomethyl)cyclohexane using a protein consisting of an amino acid sequence having 60% or more identity to an amino acid sequence shown in any one of SEQ ID NOs. 1 to 4 and 103 to 105 and having transamination activity, and a protein consisting of an amino acid sequence having 50% or more identity to an amino acid sequence shown in any one of SEQ ID NOs. 19 to 22, 35 to 46 and 127 to 139 and having aldehyde dehydrogenase activity.

**EP 4 541 902 A1**

## Description

### Technical Field

The present invention relates to a protein used in the production of 4-(aminomethyl)cyclohexane-1-carboxylic acid and a method for producing 4-(aminomethyl)cyclohexane-1-carboxylic acid using the protein. In addition, the present invention relates to a method for efficiently producing trans-4-(aminomethyl)cyclohexane-1-carboxylic acid.

### Background Art

4-(aminomethyl)cyclohexane-1-carboxylic acid (AMCHA) is an artificially synthesized amino acid developed as an antiplasmin agent (Non-Patent Literature 1). AMCHA has cis- and trans-stereoisomers. Among these, it is confirmed in Non-Patent Literature 2 that the trans-form has an antiplasmin effect. Trans-4-(aminomethyl)cyclohexane-1-carboxylic acid (t-AMCHA), which is a trans-form of 4-(aminomethyl)cyclohexane-1-carboxylic acid, is also called tranexamic acid (TXA), and is used as a hemostatic agent and an anti-inflammatory agent in order to prevent and treat bleeding due to its antiplasmin effect (Non-Patent Literature 3 and 4).

As methods for producing 4-(aminomethyl)cyclohexane-1-carboxylic acid, chemical synthesis methods are mainly known. For example, Patent Literature 1 discloses a method in which 4-(chloromethyl)benzoic acid is used as a starting substrate, 4-(aminomethyl)cyclohexane-1-carboxylic acid is produced through an amination reaction and a hydrogenation reaction, a three-step chemical synthesis reaction for an isomerization reaction is then performed, and trans-4-(aminomethyl)cyclohexane-1-carboxylic acid is produced.

Chemical synthesis methods, including the above method, require a reaction at a high temperature and a high pressure, and have problems of high energy-costs and a large environmental load.

As an alternative to chemical synthesis methods, a production method using microorganisms may be exemplified. Patent Literature 2 discloses a method for producing trans-4-(aminomethyl)cyclohexane-1-carboxylic acid by bringing a mixture of cis- and trans-1,4-bis(aminomethyl)-cyclohexane into contact with microorganisms belonging to the genus *Corynebacterium* or *Nocardia.* However, since this method uses a conversion reaction using microorganisms themselves isolated from nature, the productivity of 4-(aminomethyl)cyclohexane-1-carboxylic acid is low, and the active enzyme has not been identified.

Citation List

### Patent Literature

[Patent Literature 1] CN114014768A
[Patent Literature 2] Japanese Unexamined Patent Publication No. S63-152991

### Non-Patent Literature

[Non-Patent Literature 1] Keio Journal of Medicine (1962)11, 8, 105-115
[Non-Patent Literature 2] Keio Journal of Medicine (1964) 18, 4, 177-185
[Non-Patent Literature 3] European Journal of Haematology (2020) 104, 2, 79-87
[Non-Patent Literature 4] Journal of Trauma and Acute Care Surgery (2019) 86, 1, 101-107

### Summary of Invention

### Technical Problem

An object of the present invention is to provide a method for producing 4-(aminomethyl)cyclohexane-1-carboxylic acid based on an enzyme reaction using 1,4-bis(aminomethyl)cyclohexane as a substrate and a protein derived from microorganisms that can be used in the enzyme reaction.

**Solution to Problem**

So far, no reaction pathway through which 4-(aminomethyl)cyclohexane-1-carboxylic acid is produced from 1,4-bis(aminomethyl)cyclohexane is known. Therefore, the inventors assumed a two-step enzyme reaction pathway involving an aminotransferase and an aldehyde dehydrogenase, and selected proteins that may have desired activity. The inventors found that, when the protein is expressed in an *E. coli* strain and purified, and an enzyme reaction is performed, it is possible to produce 4-(aminomethyl)cyclohexane-1-carboxylic acid from 1,4-bis(aminomethyl)cyclohexane, thereby leading to the completion of the present invention.

The present invention relates to, for example, the following inventions.

[1] A protein having transamination activity of transferring an amino group of 1,4-bis(aminomethyl)cyclohexane to another compound to produce 4-(aminomethyl)cyclohexane-1-carbaldehyde and consisting of an amino acid sequence having 60% or more identity to an amino acid sequence shown in any one of SEQ ID NOs. 1 to 4 and 103 to 105.

[2] DNA encoding the protein according to [1].

[3] Recombinant DNA comprising the DNA according to [2].

[4] A recombinant cell comprising the DNA according to [2] or obtained by transforming a host cell with the recombinant DNA according to [3].

[5] A protein having aldehyde dehydrogenase activity of oxidizing an aldehyde group of 4-(aminomethyl)cyclohexane-1-carbaldehyde to produce 4-(aminomethyl)cyclohexane-1-carboxylic acid and consisting of an amino acid sequence having 50% or more identity to an amino acid sequence shown in any one of SEQ ID NOs. 19 to 22, 35 to 46 and 127 to 139.

[6] A protein having aldehyde dehydrogenase activity of oxidizing an aldehyde group of 4-(aminomethyl)cyclohexane-1-carbaldehyde to produce 4-(aminomethyl)cyclohexane-1-carboxylic acid and consisting of an amino acid sequence having 60% or more identity to an amino acid sequence shown in any one of SEQ ID NOs. 19 to 22, 35 to 46 and 127 to 139.

[7] DNA encoding the protein according to [5] or [6].

[8] Recombinant DNA comprising the DNA according to [7]

[9] A recombinant cell comprising the DNA according to [7] or obtained by transforming a host cell with the recombinant DNA according to [8].

[10] A method for producing cis- and/or trans- 4-(aminomethyl)cyclohexane-1-carboxylic acid from cis- and/or trans-1,4-bis(aminomethyl)cyclohexane, the production method comprising:

(i) a step of producing 4-(aminomethyl)cyclohexane-1-carbaldehyde from 1,4-bis(aminomethyl)cyclohexane in the presence of an aminotransferase, and
(ii) a step of producing 4-(aminomethyl)cyclohexane-1-carboxylic acid from 4-(aminomethyl)cyclohexane-1-carbaldehyde in the presence of an aldehyde dehydrogenase.

[11] The production method according to [10],
wherein the aminotransferase is the protein according to [1] and the aldehyde dehydrogenase is the protein according to [5] or [6].

[12] The production method according to [10] or [11],
wherein, in a part or all of the method, alanine dehydrogenase and/or NADH oxidase is allowed to coexist.

[13] The production method according to any one of [10] to [12],
wherein trans-4-(aminomethyl)cyclohexane-1-carboxylic acid is produced from cis- and/or trans- 1,4-bis(aminomethyl)cyclohexane.

[14] The production method according to any one of [10] to [12],
wherein cis-4-(aminomethyl)cyclohexane-1-carboxylic acid is produced from cis- and/or trans- 1,4-bis(aminomethyl)cyclohexane.

[15] The production method according to any one of [10] to [13],
wherein a part or all of the method is performed under neutral or basic conditions.

[16] The production method according to [15],
wherein a part or all of the method is performed in the presence of a secondary amine.

[17] A protein which is an aldehyde dehydrogenase and has aldehyde dehydrogenase activity of oxidizing an aldehyde group of 4-(aminomethyl)cyclohexane-1-carbaldehyde to produce 4-(aminomethyl)cyclohexane-1-carboxylic acid.

[18] The protein according to [17], wherein the protein is a benzaldehyde dehydrogenase and has aldehyde dehydrogenase activity of oxidizing an aldehyde group of 4-(aminomethyl)cyclohexane-1-carbaldehyde to produce 4-(aminomethyl)cyclohexane-1-carboxylic acid.

[19] The protein according to [17] or [18],

wherein the aldehyde dehydrogenase is an aldehyde dehydrogenase derived from bacteria belonging to the genus *Pseudomonas*.

[20] The protein according to [17] or [18], wherein the protein is consisting of an amino acid sequence having 50% or more identity to an amino acid sequence shown in any one of SEQ ID NOs. 19 to 22, 35 to 46 and 127 to 139.

[21] A protein having aldehyde dehydrogenase activity of producing 4-(aminomethyl)cyclohexane-1-carboxylic acid from 4-(aminomethyl)cyclohexane-1-carbaldehyde and consisting of an amino acid sequence having 50% or more identity to an amino acid sequence shown in SEQ ID NO. 20, wherein the amino acid sequence contains at least one of the following amino acid residues (1) to (26) when aligned with the amino acid sequence shown in SEQ ID NO. 20:

(1) an amino acid residue at a position corresponding to position 35 which is a glycine residue (G)
(2) an amino acid residue at a position corresponding to position 65 which is an alanine residue (A)
(3) an amino acid residue at a position corresponding to position 82 which is a leucine residue (L)
(4) an amino acid residue at a position corresponding to position 104 which is a glycine residue (G)
(5) an amino acid residue at a position corresponding to position 109 which is a glycine residue (G)
(6) an amino acid residue at a position corresponding to position 110 which is an isoleucine residue (I)
(7) an amino acid residue at a position corresponding to position 117 which is a glutamine residue (Q)
(8) an amino acid residue at a position corresponding to position 122 which is an alanine residue (A)
(9) an amino acid residue at a position corresponding to position 124 which is a leucine residue (L)
(10) an amino acid residue at a position corresponding to position 134 which is a valine residue (V)
(11) an amino acid residue at a position corresponding to position 144 which is a valine residue (V)
(12) an amino acid residue at a position corresponding to position 160 which is a phenylalanine residue (F)
(13) an amino acid residue at a position corresponding to position 271 which is a glycine residue (G)
(14) an amino acid residue at a position corresponding to position 290 which is an alanine residue (A)
(15) an amino acid residue at a position corresponding to position 319 which is an aspartic acid residue (D)
(16) an amino acid residue at a position corresponding to position 333 which is an aspartic acid residue (D)
(17) an amino acid residue at a position corresponding to position 365 which is a glutamine residue (Q)
(18) an amino acid residue at a position corresponding to position 390 which is an isoleucine residue (I)
(19) an amino acid residue at a position corresponding to position 420 which is an alanine residue (A)
(20) an amino acid residue at a position corresponding to position 423 which is a leucine residue (L)
(21) an amino acid residue at a position corresponding to position 434 which is a valine residue (V)
(22) an amino acid residue at a position corresponding to position 440 which is a proline residue (P)
(23) an amino acid residue at a position corresponding to position 443 which is a cysteine residue (C)
(24) an amino acid residue at a position corresponding to position 445 which is a proline residue (P)
(25) an amino acid residue at a position corresponding to position 466 which is a serine residue (S)
(26) an amino acid residue at a position corresponding to position 467 which is an isoleucine residue (I)

[22] DNA encoding the protein according to any one of [17] to [21].

[23] Recombinant DNA comprising the DNA according to [22].

[24] A recombinant cell comprising the DNA according to [22] or obtained by transforming a host cell with the recombinant DNA according to [23].

[25] The production method according to [10],

wherein the aminotransferase is the protein according to [1], and the aldehyde dehydrogenase is the protein according to any one of [17] to [21].

[26] The production method according to [25],

wherein, in a part or all of the method, alanine dehydrogenase and/or NADH oxidase is allowed to coexist.

[27] The production method according to [25] or [26],

wherein trans-4-(aminomethyl)cyclohexane-1-carboxylic acid is produced from cis- and/or trans- 1,4-bis(aminomethyl)cyclohexane.

[28] The production method according to [25] or [26],

wherein cis-4-(aminomethyl)cyclohexane-1-carboxylic acid is produced from cis- and/or trans- 1,4-bis(aminomethyl) cyclohexane.

[29] The production method according to any one of [25] to [28],

wherein a part or all of the method is performed under neutral or basic conditions.

[30] The production method according to any one of [25] to [28],

wherein a part or all of the method is performed in the presence of a secondary amine.

**Advantageous Effects of Invention**

According to the protein having transamination activity and the protein having aldehyde dehydrogenase activity of the present invention, 4-(aminomethyl)cyclohexane-1-carboxylic acid can be produced using 1,4-bis(aminomethyl) cyclohexane substrate. In addition, when substrate specificity of the protein having aldehyde dehydrogenase activity is used according to the three-dimensional structure of desired 4-(aminomethyl)cyclohexane-1-carboxylic acid, even if a mixture of a cis-form and a trans-form is used as a substrate, it is possible to selectively produce a large amount of a compound with a desired structure.

According to the production method of the present invention, it is possible to efficiently produce 4-(aminomethyl) cyclohexane-1-carboxylic acid while reducing production costs without requiring a high temperature and a high pressure. In addition, when a keto acid and/or a coenzyme or a regeneration system thereof is introduced into the reaction system, the enzyme reaction can be performed more efficiently and the productivity of 4-(aminomethyl)cyclohexane-1-carboxylic acid can be improved. In addition, when a mixture of cis- and trans-1,4-bis(aminomethyl)cyclohexane is used as a substrate, if the aldehyde dehydrogenase of the present invention which selectively acts on trans-4-(aminomethyl) cyclohexane-1-carbaldehyde is used and the reaction is performed under neutral or basic conditions, the isomerization reaction of the intermediate 4-(aminomethyl)cyclohexane-1-carbaldehyde from a cis-form to a trans-form is promoted, and thus it is possible to produce trans-4-(aminomethyl)cyclohexane-carboxylic acid (tranexamic acid) at a high yield and with high selectivity according to a dynamic kinetic resolution.

**Brief Description of Drawings**

FIG. 1 shows a scheme for producing cis- or trans-4-(aminomethyl)cyclohexane-1-carbaldehyde and cis- or trans-4-(aminomethyl)cyclohexane-1-carboxylic acid from cis- or trans-1,4-bis(aminomethyl)cyclohexane in one embodiment.
FIG. 2 is a schematic diagram showing a method for constructing an aminotransferase (AT) expression plasmid (pQE80L-PpAT8) in Example 1.
FIG. 3 is an LC-MS chromatogram (EIC:142.10 m/z) of a PatA enzyme reaction solution in Example 1.
FIG. 4 is a schematic diagram showing a method for constructing an AT and aldehyde dehydrogenase (ALDH) co-expression plasmid (pET28a-PatA-XylC) in Example 7.
FIG. 5A and FIG. 5B are diagrams showing the results of alignment of amino acid sequences of an aldehyde dehydrogenase in Example 11.

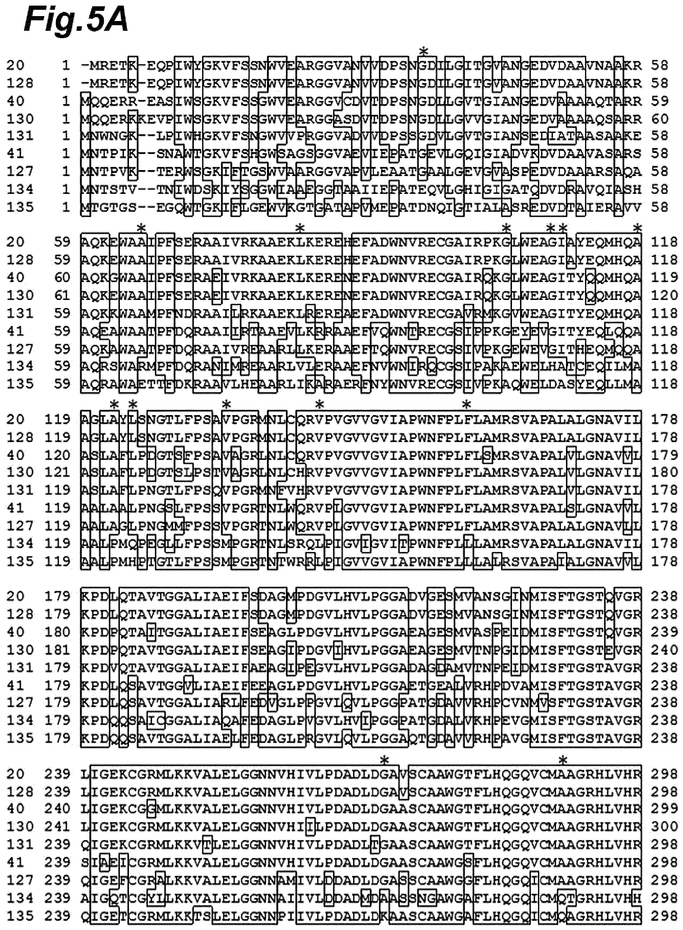

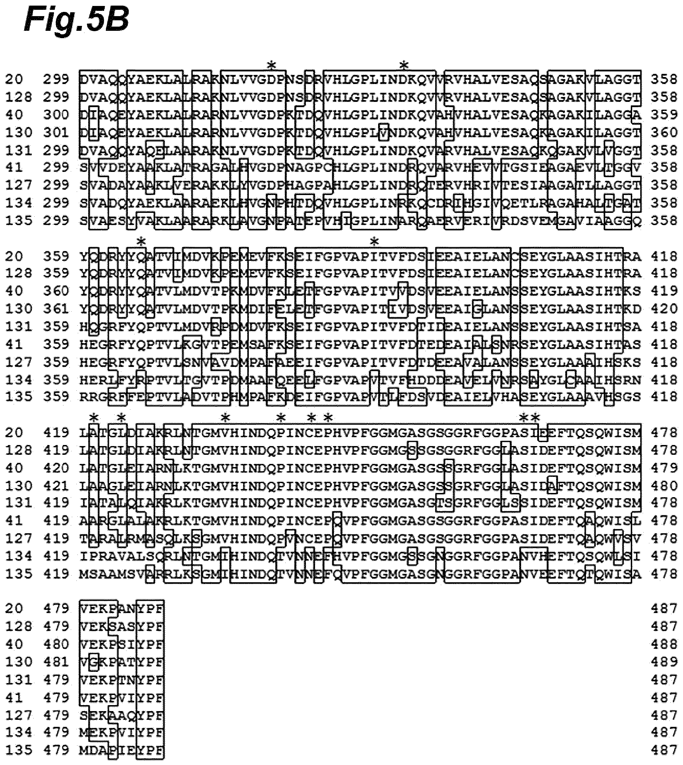

FIG. 5A and FIG. 5B are diagrams showing the results of alignment of amino acid sequences of an aldehyde dehydrogenase in Example 11.

**Description of Embodiments**

1. Compound and enzyme reaction

The target compound in the present invention, 4-(aminomethyl)cyclohexane-1-carboxylic acid (hereinafter referred to as "AMCHA" in some cases), is a non-natural amino acid. 4-(Aminomethyl)cyclohexane-1-carboxylic acid includes cis-4-(aminomethyl)cyclohexane-1-carboxylic acid, which is a cis-form, and trans-4-(aminomethyl)cyclohexane-1-carboxylic acid, which is a trans-form. Among these, trans-4-(aminomethyl)cyclohexane-1-carboxylic acid is also called tranexamic acid (hereinafter referred to as "TXA" in some cases). Unless otherwise specified herein, "tranexamic acid" or "TXA" indicates trans-4-(aminomethyl)cyclohexane-1-carboxylic acid and cis-4-(aminomethyl)cyclohexane-1-carboxylic acid is referred to as cis-tranexamic acid or cis-TXA.

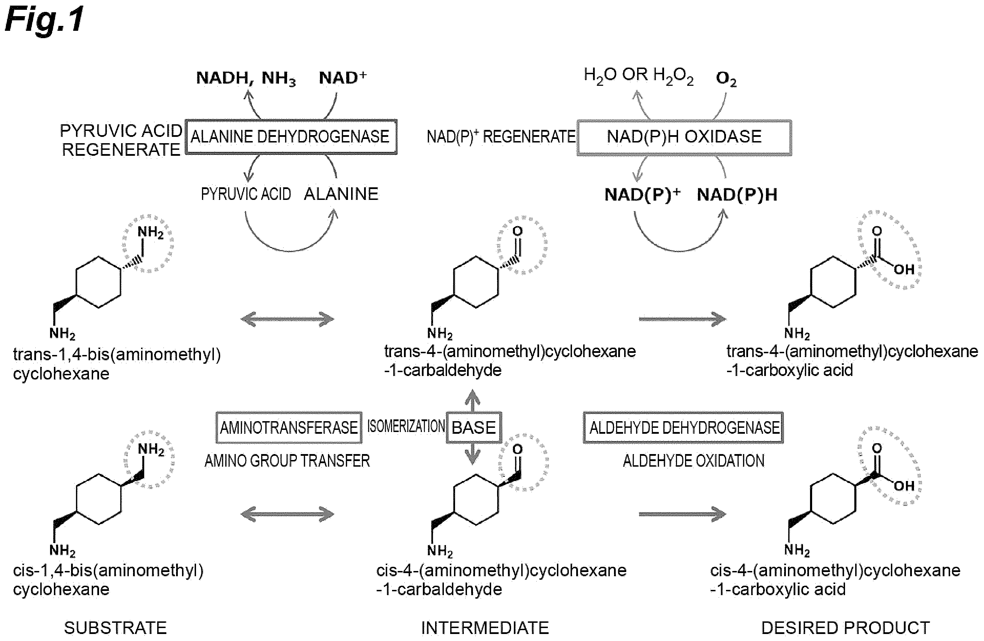

The enzyme reaction in the present invention includes a two-step reaction as shown below. In a first step (step (i)), the substrate compound 1,4-bis(aminomethyl)cyclohexane reacts with a keto acid (for example, pyruvic acid), which is a compound that can provide a carbonyl group, and is converted into the intermediate 4-(aminomethyl)cyclohexane-1-carbaldehyde and a compound that accepts an amino group (alanine when pyruvic acid is used as a keto acid) using an aminotransferase (AT). In a second step (step (ii)), the intermediate 4-(aminomethyl)cyclohexane-1-carbaldehyde is converted into 4-(aminomethyl)cyclohexane-1-carboxylic acid and NAD(P)H using an aldehyde dehydrogenase (ALDH) that uses NAD(P)$^+$ as a coenzyme (FIG. 1).

In addition, in the first step, when pyruvic acid is used as a compound for providing a carbonyl group, the produced alanine can be converted (regenerated) into pyruvic acid using an alanine dehydrogenase, and in the second step, the produced NAD(P)H can be converted (regenerated) into NAD(P)$^+$ using NAD(P)H oxidase. Alternatively, NAD(P)$^+$ can be regenerated by electrically oxidizing NAD(P)H (FIG. 1).

The substrate compound 1,4-bis(aminomethyl)cyclohexane may be a mixture of a cis-form and a trans-form, a trans-form or a cis-form. The intermediate 4-(aminomethyl)cyclohexane-1-carbaldehyde may also be a mixture of a cis-form and a trans-form, a trans-form or a cis-form.

2. Protein and DNA of present invention

(1) Aminotransferase of present invention and DNA encoding it

"Aminotransferase" (expressed as "AT" or "transaminase") is a general term for enzymes that catalyze the reaction between amino acids and $\alpha$-keto acids in biochemistry. In this specification, transamination activity refers to activity of catalyzing a reaction in which an amino group is transferred from a compound having an amino group to another compound and converted into a carbonyl group.

The protein having transamination activity of the present invention is a protein having activity of transferring an amino group of 1,4-bis(aminomethyl)cyclohexane to another compound to produce 4-(aminomethyl)cyclohexane-1-carbaldehyde. That is, it is a protein that has activity of mediating a reaction from 1,4-bis(aminomethyl)cyclohexane to 4-(aminomethyl)cyclohexane-1-carbaldehyde, specifically, a protein that has activity of removing an amino group from 1,4-bis(aminomethyl)cyclohexane to produce 4-(aminomethyl)cyclohexane-1-carbaldehyde. In this specification, these proteins are collectively referred to as an "aminotransferase of the present invention" or "AT of the present invention."

The AT of one embodiment is, for example, a protein consisting of an amino acid sequence having 60% or more (preferably, 70% or more, 75% or more, 80% or more, 90% or more, 93% or more, 95% or more, or 98% or more) identity to an amino acid sequence shown in any one of SEQ ID NOs. 1 to 4 and 103 to 105 and having transamination activity.

The AT of the present embodiment is a protein consisting of an amino acid sequence shown in any one of SEQ ID NOs. 1 to 4 and 103 to 105. The protein having an amino acid sequence shown in SEQ ID NO. 1 is an aminotransferase PpAT8 derived from *Pseudomonas putida* KT2440, the protein having an amino acid sequence shown in SEQ ID NO. 2 is an aminotransferase PpAT2 derived from *Pseudomonas putida* KT2440, the protein having an amino acid sequence shown in SEQ ID NO. 3 is an aminotransferase AsAT5 derived from *Aeromonas salmonicida subsp. salmonicida,* and the protein having an amino acid sequence shown in SEQ ID NO. 4 is an aminotransferase PatA derived from *Escherichia coli* K12 MG1655. The protein consisting of an amino acid sequences shown in SEQ ID NOs. 103 to 105 is a homolog protein (homologous protein) of a protein (PpAT8 or PatA) consisting of an amino acid sequence shown in SEQ ID NO. 1 or 4 derived from microorganisms shown in Table 3. So far, these proteins are not known to have activity of transferring an amino group of 1,4-bis(aminomethyl)cyclohexane to another compound to produce 4-(aminomethyl)cyclohexane-1-carbaldehyde.

The AT of another embodiment is a mutant protein or a homologous protein of the protein consisting of an amino acid sequence shown in any one of SEQ ID NOs. 1 to 4 and 103 to 105, which is a protein consisting of an amino acid sequence having 60% or more (preferably, 70% or more, 75% or more, 80% or more, 90% or more, 93% or more, 95% or more, or 98% or more) identity to an amino acid sequence shown in any one of SEQ ID NOs. 1 to 4 and 103 to 105 and having transamination activity.

In this specification, the mutant protein is a protein obtained by artificially deleting or substituting amino acid residues in a base protein or artificially inserting or adding amino acid residues into a base protein. In this specification, homologous proteins are a group of proteins that organisms existing in nature have and have evolutionary origins derived from the same protein. Homologous proteins have similar structures and functions.

In a mutant protein, when it is described that amino acids are deleted, substituted, inserted or added, this means that, at any position in the amino acid sequence, 1 to 20 amino acids may be deleted, substituted, inserted or added, and for example, 1 to 15, 1 to 10, 1 to 5, 1 to 4, 1 to 3, 1 to 2, or 1 amino acid may be deleted, substituted, inserted or added.

The amino acids substituted, inserted or added may be either naturally occurring or non-naturally occurring. Examples of natural amino acids include L-alanine, L-asparagine, L-aspartic acid, L-glutamine, L-glutamic acid, glycine, L-histidine, L-isoleucine, L-leucine, L-lysine, L-arginine, L-methionine, L-phenylalanine, L-proline, L-serine, L-threonine, L-tryptophan, L-tyrosine, L-valine, and L-cysteine.

Amino acids that can be substituted for each other are exemplified below. Amino acids contained in the same group can be substituted for each other.

Group A: leucine, isoleucine, norleucine, valine, norvaline, alanine, 2-aminobutanoic acid, methionine, o-methylserine, t-butylglycine, t-butylalanine, cyclohexylalanine
Group B: aspartic acid, glutamic acid, isoaspartic acid, isoglutamic acid, 2-aminoadipic acid, 2-aminosuberic acid
Group C: asparagine, glutamine
Group D: lysine, arginine, ornithine, 2,4-diaminobutanoic acid, 2,3-diaminopropionic acid
Group E: proline, 3-hydroxyproline, 4-hydroxyproline
Group F: serine, threonine, homoserine

Group G: phenylalanine, tyrosine

An amino acid sequence of a mutant protein or a homologous protein of a protein consisting of an amino acid sequence shown in any one of SEQ ID NOs. 1 to 4 and 103 to 105 has at least 60% or more, preferably 70% or more, 75% or more, 80% or more, 90% or more, or 93% or more, more preferably 95% or more, and most preferably 98% or more identity to an amino acid sequence shown in any one of SEQ ID NOs. 1 to 4 and 103 to 105.

The identity (also called a homology) of amino acid sequences or nucleotide sequences can be determined using the algorithm BLAST [Pro. Nat. Acad. Sci. USA, 90, 5873 (1993)] and FASTA [Methods Enzymol., 183, 63 (1990)] by Karlin and Altschul. Based on this algorithm BLAST, programs called BLASTN and BLASTX have been developed [J. Mol. Biol., 215, 403 (1990)]. When a nucleotide sequence is analyzed using BLASTN based on BLAST, the parameters are set to, for example, Score=100 and word length=12. In addition, when an amino acid sequence is analyzed using BLASTX based on BLAST, the parameters are set to, for example, score=50 and word length=3. When BLAST and Gap ped BLAST programs are used, the default parameters of the programs are used. Specific techniques for these analysis methods are known.

Having transamination activity can be confirmed by, for example, the following method. First, recombinant DNA containing DNA encoding a protein of which the activity is to be confirmed is prepared by a method described below. Next, microorganisms such as *E. coli* are transformed using the recombinant DNA, the obtained microorganisms are cultured, and 1,4-bis(aminomethyl)cyclohexane and pyruvic acid are added to the medium to produce 4-(aminomethyl)cyclohexane-1-carbaldehyde. Finally, when 4-(aminomethyl)cyclohexane-1-carbaldehyde in the bacterial cells or in the culture supernatant is detected using a general analysis method such as HPLC, it is possible to confirm that the target protein has transamination activity.

The DNA encoding the AT of one embodiment is DNA consisting of an amino acid sequence having 60% or more (preferably, 70% or more, 75% or more, 80% or more, 90% or more, 93% or more, 95% or more, or 98% or more) identity to an amino acid sequence shown in any one of SEQ ID NOs. 1 to 4 and 103 to 105 and encoding a protein having transamination activity. In this specification, DNA may be a gene or a part of a gene.

The DNA encoding the AT of the present embodiment is a nucleotide sequence encoding a protein consisting of an amino acid sequence shown in any one of SEQ ID NOs. 1 to 4 and 103 to 105, and examples thereof include DNA consisting of a nucleotide sequence shown in any one of SEQ ID NOs. 5 to 8 and 108 to 110.

The DNA encoding the AT of another embodiment is DNA encoding a mutant protein or a homologous protein of a protein consisting of an amino acid sequence shown in any one of SEQ ID NOs. 1 to 4 and 103 to 105. In the DNA encoding a mutant protein or a homologous protein, at any position of the nucleotide sequence shown in any one of SEQ ID NOs. 5 to 8 and 108 to 110, 1 to 50 bases may be deleted, substituted, inserted or added, and for example, 1 to 40, 1 to 30, 1 to 25, 1 to 20, 1 to 15, 1 to 10, 1 to 5, 1 to 4, 1 to 3, 1 to 2, or 1 base may be deleted, substituted, inserted or added. In addition, the DNA encoding a mutant protein or a homologous protein is preferably consisting of a nucleotide sequence having at least 60% or more, preferably 70% or more, 75% or more, 80% or more, 85% or more, 90% or more, or 93% or more, more preferably 95% or more, and most preferably 98% or more identity to a nucleotide sequence shown in any one of SEQ ID NOs. 5 to 8 and 108 to 110, and more preferably consisting of a nucleotide sequence shown in any one of SEQ ID NOs. 5 to 8 and 108 to 110.

DNA consisting of a nucleotide sequence shown in any one of SEQ ID NOs. 5 to 8 and 108 to 110 or DNA encoding a homologous protein with a known sequence may be amplified by PCR using genomic DNA as a template and appropriate primers. In this case, the end of the primer may contain a DNA sequence for cloning into an expression vector, such as in a restriction enzyme site. For a homologous protein, a probe or primer can be designed based on the search to obtain DNA encoding the homologous protein using microorganisms having the DNA. The DNA encoding a mutant protein can be obtained using error-prone PCR, a site directed mutagenesis method by PCR, a commercially available site directed mutagenesis kit or the like. In addition, for the DNA encoding the AT of one embodiment, based on the determined DNA nucleotide sequence, using an NTS M series DNA synthesis device (commercially available from Nihon Techno Service Co., Ltd.) or the like, chemical synthesis can be performed to prepare desired DNA.

Here, when bases in DNA of one embodiment are substituted so that optimal codons for expressing in host cells are obtained, it is possible to improve the expression level of the protein that the DNA encodes. Information on the codon usage in host cells can be obtained from public databases.

The DNA encoding the AT of one embodiment may be DNA that hybridizes to DNA consisting of a nucleotide sequence complementary to a nucleotide sequence shown in any one of SEQ ID NOs. 5 to 8 and 108 to 110 under stringent conditions. Hybridization is a process (step) in which DNA hybridizes to DNA having a specific nucleotide sequence or a part of the DNA. Therefore, the nucleotide sequence of DNA having the specific nucleotide sequence or DNA that hybridizes to a part of the DNA may be a length of DNA that is useful as a probe in northern or southern blot analysis or can be used as an oligonucleotide primer in PCR analysis.

Examples of DNA used as a probe include DNA having at least 100 bases or more, preferably 200 bases or more, and more preferably 500 bases or more, and examples of DNA used as a primer include DNA having at least 10 bases or more, and preferably 15 bases or more.

The methods for DNA hybridization experiments are well-known, and hybridization conditions can be determined and experiments can be performed, for example, according to Molecular Cloning, 4th Edition (Cold Spring Harbor Laboratory Press (2012)), Methods for General and Molecular Bacteriology (ASM Press (1994)), and Immunology methods manual (Academic pres (1997)) as well as numerous other standard textbooks.

In addition, DNA that hybridizes under stringent conditions can also be obtained according to the instructions bundled in a commercially available hybridization kit. Examples of commercially available hybridization kits include a Random Primed DNA Labeling Kit (commercially available from Roche Diagnostics Corporation) in which a probe is prepared by a random prime method and hybridization is performed under stringent conditions.

Examples of stringent conditions include conditions in which a filter on which DNA is immobilized and probe DNA are incubated overnight at 42°C in a solution containing 50% formamide, 5×SSC (750 mM sodium chloride, 75 mM sodium citrate), 50 mM sodium phosphate (pH 7.6), 5×Denhardt's solution, 10% dextran sulfate, and 20 µg/L of denatured salmon sperm DNA, and the filter is then washed, for example, in a 0.2×SSC solution at about 65°C.

The various conditions described above can also be set by adding or changing a blocking reagent used for blocking background in hybridization experiments. The addition of the above blocking reagent may be followed by modification of hybridization conditions in order to conform to the conditions.

Examples of DNA that can hybridize under the above stringent conditions include DNA consisting of a nucleotide sequence having at least 60% or more, preferably 70% or more, 75% or more, 80% or more, 85% or more, 90% or more, or 93% or more, more preferably 95% or more, and most preferably 98% or more identity to a nucleotide sequence shown in any one of SEQ ID NOs. 5 to 8 and 108 to 110, for example, when calculated using programs such as the BLAST and FASTA.

(2) Aldehyde dehydrogenase of present invention and DNA encoding it

The aldehyde dehydrogenase (ALDH) is a general term for a group of enzymes that catalyze an oxidation of aldehyde to carboxylic acid using NAD(P)$^+$ as a coenzyme in biochemistry. In this specification, aldehyde dehydrogenase activity refers to activity of catalyzing a reaction in which an aldehyde group of a compound having an aldehyde group is oxidized and converted into a carboxy group.

The protein having aldehyde dehydrogenase activity of the present invention is a protein having activity of oxidizing an aldehyde group of 4-(aminomethyl)cyclohexane-1-carbaldehyde to produce 4-(aminomethyl)cyclohexane-1-carboxylic acid. That is, it is a protein having activity of catalyzing a reaction from 4-(aminomethyl)cyclohexane-1-carbaldehyde to 4-(aminomethyl)cyclohexane-1-carboxylic acid. In this specification, these proteins are collectively referred as an "aldehyde dehydrogenase of the present invention" or "ALDH of the present invention."

An ALDH of one embodiment is, for example, a protein consisting of an amino acid sequence having 50% or more, or 60% or more (preferably, 70% or more, 75% or more, 80% or more, 90% or more, 93% or more, 95% or more, or 98% or more) identity to an amino acid sequence shown in any one of SEQ ID NOs. 19 to 22, 35 to 46 and 127 to 139 and having aldehyde dehydrogenase activity.

The ALDH of the present embodiment may be a protein consisting of an amino acid sequence shown in any one of SEQ ID NOs. 19 to 22, 35 to 46, and 127 to 139. The protein having an amino acid sequence shown in SEQ ID NO. 19 is a gamma-aminobutyraldehyde dehydrogenase PatD derived from *Escherichia coli* K12 MG1655, the protein having an amino acid sequence shown in SEQ ID NO. 20 is a benzaldehyde dehydrogenase XylC derived from *Pseudomonas putida* CSV86, the protein having an amino acid sequence shown in SEQ ID NO. 21 is a phenylacetaldehyde dehydrogenase StyD derived from *Pseudomonas putida* S12, and the protein having an amino acid sequence shown in SEQ ID NO. 22 is a 4-hydroxybenzaldehyde dehydrogenase PchA derived from *Pseudomonas putida* NCIMB 9866. The protein consisting of an amino acid sequence shown in any one of SEQ ID NOs. 35 to 46 and 127 to 139 is a homolog protein (homologous protein) of a protein (PatD, XylC or StyD) consisting of an amino acid sequence shown in SEQ ID NO. 19, 20 or 21 derived from microorganisms shown in Table 13 and Table 20. So far, these proteins are not known to have activity of oxidizing an aldehyde group of 4-(aminomethyl)cyclohexane-1-carbaldehyde to produce 4-(aminomethyl)cyclohexane-1-carboxylic acid.

An ALDH of another embodiment is a mutant protein or a homologous protein of the protein consisting of an amino acid sequence shown in any one of SEQ ID NOs. 19 to 22, 35 to 46, and 127 to 139, which is a protein consisting of an amino acid sequence having 50% or more, or 60% or more identity to an amino acid sequence shown in any one of SEQ ID NOs. 19 to 22, 35 to 46, and 127 to 139 and having aldehyde dehydrogenase activity. The amino acid sequence of the mutant protein or the homologous protein has at least 50% or more, or 60% or more, preferably 70% or more, 75% or more, 80% or more or 85% or more, more preferably 90% or more, still more preferably 93% or more or 95% or more, and most preferably 98% or more identity to an amino acid sequence shown in any one of SEQ ID NOs. 19 to 22, 35 to 46, and 127 to 139.

Having aldehyde dehydrogenase activity can be confirmed by, for example, the following method. First, recombinant DNA containing DNA encoding a protein of which the activity is to be confirmed is prepared by a method described below. Next, microorganisms such as *E. coli* are transformed using the recombinant DNA, the obtained microorganisms are cultured, and 4-(aminomethyl)cyclohexane-1-carbaldehyde and NAD(P)$^+$ are added to the medium to produce 4-(aminomethyl)cyclohexane-1-carboxylic acid. Finally, when 4-(aminomethyl)cyclohexane-1-carboxylic acid in the bacterial cells or in the culture supernatant is detected using a general analysis method such as HPLC, it is possible to confirm that the target protein has aldehyde dehydrogenase activity.

An ALDH of still another embodiment may be a protein that has aldehyde dehydrogenase activity of producing 4-(aminomethyl)cyclohexane-1-carboxylic acid from 4-(aminomethyl)cyclohexane-1-carbaldehyde and is classified into any one or more of the following 1) to 6).

1) a protein consisting of an amino acid sequence having 71% or more identity to an amino acid sequence shown in SEQ ID NO. 19
2) a protein consisting of an amino acid sequence having 68% or more identity to an amino acid sequence shown in SEQ ID NO. 20
3) a protein which is an aldehyde dehydrogenase derived from the genus *Psudomonas* and is consisting of an amino acid sequence having 50% or more identity to an amino acid sequence shown in SEQ ID NO. 20
4) a protein consisting of an amino acid sequence having 63% or more identity to an amino acid sequence shown in SEQ ID NO. 21
5) a protein consisting of an amino acid sequence having 56.5% or more identity to an amino acid sequence shown in SEQ ID NO. 40
6) a protein consisting of an amino acid sequence having 63.9% or more identity to an amino acid sequence shown in SEQ ID NO. 41

Examples of such proteins having aldehyde dehydrogenase activity include proteins consisting of an amino acid sequence shown in any one of SEQ ID NOs. 19 to 21, 35 to 41, 44 to 46, 127 to 131, 133 to 135, 138, and 139.

An ALDH of yet another embodiment is preferably an aldehyde dehydrogenase derived from bacteria belonging to the genus *Pseudomonas.*

Examples of bacteria belonging to the genus *Pseudomonas* include *Pseudomonas putida, Pseudomonas aeruginosa, Pseudomonas sp., Pseudomonas sp.* MAP12, *Pseudomonas fluorescens, Pseudomonas* syringae, *Pseudomonas amygdali, Pseudomonas oryzihabitans, Pseudomonas maltophilia, Pseudomonas trivialis, Pseudomonas savastanoi,* and *Pseudomonas stutzeri.*

An ALDH of another embodiment is a benzaldehyde dehydrogenase, which is a protein having aldehyde dehydrogenase activity of oxidizing an aldehyde group of 4-(aminomethyl)cyclohexane-1-carbaldehyde to produce 4-(aminomethyl)cyclohexane-1-carboxylic acid.

The benzaldehyde dehydrogenase of the present embodiment has aldehyde dehydrogenase activity of oxidizing an aldehyde group of 4-(aminomethyl)cyclohexane-1-carbaldehyde to produce 4-(aminomethyl)cyclohexane-1-carboxylic acid. The benzaldehyde dehydrogenase is a type of aldehyde dehydrogenase, and is a general term for a group of enzymes that catalyzes an oxidation of particularly benzaldehyde to benzoic acid.

The benzaldehyde dehydrogenase of the present embodiment may be a benzaldehyde dehydrogenase derived from microorganisms expressing benzaldehyde dehydrogenases in addition to the bacteria belonging to the genus *Pseudomonas,* and examples of microorganisms expressing benzaldehyde dehydrogenases include *Hydrogenophaga aromaticivorans, Alteromonas, Tepidiphilus succinatimandens, Halomonas cupida, Glaciimonas immobilis, Paraburkholderia unamae, Marinobacter salsuginis, Aromatoleum toluclasticum, Burkholderia_sp., Burkholderia_sp._D7, Bacillus subtilis, Acinetobacter guillouiae, Polaromonas sp., Bacillus sp., Chloroflexi bacterium, Halioxenophilus aromaticivorans, Novosphingobium aromaticivorans, Oceanobacillus iheyensis, Sphingobium chungbukense, Streptomyces violaceoruber, Rhodococcus aetherivorans, Xanthomonas campestris, Streptomyces calvus, Prauserella muralis, Acinetobacter calcoaceticus, Xanthomonas campestris, Acidovorax sp.,* and *Streptomyces sp..*

The benzaldehyde dehydrogenase of one embodiment may be a protein consisting of an amino acid sequence shown in any one of SEQ ID NOs. 20, 40, 41, 43, 127 to 137, and 139. In addition, in another embodiment, the benzaldehyde dehydrogenase is a mutant protein or a homologous protein of the protein consisting of an amino acid sequence shown in any one of SEQ ID NOs. 20, 40, 41, 43, 127 to 137, and 139 and may be a protein consisting of an amino acid sequence having 50% or more, or 60% or more (preferably 65% or more, 70% or more, 75% or more, 80% or more, 90% or more, 93% or more, 95% or more, or 98% or more) identity to an amino acid sequence shown in any one of SEQ ID NOs. 20, 40, 41, 43, 127 to 137, and 139, and having aldehyde dehydrogenase activity.

A benzaldehyde dehydrogenase of still another embodiment may be a protein that has aldehyde dehydrogenase activity of producing 4-(aminomethyl)cyclohexane-1-carboxylic acid from 4-(aminomethyl)cyclohexane-1-carbaldehyde and is classified into any one or more of the following 7) to 12).

7) a protein consisting of an amino acid sequence having 71% or more identity to an amino acid sequence shown in SEQ ID NO. 19

8) a protein consisting of an amino acid sequence having 68% or more identity to an amino acid sequence shown in SEQ ID NO. 20

9) a protein which is an aldehyde dehydrogenase derived from the genus *Psudomonas* and is consisting of an amino acid sequence having 50% or more identity to an amino acid sequence shown in SEQ ID NO. 20

10) a protein consisting of an amino acid sequence having 63% or more identity to an amino acid sequence shown in SEQ ID NO. 21

11) a protein consisting of an amino acid sequence having 56.5% or more identity to an amino acid sequence shown in SEQ ID NO. 40

12) a protein consisting of an amino acid sequence having 63.9% or more identity to an amino acid sequence shown in SEQ ID NO. 41

Examples of such benzaldehyde dehydrogenases include proteins consisting of an amino acid sequence shown in any one of SEQ ID NOs. 20, 40, 41, 127 to 131, 133 to 135, and 139.

An ALDH of still another embodiment is a protein having aldehyde dehydrogenase activity of producing 4-(aminomethyl)cyclohexane-1-carboxylic acid from 4-(aminomethyl)cyclohexane-1-carbaldehyde and consisting of an amino acid sequence having 50% or more (preferably 55% or more, 60% or more, 65% or more, 70% or more, 75% or more, 80% or more, 85% or more, 90% or more, 93% or more, 95% or more, or 98% or more) identity to an amino acid sequence shown in SEQ ID NO. 20, which is the amino acid sequence containing at least one of the following amino acid residues (1) to (26) when aligned with the amino acid sequence shown in SEQ ID NO. 20:

(1) an amino acid residue at a position corresponding to position 35 is a glycine residue (G)

(2) an amino acid residue at a position corresponding to position 65 is an alanine residue (A)

(3) an amino acid residue at a position corresponding to position 82 is a leucine residue (L)

(4) an amino acid residue at a position corresponding to position 104 is a glycine residue (G)

(5) an amino acid residue at a position corresponding to position 109 is a glycine residue (G)

(6) an amino acid residue at a position corresponding to position 110 is an isoleucine residue (I)

(7) an amino acid residue at a position corresponding to position 117 is a glutamine residue (Q)

(8) an amino acid residue at a position corresponding to position 122 is an alanine residue (A)

(9) an amino acid residue at a position corresponding to position 124 is a leucine residue (L)

(10) an amino acid residue at a position corresponding to position 134 is a valine residue (V)

(11) an amino acid residue at a position corresponding to position 144 is a valine residue (V)

(12) an amino acid residue at a position corresponding to position 160 is a phenylalanine residue (F)

(13) an amino acid residue at a position corresponding to position 271 is a glycine residue (G)

(14) an amino acid residue at a position corresponding to position 290 is an alanine residue (A)

(15) an amino acid residue at a position corresponding to position 319 is an aspartic acid residue (D)

(16) an amino acid residue at a position corresponding to position 333 is an aspartic acid residue (D)

(17) an amino acid residue at a position corresponding to position 365 is a glutamine residue (Q)

(18) an amino acid residue at a position corresponding to position 390 is an isoleucine residue (I)

(19) an amino acid residue at a position corresponding to position 420 is an alanine residue (A)

(20) an amino acid residue at a position corresponding to position 423 is a leucine residue (L)

(21) an amino acid residue at a position corresponding to position 434 is a valine residue (V)

(22) an amino acid residue at a position corresponding to position 440 is a proline residue (P)

(23) an amino acid residue at a position corresponding to position 443 is a cysteine residue (C)

(24) an amino acid residue at a position corresponding to position 445 is a proline residue (P)

(25) an amino acid residue at a position corresponding to position 466 is a serine residue (S)

(26) an amino acid residue at a position corresponding to position 467 is an isoleucine residue (I)

Here, for example, "the amino acid residue at a position corresponding to position 35" is an amino acid residue in the amino acid sequence of a target protein, which is located at a position corresponding to the 35th amino acid residue in SEQ ID NO. 20 when the amino acid sequence of the target protein is aligned with an amino acid sequence shown in SEQ ID NO. 20.

As shown in examples to be described below, it suggests that the above amino acid residues (1) to (26) are amino acid residues conserved in ALDH which have particularly high specificity for trans-4-(aminomethyl)cyclohexane-1-carbaldehyde. Therefore, it is thought that, when the amino acid sequence of the aldehyde dehydrogenase of the present embodiment contains at least one of the amino acid residues (1) to (26), it has specificity for trans-4-(aminomethyl)cyclohexane-1-carbaldehyde. That is, the ALDH of the present embodiment can have aldehyde dehydrogenase activity with high trans-selectivity (also called trans-specificity). Here, high trans-selectivity means that, when 4-(aminomethyl)cyclohexane-1-carboxylic acid is produced using ALDH, in the produced 4-(aminomethyl)cyclohexane-1-carboxylic acid containing tranexamic acid (TXA) and/or cis-TXA, the trans proportion of 4-(aminomethyl)cyclohexane-1-carboxylic acid calculated using a calculation formula shown in the following Math. 2 is more than 50%, and particularly, high trans-specificity means that the trans proportion is 70% or more. Here, in this specification, "selectivity" is treated as synonymous with "specificity," and "selective" is treated as synonymous with "specific."

The amino acid sequence of the aldehyde dehydrogenase of the present embodiment may contain at least one of the amino acid residues (1) to (26), and may contain, for example, 2 or more, 3 or more, 4 or more, 5 or more, 6 or more, 7 or more, 8 or more, 9 or more, 10 or more, 11 or more, 12 or more, 13 or more, 14 or more, 15 or more, 16 or more, 17 or more, 18 or more, 19 or more, 20 or more, 21 or more, 22 or more, 23 or more, 24 or more, 25 or more, or all of 26 groups.

The alignment of amino acid sequences can be created using, for example, a known alignment program Clustal Omega. Clustal Omega is available, for example, at https://www.ebi.ac.uk/Tools/msa/clustalo/. When the alignment is created using Clustal Omega, for example, default values can be used for parameters.

The aldehyde dehydrogenase of the present embodiment may be a protein consisting of an amino acid sequence having 50% or more (preferably 55% or more, 60% or more, 65% or more, 70% or more, 75% or more, 80% or more, 85% or more, 90% or more, 93% or more, 95% or more, or 98% or more) identity to an amino acid sequence shown in any one of SEQ ID NOs. 20, 40, 41, 127, 128, 130, 131, and 133 and having aldehyde dehydrogenase activity.

The DNA encoding the aldehyde dehydrogenase (ALDH) of the present embodiment is DNA encoding the protein having aldehyde dehydrogenase activity. Examples of DNA encoding an ALDH of one embodiment include DNA consisting of a nucleotide sequence encoding a protein consisting of an amino acid sequence shown in any one of SEQ ID NOs. 19 to 22, 35 to 46, and 127 to 139, for example, a nucleotide sequence shown in any one of SEQ ID NOs. 23 to 26, 47 to 58, and 140 to 152.

The DNA encoding an ALDH of another embodiment is DNA encoding a mutant protein or a homologous protein of a protein consisting of an amino acid sequence shown in any one of SEQ ID NOs. 19 to 22, 35 to 46 and 127 to 139. In the DNA encoding a mutant protein or a homologous protein, at any position of a nucleotide sequence shown in any one of SEQ ID NOs. 23 to 26, 47 to 58, and 140 to 152, 1 to 50 bases may be deleted, substituted, inserted or added, and for example, 1 to 40, 1 to 30, 1 to 25, 1 to 20, 1 to 15, 1 to 10, 1 to 5, 1 to 4, 1 to 3, 1 to 2, or 1 base may be deleted, substituted, inserted or added. The DNA encoding an ALDH of one embodiment is preferably consisting of a nucleotide sequence having at least 50% or more or 60% or more, preferably 70% or more, 75% or more, 80% or more, 85% or more, or 90% or more, more preferably 93% or more or 95% or more, and most preferably 98% or more identity to a nucleotide sequence shown in any one of SEQ ID NOs. 23 to 26, 47 to 58, and 140 to 152, and more preferably consisting of a nucleotide sequence shown in any one of SEQ ID NOs. 23 to 26, 47 to 58 and 140 to 152.

DNA encoding an ALDH of one embodiment may be DNA that hybridizes to DNA consisting of a nucleotide sequence complementary to a nucleotide sequence shown in any one of SEQ ID NOs. 23 to 26, 47 to 58, and 140 to 152 under stringent conditions. Examples of DNA that can hybridize under stringent conditions include DNA consisting of a nucleotide sequence having at least 50% or more or 60% or more, preferably 70% or more, 75% or more, 80% or more, 85% or more, or 90% or more, more preferably 93% or more or 95% or more, and most preferably 98% or more identity to a nucleotide sequence shown in any one of SEQ ID NOs. 23 to 26, 47 to 58, and 140 to 152, for example, when calculated using programs such as BLAST and FASTA.

3. Recombinant DNA of present invention

The recombinant DNA of the present invention contains DNA encoding the aminotransferase of the present invention and/or DNA encoding the aldehyde dehydrogenase of the present invention. It may further contain DNA encoding alanine dehydrogenase and/or DNA encoding NADH oxidase which will be described below. In the recombinant DNA of the present invention, one recombinant DNA may contain DNA encoding only one type of enzyme or DNA encoding a plurality of types of enzymes. In the case of a plurality of types of enzymes, the enzymes may be controlled by a single promoter or by separate promoters.

The recombinant DNA of the present invention is a vector that is autonomously replicable in host cells and/or a vector that can be integrated into host cell chromosome, and is a vector that can transcribe the DNA.

When prokaryotes such as bacteria are used as host cells, the recombinant DNA of the present invention preferably further contains a promoter, a ribosome binding sequence, and a transcription termination sequence in addition to the DNA encoding the various enzymes, and may further contain a gene that controls the promoter. Here, it is preferable to adjust the distance between the Shine-Dalgarno sequence, which is a ribosome binding sequence, and the initiation codon, to an appropriate distance, for example, 6 to 18 bases. In the recombinant DNA of the present invention, a transcription termination sequence is not necessarily required for expression of DNA encoding the various enzymes, but it is preferable to provide a transcription termination sequence immediately downstream of the structural gene.

The vector is not particularly limited as long as it is an appropriate DNA molecule for introducing desired DNA into a host, and proliferating and expressing it, and in addition to plasmids, for example, artificial chromosomes, vectors using transposons, and cosmids may be used.

When microorganisms belonging to the genus *Escherichia* are used as host cells into which the recombinant DNA of the present invention is introduced, examples of vectors include pColdI, pSTV28, pSTV29, and pUC118 (all commercially available from Takara Bio Inc.), pMW118, pMW119, and pMW218 (all commercially available from Nippon Gene Co., Ltd.), pET21a, pET28a, pCDF-1b, and pRSF-1b (all commercially available from Merck commercially available from Millipore), pMAL-c5x (commercially available from New England Biolabs), pGEX-4T-1 and pTrc99A (all commercially available from GE Healthcare Bio-Sciences), pTrcHis and pSE280 (all commercially available from Thermo Fisher Scientific Inc.), pGEMEX-1 (commercially available from Promega Corporation), pQE-30, pQE-60, and pQE80L (all commercially available from QIAGEN), pET-3, pBluescriptII SK(+), and pBluescriptII KS(-) (all commercially available from Agilent Technologies, Inc.), pKYP10 (Japanese Unexamined Patent Publication No. S58-110600), pKYP200 [Agric. Biol. Chem., 48, 669 (1984)], pLSA1 [Agric. Biol. Chem., 53, 277 (1989)], pGEL1 [Proc.Natl. Acad. Sci., USA, 82, 4306 (1985)], pTrS30 [prepared from *Escherichia coli* JM109/pTrS30 (FERM BP-5407)], pTrS32 [prepared from *Escherichia coli* JM109/pTrS32 (FERM BP-5408)], pTK31 [APPLIED AND ENVIRONMENTAL MICROBIOLOGY, 2007, Vol. 73, No. 20, p 6378-6385], pPE167 (Appl. Environ. Microbiol. 2007, 73:6378-6385), pPAC31 (WO98/12343), pUC19 [Gene, 33, 103 (1985)], and pPA1 (Japanese Unexamined Patent Publication No. S63-233798).

The promoter when the vector is used may be any promoter that functions in cells of microorganisms belonging to the genus *Escherichia,* and for example, promoters derived from *Escherichia coli* and phages such as a trp promoter, a gapA promoter, a lac promoter, a PL promoter, a PR promoter, and a PSE promoter can be used. In addition, artificially designed and modified promoters such as a promoter in which two trp promoters are arranged in series, a tac promoter, a trc promoter, a lacT5 promoter, a lacT7 promoter, and a let I promoter can be used.

When *coryneform* bacteria are used as host cells into which the recombinant DNA of the present invention is introduced, examples of vectors include pCG1 (Japanese Unexamined Patent Publication No. S57-134500), pCG2 (Japanese Unexamined Patent Publication No. S58-35197), pCG4 (Japanese Unexamined Patent Publication No. S57-183799), pCG11 (Japanese Unexamined Patent Publication No. S57-134500), pCG116, pCE54, and pCB101 (all Japanese Unexamined Patent Publication No. S58-105999), and pCE51, pCE52, and pCE53 [all Molecular and General Genetics, 196, 175 (1984)].

The promoter when the vector is used may be any promoter that functions in cells of *coryneform* bacteria, and for example, a P54-6 promoter [Appl. Microbiol. Biotechnol., 53, p674-679 (2000)] can be used.

When yeast strains are used as host cells into which recombinant DNA of the present invention is introduced, examples of vectors include YEp13(ATCC37115), YEp24(ATCC37051), YCp50(ATCC37419), pHS19, and pHS15.

The promoter when the vector is used may be any promoter that functions in yeast strain cells, and examples of promoters include a PHO5 promoter, a PGK promoter, a GAP promoter, an ADH promoter, a gall promoter, a gal10 promoter, a heat shock polypeptide promoter, an MF$\alpha$1 promoter, and a CUP1 promoter.

The recombinant DNA of the present invention can be prepared, for example, using an In-Fusion (trademark) HD Cloning Kit (commercially available from Takara Bio Inc.) or by treating a DNA fragment encoding a desired enzyme with a restriction enzyme, and inserting it downstream of the promoter of the appropriate expression vector.

4. Recombinant cell of present invention

A recombinant cell of the present invention is a recombinant cell that contains the DNA of the present invention or is obtained by transforming a host cell with the recombinant DNA of the present invention. In addition, it may further contain DNA encoding alanine dehydrogenase and/or DNA encoding NADH oxidase which will be described below. In recombinant cells, the DNA or recombinant DNA of the present invention may be incorporated into the genome or may be provided as an autonomously replicable plasmid, but the DNA of the present invention in a transcribable state may be contained. One host cell may contain only one type of DNA or may contain two or more types of DNA.

The host cell may be any of prokaryotes, yeasts, animal cells, insect cells, and plant cells, and examples of host cells include preferably prokaryotes and yeast strains, more preferably, prokaryotes belonging to the genus *Escherichia,* the genus *Serratia,* the genus *Bacillus,* the genus *Brevibacterium,* the genus *Corynebacterium,* the genus *Microbacterium,* or the genus *Pseudomonas,* and yeast strains belonging to the genus *Saccharomyces,* the genus *Schizosaccharomyces,* the genus *Kluyveromyces,* the genus *Trichosporon,* the genus *Schwanniomyces,* the genus *Pichia,* or the genus *Candida,* and most preferably, prokaryotes such as *Escherichia coli* BL21 codon plus, *Escherichia coli* XL1-Blue, *Escherichia coli* XL2-Blue (all commercially available from Agilent Technologies, Inc.), *Escherichia coli* BL21(DE3) (commercially available from Novagen Co., Ltd.), *Escherichia coli* BL21(DE3)pLysS (commercially available from Merck Millipore), *Escherichia coli* DH5$\alpha$, *Escherichia coli* HST08Premium, *Escherichia coli* HST02, *Escherichia coli* HST04 dam-/dcm-, *Escherichia coli* JM109, *Escherichia coli* HB101, *Escherichia coli*CJ236, *Escherichia coli* BMH71-18 mutS, *Escherichia coli* MV1184, and *Escherichia coli* TH2 (all commercially available from Takara Bio Inc.), *Escherichia coli* W, *Escherichia coli* JM101, *Escherichia coli* W3110, *Escherichia coli* MG1655, *Escherichia coli* DH1, *Escherichia coli* MC1000, *Escherichia coli* W1485, *Escherichia coli* MP347, *Escherichia coli* NM522, *Escherichia coli* ATCC9637, *Serratia*

*ficaria, Serratia fonticola, Serratia liquefaciens, Serratia marcescens, Bacillus subtilis, Bacillus amyloliquefaciens, Brevibacterium immariophilum* ATCC14068, *Brevibacterium saccharolyticum* ATCC14066, *Corynebacterium ammoniagenes, Corynebacterium glutamicum* ATCC13032, *Corynebacterium glutamicum* ATCC14067, *Corynebacterium glutamicum* ATCC13869, *Corynebacterium acetoacidophilum* ATCC13870, *Microbacterium ammoniaphilum* ATCC15354, and *Pseudomonas sp.* D-0110, and yeast strains such as *Saccharomyces cerevisiae, Schizosaccharomyces pombe, Kluyveromyces lactis, Trichosporon pullulans, Schwanniomyces alluvius, Pichia pastoris,* and *Candida utilis.*

Examples of methods for introducing the recombinant DNA of the present invention into host cells as an autonomously replicable plasmid include a method using calcium ions [Proc. Natl. Acad. Sci., USA, 69, 2110 (1972)], a protoplast method (Japanese Unexamined Patent Publication No. S63-248394), an electroporation method [Nucleic Acids Res., 16, 6127 (1988)], a spheroplast method [Proc. Natl. Acad. Sci., USA, 81, 4889 (1984)], and a lithium acetate method [J. Bacteriol., 153, 163 (1983)].

Examples of methods for introducing the recombinant DNA of the present invention into host cell chromosomes include a homologous recombination method. Examples of homologous recombination methods include a method using a homologous recombination plasmid that can be prepared by linking to a plasmid DNA having a drug resistance gene that cannot be autonomously replicated in host cells into which a recombinant DNA is to be introduced. Examples of methods using homologous recombination frequently used in *Escherichia coli* include a method for introducing a recombinant DNA using a homologous recombination system of lambda phage [Proc. Natl. Acad. Sci. USA, 97, 6641-6645 (2000)].

In addition, it is possible to obtain *E. coli* in which a desired region on the chromosomal DNA of a host cell is substituted with the DNA or recombinant DNA of the present invention using a selection method utilizing the fact that *E. coli* becomes susceptible to sucrose due to *Bacillus subtilis* levansucrase incorporated into the chromosome together with the recombinant DNA, a selection method utilizing the fact that *E. coli* becomes susceptible to streptomycin by incorporating a wild-type rpsL gene into *E. coli* having a mutant rpsL gene that is resistant to streptomycin [Mol. Microbiol., 55, 137 (2005), Biosci. Biotechnol.Biochem., 71, 2905 (2007)] or the like.

5. Production method of present invention

The production method of the present invention is a method for producing 4-(aminomethyl)cyclohexane-1-carboxylic acid from 1,4-bis(aminomethyl)cyclohexane, including

(i) a step of producing 4-(aminomethyl)cyclohexane-1-carbaldehyde from 1,4-bis(aminomethyl)cyclohexane in the presence of an aminotransferase, and
(ii) a step of producing 4-(aminomethyl)cyclohexane-1-carboxylic acid from 4-(aminomethyl)cyclohexane-1-carbaldehyde in the presence of an aldehyde dehydrogenase.

The production method of the present embodiment includes allowing alanine dehydrogenase and/or NADH oxidase to coexist in a part or all of the method. Particularly, in the first step (step (i)), it is preferable to allow alanine dehydrogenase and/or NADH oxidase to coexist or it is preferable to allow NADH oxidase coexist in the second step (step (ii)).

When the substrate substance (1,4-bis(aminomethyl)cyclohexane used in the first step or 4-(aminomethyl) cyclohexane-1-carbaldehyde used in the second step) of each step of the production method of the present invention has a different three-dimensional structure from a desired product (4-(aminomethyl)cyclohexane-1-carbaldehyde in the first step or 4-(aminomethyl)cyclohexane-1-carboxylic acid in the second step) in each step, it is preferable that a part or all of the production method of the present embodiment be performed under neutral or basic conditions, and for example, it is preferable to perform a part or all of the step of the first step and/or a part or all of the step of the second step under neutral or basic conditions. When the reaction is performed under neutral or basic conditions, the isomerization of cis-4-(aminomethyl)cyclohexane-1-carbaldehyde to trans-4-(aminomethyl)cyclohexane-1-carbaldehyde serving as a reaction intermediate or the isomerization of a trans-form to a cis-form is promoted. The isomerization is particularly likely to occur under basic conditions of pH 9 or more, and the isomerization of 4-(aminomethyl)cyclohexane-1-carbaldehyde reaches equilibrium at a trans proportion of about 60%. Therefore, for example, when the tranexamic acid is the final target product, even if a cis-form or a mixture of a cis-form and a trans-form is used as the substrate, it is possible to obtain a larger amount of trans-4-(aminomethyl)cyclohexane-1-carboxylic acid by using an aldehyde dehydrogenase with high trans-selectivity to be described below, that is, it is possible to obtain a higher trans proportion in the product mixture. Accordingly, it is possible to produce a tranexamic acid at a high yield. On the other hand, when the trans-form is used as the substrate, isomerization can be prevented by making the reaction solution acidic (for example, pH 4 to 6), and it is possible to obtain a larger amount of the trans-form product.

The neutral condition means that the pH of the reaction solution is around 7, the basic condition means that the pH of the reaction solution is more than 7 to 12, and the neutral or basic condition means that the pH of the reaction solution is preferably 7 to 12, and more preferably 7 to 10. The method for making the solution basic is not particularly limited, and it can be achieved by adding an alkaline solution, urea, calcium carbonate, ammonia or the like. When it is described that a part of the step is performed under neutral or basic conditions, this means that the conditions are neutral or basic from the beginning to the middle of the step, or from the middle to the end of the step. Here, the beginning of the step is before the enzyme is added to the reaction solution, and the end of the step is after the enzyme reaction is completed (the same applies hereinafter). When the cis-form is isomerized to the trans-form according to neutral or basic conditions, it is possible to obtain an isomerization rate of 20% or more, for example, 40% or more, 50% or more, or 60% or more. In addition, when the trans-form is isomerized to the cis-form, it is possible to obtain an isomerization rate of 20% or more, for example, 30% or more or 35% or more.

When the substrate substance in each step of the production method of the present invention has a different three-dimensional structure from a desired product, it is preferable that a part or all of reaction step be performed in the presence of a secondary amine. The presence of a secondary amine promotes the isomerization of 4-(aminomethyl) cyclohexane-1-carbaldehyde. Examples of secondary amines include L-proline, pyrrolidine, pyrrolidine derivatives, and trans-4-hydroxy-L-proline. The amount of the secondary amine added to the reaction solution may be 0.01 to 500 mM, and is preferably 0.1 to 200 mM. When a secondary amine is added, it is possible to obtain a cis-form to trans-form isomerization rate of 20% or more, for example, 60% or more, and it is possible to obtain a trans-form to cis-form isomerization rate of 20% or more, for example, 30% or more or 35% or more.


(1) Step of producing 4-(aminomethyl)cyclohexane-1-carbaldehyde

The production method of the present invention includes (i) a step of producing 4-(aminomethyl)cyclohexane-1-carbaldehyde from 1,4-bis(aminomethyl)cyclohexane in the presence of an aminotransferase (hereinafter referred to as a first step). The aminotransferase is preferably the aminotransferase of the present invention. The presence of an aminotransferase means that an active aminotransferase is present in the reaction system in (i) so that the reaction in (i) can be catalyzed. For example, the method includes a step of adding the aminotransferase of the present invention to the reaction system or allowing cells that contain DNA encoding the aminotransferase of the present invention and can express the aminotransferase or recombinant cells obtained by transforming host cells with the recombinant DNA containing DNA encoding the aminotransferase of the present invention, which are cells that can express the aminotransferase, to react. Here, allowing cells to react means adding a substrate substance to a cell culture system and allowing it to react with proteins expressed by the cells.

In the first step, a protein having transamination activity may be used as an enzyme source, the enzyme source and a reaction substrate may be present in an aqueous medium to produce and accumulate 4-(aminomethyl)cyclohexane-1-carbaldehyde in the aqueous medium, cells having an ability to produce an aminotransferase may be cultured in the medium, and 4-(aminomethyl)cyclohexane-1-carbaldehyde may be produced and accumulated in the culture material.

In the method using an enzyme source, the enzyme source may be a purified protein or may be a culture material obtained by culturing cells having an ability to produce a protein having desired activity in a medium or a treated product of the culture material.

The culture material or the treated product of the culture material contains a protein having desired activity as an enzyme source. Examples of treated products of the culture material include a concentrated product of the culture material, a dried product of the culture material, bacterial cells obtained by centrifuging the culture material, a dried product of the bacterial cells, a freeze-dried product of the bacterial cells, a surfactant-treated product of the bacterial cells, an ultrasonically treated product of the bacterial cells, a product obtained by mechanically grinding the bacterial cells, a solvent-treated product of the bacterial cells, an enzyme-treated product of the bacterial cells, protein fractions of the bacterial cells, an immobilized product of the bacterial cells and an enzyme preparation obtained by extracting the bacterial cells.

In the method using the enzyme source in the first step, when a purified protein is used as the enzyme source, the amount of the protein having transamination activity for 1,4-bis(aminomethyl)cyclohexane may be 0.01 to 100 wt% and is preferably 0.1 to 50 wt%. Regarding the enzyme source, when a culture material or a treated product of the culture material is used as the enzyme source, the amount of the enzyme source varies depending on the specific activity of the enzyme source or the like, and it may be, for example, 5 to 1,000 wt% and is preferably 10 to 400 wt%, in terms of the weight of wet bacterial cells, relative to 1,4-bis(aminomethyl)cyclohexane.

Examples of aqueous media include buffer solutions such as water, phosphate, carbonate, acetate, borate, citrate, tris, 2-morpholinoethanesulfonic acid (hereinafter referred to as MES), 3-morpholinopropanesulfonic acid (hereinafter referred to as MOPS), and N-cyclohexyl-2-aminoethanesulfonic acid (hereinafter referred to as CHES), alcohols such as methanol and ethanol, esters such as ethyl acetate, ketones such as acetone, and amides such as acetamide. In addition, the culture solution of microorganisms used as the enzyme source can also be used as the aqueous medium.

As described in the above 1., the reaction of producing 4-(aminomethyl)cyclohexane-1-carbaldehyde from 1,4-bis(aminomethyl)cyclohexane is an enzyme reaction catalyzed by an aminotransferase (protein having transamination activity) in the presence of a compound capable of providing a carbonyl group. Therefore, in the first step, it is desirable to allow a compound capable of providing a carbonyl group to coexist.

Examples of compounds capable of providing a carbonyl group include a keto acid, and preferable examples thereof include pyruvic acid, α-ketoglutaric acid, and α-ketobutyric acid.

The keto acid may be of any origin as long as it serves as a substrate for the aminotransferase of the present invention, and a culture material of microorganisms having an ability to produce a keto acid or a treated product of the culture material may be used without change or a keto acid collected from the culture material or a treated product of the culture material may be used.

In addition, when microorganisms are cultured in a medium to produce and accumulate 4-(aminomethyl) cyclohexane-1-carbaldehyde, as the microorganisms, microorganisms having an ability to produce a keto acid may be used. Examples of microorganisms having an ability to produce a keto acid include microorganisms in which genes for a pyruvate dehydrogenase complex involved in pyruvate degradation in vivo have been deleted or attenuated, microorganisms in which lactate dehydrogenase genes have been deleted or attenuated, and microorganisms in which pyruvic acid oxidase genes have been deleted or attenuated. Here, "attenuating" means reducing the expression level of a target gene (here, a gene for a pyruvate dehydrogenase complex).

In the first step, when a keto acid is added, the amount added may be a molar ratio of 0.01 to 100 and is preferably 0.01 to 60, relative to 1,4-bis(aminomethyl)cyclohexane.

In the reaction step in the first step, when pyruvic acid is used as a keto acid, it is preferable to allow alanine dehydrogenase (AlaDH) to coexist. As described in the above 1, AlaDH is an enzyme that uses $NAD^+$ as a coenzyme and can catalyze conversion (regeneration) of the produced alanine to pyruvic acid. Therefore, when AlaDH is allowed to coexist, pyruvic acid is reused, and 4-(aminomethyl)cyclohexane-1-carbaldehyde can be efficiently produced with a small amount of raw materials. In addition, when AlaDH is allowed to coexist, it is desirable to add $NAD^+$ required for the reaction.

The AlaDH is one of alanine, aspartate and glutamate metabolic enzymes and is a redox enzyme that catalyzes the reversible reaction between (alanine+water+$NAD^+$) and (pyruvic acid+$NH_3$+NADH+$H^+$). In the first step of the method for producing tranexamic acid of the present invention, alanine produced when pyruvic acid is used as a keto acid can be converted to pyruvic acid.

The AlaDH is not particularly limited, and may be, for example, AlaDH derived from *Bacillus subtilis, Alkalihalobacillus pseudofirmus,* or *Staphylococcus aureus,* and specific examples thereof include alanine dehydrogenase BsAlaDH derived from *Bacillus subtilis* 168 (accession number: WP_003243280.1), alanine dehydrogenase ApAlaDH derived from *Alkalihalobacillus pseudofirmus* (accession number: WP_012957376.1), and alanine dehydrogenase SaAlaDH derived from *Staphylococcus aureus* (accession number: WP_000689998.1).

Allowing AlaDH to coexist may mean, in the enzyme reaction in the first step, adding a culture material of cells expressing AlaDH or a treated product of the culture material, co-culturing cells expressing AlaDH or incorporating DNA encoding AlaDH into the recombinant cell.

The amount of AlaDH may be 0.01 to 100 wt% and is preferably 0.1 to 50 wt%, relative to the alanine produced in the transamination reaction. When a culture material or a treated product of the culture material is used as the enzyme source, the amount of the enzyme source varies depending on the specific activity of the enzyme source or the like, and for example, it may be 5 to 1,000 wt% and is preferably 10 to 400 wt%, in terms of weight of wet bacterial cells, relative to 1,4-bis(aminomethyl)cyclohexane. In addition, it is desirable to add $NAD^+$. The amount of $NAD^+$ added may be a molar ratio of 0.01 to 100 and is preferably 0.01 to 60, relative to the alanine produced in the transamination reaction.

The substrate substance 1,4-bis(aminomethyl)cyclohexane may be any of a trans-form, a cis-form or a mixture of a trans-form and a cis-form, and a mixture of a trans-form and a cis-form is preferable in consideration of ease of availability of raw materials. In addition, desired 4-(aminomethyl)cyclohexane-1-carbaldehyde may be any of a trans-form, a cis-form or a mixture of a trans-form and a cis-form. 4-(aminomethyl)cyclohexane-1-carbaldehyde to be produced is preferably in a trans-form when it is supplied to produce tranexamic acid. In a mixture of a trans-form and a cis-form, the proportions of trans-forms and cis-forms are not important but the proportion of trans-forms is preferably more than 50%.

(2) Step of producing 4-(aminomethyl)cyclohexane-1-carboxylic acid

The production method of the present invention includes (ii) a step of producing 4-(aminomethyl)cyclohexane-1-carboxylic acid from 4-(aminomethyl)cyclohexane-1-carbaldehyde in the presence of an aldehyde dehydrogenase (hereinafter referred to as a second step). Preferably, the production method includes a step of allowing 4-(aminomethyl)cyclohexane-1-carbaldehyde to react with the protein having aldehyde dehydrogenase activity of the present invention, cells containing DNA encoding the protein having aldehyde dehydrogenase activity of the present invention, or recombinant cells obtained by transforming host cells with the recombinant DNA containing DNA encoding the protein having aldehyde dehydrogenase activity of the present invention.

In the second step, the protein having aldehyde dehydrogenase activity is used as an enzyme source, the enzyme source and a reaction substrate may be present in an aqueous medium to produce and accumulate 4-(aminomethyl)cyclohexane-1-carboxylic acid in the aqueous medium, microorganisms having an ability to produce an aldehyde dehydrogenase may be cultured in a medium, and 4-(aminomethyl)cyclohexane-1-carboxylic acid may be produced or accumulated in the culture material.

As described in the above 1., the reaction of producing 4-(aminomethyl)cyclohexane-1-carboxylic acid from 4-(aminomethyl)cyclohexane-1-carbaldehyde is an enzyme reaction catalyzed by an aldehyde dehydrogenase (protein having aldehyde dehydrogenase activity) in the presence of the coenzyme $NAD(P)^+$. Aldehyde dehydrogenases include an $NAD^+$ type that prefers $NAD^+$ as a coenzyme, an $NADP^+$ type that prefers $NADP^+$ as a coenzyme, and a type that can use both $NAD^+$ and $NADP^+$ as coenzymes. Therefore, it is preferable to allow a highly available coenzyme for the aldehyde dehydrogenase used to coexist.

The amount of $NAD(P)^+$ may be in a molar ratio of 0.01 to 100 and preferably 0.01 to 60, relative to 4-(aminomethyl)cyclohexane-1-carbaldehyde.

In addition, in a method for supplying a coenzyme, microorganisms having an ability to produce a coenzyme can be used as hosts for expressing an aldehyde dehydrogenase. Therefore, it is expected to further improve the amount of 4-(aminomethyl)cyclohexane-1-carboxylic acid produced. Examples of microorganisms having an ability to produce a coenzyme include microorganisms in which genes involved in $NAD(P)^+$ degradation in vivo have been deleted or attenuated, microorganisms in which genes (for example, pncB, nadA, nadB) involved in $NAD(P)^+$ production have been enhanced or introduced, microorganisms in which genes (for example, nadR) that inhibit expression or functions of a gene product involved in the $NAD(P)^+$ production have been deleted or attenuated, and microorganisms in which genes (for example, mazG and nudC) involved in $NAD(P)^+$ degradation have been deleted or attenuated.

The amount of aldehyde dehydrogenase may be 0.01 to 100 wt% and is preferably 0.1 to 50 wt%, relative to 1,4-bis(aminomethyl)cyclohexane. When a culture material or a treated product of the culture material is used as the enzyme source, the amount of the enzyme source varies depending on the specific activity of the enzyme source or the like, and it may be, for example, 5 to 1,000 wt% and is preferably 10 to 400 wt%, in terms of the weight of wet bacterial cells, relative to 4-(aminomethyl)cyclohexane-1-carbaldehyde.

In the step in the second step, it is preferable to allow an NAD(P)H oxidase (NOX) to coexist.

The NAD(P)H oxidase is one of metabolic enzymes, and is a redox enzyme that catalyzes the reaction of converting $(NAD(P)H+H^++O_2)$ to $(NAD(P)^++H_2O_2$ or $H_2O)$. The NAD(P)H produced in the first and second steps of the method for producing 4-(aminomethyl)cyclohexane-1-carboxylic acid of the present invention can be converted to $NAD(P)^+$. Therefore, when an NAD(P)H oxidase is allowed to coexist, $NAD(P)^+$ is reused, and 4-(aminomethyl)cyclohexane-1-carboxylic acid can be efficiently produced with a small amount of raw materials.

The NOX is not particularly limited, and may be, for example, NOX derived from *Bacillus subtilis, Streptococcus mutans, Lactococcus lactis,* or *Enterococcus feacalis,* and specific examples thereof include NADH oxidase BsNOX derived from *Bacillus subtilis* 168 (accession number: NP_389836.1), NADH oxidase SmNOX derived from *Streptococcus mutans* (accession number: WP_002268044.1), NADH oxidase LINOX derived from *Lactococcus lactis* (accession number: CAL97012.1), and NADH oxidase EfNOX derived from *Enterococcus feacalis* (accession number: WP_002361833.1).

Allowing an NAD(P)H oxidase to coexist may mean adding a culture material of cells expressing an NAD(P)H oxidase or a treated product of the culture material, co-culturing cells expressing an NAD(P)H oxidase, or incorporating DNA encoding an NAD(P)H oxidase into the recombinant cell.

The amount of NAD(P)H oxidase may be 0.001 to 100 wt% and is preferably 0.01 to 50 wt% with respect to a total amount of NAD(P)H produced when alanine dehydrogenase is used in the first step and NAD(P)H produced using ALDH in the second step. When a culture material or a treated product of the culture material is used as the enzyme source, the amount of the enzyme source varies depending on the specific activity of the enzyme source or the like, and it may be, for example, 0.5 to 1,000 wt% and is preferably 1 to 400 wt%, in terms of the wet bacteria weight, relative to 1,4-bis(aminomethyl)cyclohexane.

Instead of allowing an NAD(P)H oxidase to coexist, NAD(P)H can be electrically oxidized to regenerate $NAD(P)^+$.

The substrate substance 4-(aminomethyl)cyclohexane-1-carbaldehyde may be any of a trans-form, a cis-form or a mixture of a trans-form and a cis-form. When the substrate and the target compound have different three-dimensional structures, it is preferable to perform a part or all of the method under neutral or basic conditions, and more preferable to perform a part or all of the method in the presence of a secondary amine. When the substrate and the target compound have the same three-dimensional structure, it is desirable to perform a part or all of the method under acidic conditions.

The desired 4-(aminomethyl)cyclohexane-1-carboxylic acid may be any of a cis-form, a trans-form and a mixture thereof. When the cis-form (that is, cis-tranexamic acid) is desired, it is preferable to use, as the protein having aldehyde dehydrogenase activity in the second step, a protein shown in any one of SEQ ID NOs. 19, 21, 22, 35 to 37, 39, 44 to 46, 132, 134, 135 and 138, which is an enzyme that can accept a cis-form substrate of one embodiment, or a mutant or homologous protein thereof. When the trans-form (that is, tranexamic acid) is desired, it is preferable to use, as the protein having aldehyde dehydrogenase activity in the second step, a protein shown in any one of SEQ ID NOs. 20, 38, 40, 41, 127, 128, 129, 130, 131, 133, and 139, which is an enzyme with high trans-selectivity of one embodiment, or a mutant or homologous protein thereof. Among these, particularly, it is more preferable to use a protein shown in any one of SEQ ID NOs. 20, 40, 41, 127, 128, 130, 131 and 133, which is an enzyme having high trans-specificity or a mutant or homologous protein thereof. In order to obtain a mixture of a cis-form and a trans-form, any aldehyde dehydrogenase of the present invention may be used. The proportion of the trans-form in a mixture of a cis-form and a trans-form is 30% or more, and preferably, for example, 40% or more, 50% or more, 60% or more, or 70% or more.

In this specification, enzymes (ALDH) with high trans-selectivity are enzymes in which the trans proportion of 4-(aminomethyl)cyclohexane-1-carboxylic acid (AMCHA) produced by a two-step reaction performed using a mixture of cis- and trans-1,4-bis(aminomethyl)cyclohexane calculated in examples to be described below is more than 50% (for example, 51% or more, 55% or more, 60% or more, 65% or more, or 70% or more), and among these, enzymes with a trans proportion of 70% or more are enzymes with particularly high trans-selectivity. More specifically, the trans proportion of AMCHA can be calculated, for example, by multiplying the ratio of the amount of trans-AMCHA produced with respect to a total amount of the amount of trans-AMCHA produced and the amount of cis-AMCHA produced by 100. These enzymes (ALDH) with high trans-specificity allow tranexamic acid to be produced at a high yield, and it can be said that they are useful in the production of tranexamic acid.

The first step and the second step may be performed separately or simultaneously in the same system. In this case, various enzymes may be present in cells or purified proteins, or may be partially present in cells or partially purified proteins.

The cells can be cultured by a general method. The medium for culturing the cells may be either a natural medium or a synthetic medium as long as it is a medium which contains a carbon source, a nitrogen source, inorganic salts and the like that can be assimilated by the cells and allows the cells to be cultured efficiently.

In the method for producing 4-(aminomethyl)cyclohexane-1-carboxylic acid by a fermentation method, it is desirable to add the substrate 1,4-bis(aminomethyl)cyclohexane to the medium. 1,4-bis(aminomethyl)cyclohexane may be added to the medium before the culture or may be added to the culture solution during the culture.

In addition, instead of adding 1,4-bis(aminomethyl)cyclohexane to the medium, microorganisms having an ability to produce 1,4-bis(aminomethyl)cyclohexane may be co-cultured with the cells, and thus 1,4-bis(aminomethyl)cyclohexane may be supplied.

The carbon source may be any source that can be assimilated by the cells, and examples thereof include carbohydrates such as glucose, fructose, sucrose, molasses containing these, glycerol, starch and starch hydrolyzates, organic acids such as acetic acid and propionic acid, and alcohols such as ethanol and propanol.

Examples of nitrogen sources include ammonia, ammonium salts of inorganic acids or organic acids such as ammonium chloride, ammonium sulfate, ammonium acetate and ammonium phosphate, other nitrogen-containing compounds, as well as, peptone, meat extracts, yeast extracts, corn steep liquors, casein hydrolysates, soybean cakes, soybean cake hydrolyzates, various fermentation bacterial cells, and their digestive products.

Examples of inorganic salts include monopotassium phosphate, dipotassium phosphate, magnesium phosphate, magnesium sulfate, sodium chloride, ferrous sulfate, manganese sulfate, copper sulfate and calcium carbonate.

In the method for producing 4-(aminomethyl)cyclohexane-1-carboxylic acid by a fermentation method, pyruvic acid, $\alpha$-ketoglutaric acid, $\alpha$-ketobutyric acid, NAD$^+$, NADP$^+$ and the like, which are substrates for an aminotransferase or aldehyde dehydrogenase, may be added to the medium. In addition, in order to induce the isomerization of 4-(aminomethyl)cyclohexane-1-carbaldehyde, a secondary amine, preferably L-proline, pyrrolidine derivatives and the like may be added to the medium.

In addition, instead of adding the above compounds, these compounds may be supplied by co-culturing microorganisms having an ability to produce the above compounds with the cells or by co-expressing the microorganisms with the above enzymes.

Culture is preferably performed under aerobic conditions, for example, general shaking culture or submerged aeration stirring culture. The culture temperature is preferably 15 to 40°C, and the culture time is generally 5 hours to 7 days. The pH during culture is preferably maintained at 3.0 to 9.0. The pH is adjusted using an inorganic or organic acid, an alkaline solution, urea, calcium carbonate, ammonia or the like.

In addition, during culture, as necessary, an antibiotic such as ampicillin and kanamycin may be added to the medium. When recombinant cells transformed with an expression vector using an inducible promoter as the promoter are cultured, as necessary, an inducer may be added to the medium.

For example, when microorganisms transformed with an expression vector using a lac promoter are cultured, isopropyl-$\beta$-D-thiogalactopyranoside and the like may be added to the medium, and when microorganisms transformed with an expression vector using a trp promoter are cultured, indoleacrylic acid and the like may be added to the medium.

When 4-(aminomethyl)cyclohexane-1-carboxylic acid is produced in the culture material or in the bacterial cells according to the above culture, 4-(aminomethyl)cyclohexane-1-carboxylic acid, preferably tranexamic acid, can be produced. Quantification of 4-(aminomethyl)cyclohexane-1-carboxylic acid can be performed using LCMS (for example, analysis device LCMS-8040 commercially available from Shimadzu Corporation).

4-(aminomethyl)cyclohexane-1-carboxylic acid can be collected from the culture material by a combination of a general ion exchange resin method, a precipitation method and other known methods. Here, when 4-(aminomethyl)cyclohexane-1-carboxylic acid accumulates in bacterial cells, for example, the bacterial cells can be disrupted by ultrasonication or the like, and the bacterial cells can be removed by centrifugation, and 4-(aminomethyl)cyclohexane-1-carboxylic acid can be collected from the obtained supernatant by an ion exchange resin method or the like.

[Analysis Example] Analysis and quantification of 1,4-bis(aminomethyl)cyclohexane, 4-(aminomethyl)cyclohexane-1-carbaldehyde or 4-(aminomethyl)cyclohexane-1-carboxylic acid

In the examples, analysis and quantification of 1,4-bis(aminomethyl)cyclohexane, 4-(aminomethyl)cyclohexane-1-carbaldehyde or 4-(aminomethyl)cyclohexane-1-carboxylic acid were performed by the following procedure.

The reaction solution containing enzymes after the enzyme reaction, the reaction solution containing microorganisms after the resting bacterial cell reaction or the culture solution containing microorganisms after culture was centrifuged and the supernatant was collected. 1,4-bis(aminomethyl)cyclohexane, 4-(aminomethyl)cyclohexane-1-carbaldehyde or 4-(aminomethyl)cyclohexane-1-carboxylic acid contained in the supernatant was analyzed using an LCMS-8040 (commercially available from Shimadzu Corporation). Under the following analysis conditions, cis-trans isomers of 4-(aminomethyl)cyclohexane-1-carbaldehyde and 4-(aminomethyl)cyclohexane-1-carboxylic acid could be separated.

[Analysis conditions]

Column: Develosil (trademark) ODS-HG 5 $\mu$m 2.0$\times$150 mm (commercially available from Nomura Chemical Co., Ltd.)
Column temperature:40°C

Mobile phases:

(Mobile phase A) 5 mmol/L heptafluorobutyric acid water
(Mobile phase B) 5 mmol/L heptafluorobutyric acid acetonitrile
Mixing ratio of the mobile phases A and B
(0 to 5 min) 100:0
(5 to 25 min) 100:0 to 50:50
(25 to 30 min)50:50
(30 to 31 min) 50:50 to 100:0
(31 to 35 min) 100:0
Flow rate: 0.25 mL/min
Detection: positive mode

Detection ions:

(1,4-bis(aminomethyl)cyclohexane) 143.1>67.0 m/z
(4-(aminomethyl)cyclohexane-1-carbaldehyde) 142.1 m/z
(4-(aminomethyl)cyclohexane-1-carboxylic acid) 158.2>95.0 m/z

Examples will be described below in detail, and the present invention is not limited to these examples.

[Examples]

[Example 1] Search for enzyme (AT) having transamination activity of 1,4-bis(aminomethyl)cyclohexane

(1) Selection of AT to be evaluated

So far, no enzyme having transamination activity of transferring the amino group of 1,4-bis(aminomethyl)cyclohexane to produce 4-(aminomethyl)cyclohexane-1-carbaldehyde is known. In order to search for an enzyme having transamination activity with respect to a non-natural compound 1,4-bis(aminomethyl)cyclohexane, the inventors targeted 33 enzymes annotated as aminotransferases in the genome of *Pseudomonas putida* KT2440 whose genome information was disclosed within *Pseudomonas putida* that can grow in a harsh environment (Applied Microbiology and Biotechnology, 2020, 104:7745-7766), and selected PpAT8 (SEQ ID NO. 1) and PpAT2 (SEQ ID NO. 2) as enzymes that may have desired activity based on a preliminary test using transamination activity as an indicator. In addition, an aminotransferase AsAT5 (SEQ ID NO. 3) derived from *Aeromonas salmonicida subsp. Salmonicida,* which is a homolog enzyme having 65% identity to PpAT8, was set as an evaluation target. In addition, a putrescine aminotransferase PatA derived from *Escherichia coli* K12 MG1655 (SEQ ID NO. 4), which has been reported to have transamination activity with respect to putrescine and cadaverine (Journal of Bacteriology, 2012, 194, 15:4080-4088), which have two amino groups in one molecule like 1,4-bis(aminomethyl)cyclohexane, was set as an evaluation target.

(2) Construction of AT-expressing strain

PCR was performed using DNA consisting of a nucleotide sequence shown in "Primer set" in Table 1 as a primer set and DNA shown in "Template" in Table 1 as a template to obtain amplified DNA fragments. Various chromosomal DNA fragments were prepared by a general method. Here, all gene sequences used in this example were obtained from National Center for Biotechnology Information database (https://www.ncbi.nlm.nih.gov/). In addition, all PCR reactions in this example were performed using PrimeSTAR (trademark) MAX DNA Polymerase (commercially available from Takara Bio Inc.) according to instructions.

[Table 1]

| Primer set (SEQ ID NO.) | Template | Amplified DNA fragment |
|---|---|---|
| 11 and 12 | chromosomal DNA of *Pseudomonas putida* KT2440 | PpAT8 (SEQ ID NO.5) |
| 13 and 14 | chromosomal DNA of *Pseudomonas putida* KT2440 | PpAT2 (SEQ ID NO.6) |
| 15 and 16 | chromosomal DNA of *Aeromonas salmonicida subsp. salmonicida* | AsAT5 (SEQ ID NO.7) |
| 17 and 18 | chromosomal DNA of *Escherichia coli* K12 MG1655 | PatA (SEQ ID NO.8) |

PCR was performed using DNA consisting of nucleotide sequences shown in SEQ ID NOs. 9 and 10 as a primer set, and a plasmid pQE80L (commercially available from QIAGEN) containing a T5 promoter and an N-terminal His tag sequence as a template to obtain vector fragments of about 4.7 kb. Here, the 5' end of the nucleotide sequence shown in SEQ ID NO. 9 contained a sequence complementary to the 5' end of the nucleotide sequences shown in SEQ ID NOs. 11, 13, 15 and 17, and the 5' end of the nucleotide sequence shown in SEQ ID NO. 10 contained a sequence complementary to the 5' end of the nucleotide sequences shown in SEQ ID NOs. 12, 14, 16 and 18.

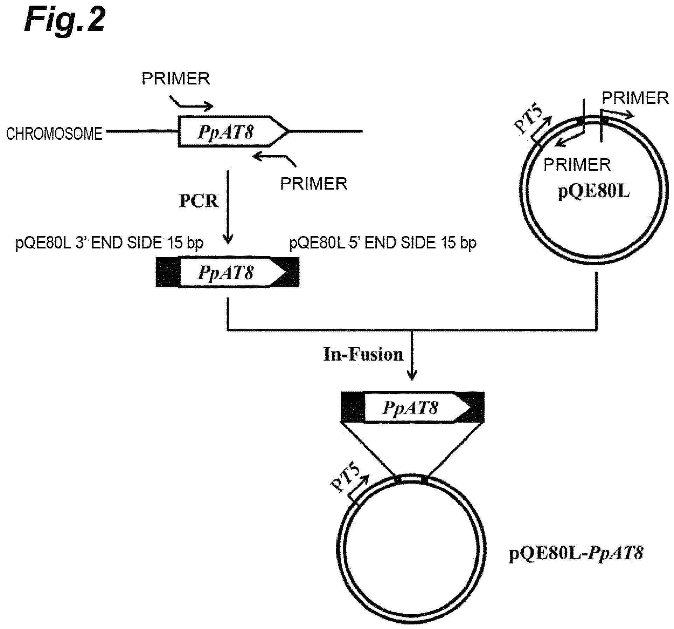

The various amplified DNA fragments and vector fragments obtained above were linked using an In-Fusion HD Cloning Kit (commercially available from Takara Bio Inc.) to construct plasmids pQE80L-PpAT8 (FIG. 2), pQE80L-PpAT2, pQE80L-AsAT5 and pQE80L-PatA, which express various ATs. *Escherichia coli* BL21(DE3) was transformed using the obtained AT-expressing plasmids pQE80L-PpAT8, pQE80L-PpAT2, pQE80L-AsAT5 and pQE80L-PatA to construct recombinant *E. coli* strains, each containing a respective plasmid.

(3) Expression of AT and enzyme purification

Various recombinant *E. coli* strains obtained in the above (2) were inoculated in a test tube containing a 2 mL LB medium containing 100 mg/L ampicillin, and cultured with shaking at 30°C for 16 hours. The culture solution was inoculated in a 250 mL Erlenmeyer flask containing a 40 mL LB medium containing 100 mg/L ampicillin and cultured with shaking at 30°C for 2 hours, Isopropyl-$\beta$-D-thiogalactopyranoside (IPTG) was then added to a final concentration of 1 mM, and the mixture was additionally cultured with shaking at 30°C for 5 hours. The culture solution was centrifuged to obtain wet bacterial cells. Enzymes were purified from the wet bacterial cells using TALON (trademark) Metal Affinity Resin (commercially available from Clontech) to obtain purified His-tagged recombinant type enzymes PpAT8, PpAT2, AsAT5 and PatA.

(4) Evaluation of transamination activity with respect to 1,4-bis(aminomethyl)cyclohexane

For the purified enzymes purified in (3), the enzyme activity of various ATs was evaluated using the amount of 4-(aminomethyl)cyclohexane-1-carbaldehyde produced as an indicator.

A 0.1 mL reaction solution (pH 7.8) containing 0.07 mg of the purified enzymes (PpAT8, PpAT2, AsAT5 or PatA) obtained in the above (3), 50 mM MOPS (pH 7.8), 10 mM magnesium chloride, 1 mM Dithiothreitol (DTT), 10 mM pyruvic acid, 0.1 mM Pyridoxal phosphate (PLP), and a 10 mM mixture of cis- and trans-1,4-bis(aminomethyl)cyclohexane (a trans proportion of 45.7%) was prepared and reacted under conditions of 30°C and 400 rpm for 24 hours. In addition, as a negative control, the reaction was performed in the same manner using a reaction solution to which an equal volume of a MOPS buffer solution was added instead of the purified enzyme solution.

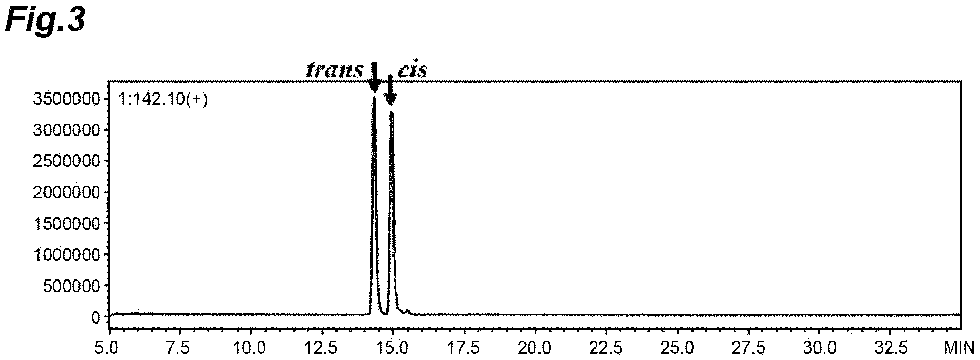

After the reaction was completed, the reaction solution was diluted and then centrifuged, and the reaction product and residual substrate contained in the supernatant were analyzed by LCMS-8040 (FIG. 3). Each peak of the trans- or cis-4-(aminomethyl)cyclohexane-1-carbaldehyde product was comprehensively determined from its elution position and the m/z value, and the amount of each product was showed by a peak area (Area value). The results are shown in Table 2.

[Table 2]

| AT | Residual substrate (mM) | Amount of 4-(aminomethyl)cyclohexane-1-carbaldehyde produced (Area value) | |
|---|---|---|---|
| | | Trans-form | Cis-form |
| -(negative control) | 9.2 | N.D. | N.D. |
| PpAT8 | 7.3 | 16079356 | 12472505 |
| PpAT2 | 9.0 | 3194819 | 2482301 |
| AsAT5 | 9.0 | 8037766 | 6369141 |
| PatA | 4.6 | 29942199 | 29023216 |

It was confirmed that 4-(aminomethyl)cyclohexane-1-carbaldehyde was produced and accumulated only when PpAT8, PpAT2, AsAT5 or PatA was added and reacted. Particularly, PatA had the smallest amount of the residual substrate and produced the largest amount of 4-(aminomethyl)cyclohexane-1-carbaldehyde. Accordingly, it was found that putrescine aminotransferase PatA derived from *Escherichia coli* K12 MG1655 had high transamination activity for 1,4-bis(aminomethyl)cyclohexane.

<Evaluation of transamination activity of AT homolog with respect to 1,4-bis(aminomethyl)cyclohexane>

(5) Search for AT homolog enzyme in database

Using the amino acid sequences (SEQ ID NOs. 5 and 8) of PpAT8 and PatA obtained in Example 1 as ATs having activity for 1,4-bis(aminomethyl)cyclohexane as queries, enzymes having 40 to 85% identity to each query enzyme were searched for using the homology search function of the BLAST (https://blast.ncbi.nlm.nih.gov/Blast.cgi) protein sequence database from the National Center for Biotechnology Information. The amino acid sequences (SEQ ID NOs. 103 to 107) of enzymes extracted by the above operation and the nucleotide sequences (SEQ ID NOs. 108 to 112) encoding the enzymes are shown in Table 3.

[Table 3]

| Query | Homolog | Identity (%) | Microorganisms of origin | Amino acid sequence | Nucleotide sequence |
|---|---|---|---|---|---|
| PpAT8 | PcAT | 84.8 | *Pseudomonas chlororaphis subsp. aureofaciens* NBRC 3521 | SEQ ID NO. 103 | SEQ ID NO. 108 |
| | PaAT | 76.4 | *Pseudomonas aeruginosa* PAO1 | SEQ ID NO. 104 | SEQ ID NO. 109 |
| | HeAT1 | 63.4 | *Halomonas elongata* DSM 2581 | SEQ ID NO. 105 | SEQ ID NO. 110 |
| | HeAT2 | 41.2 | *Halomonas elongata* DSM 2581 | SEQ ID NO. 106 | SEQ ID NO. 111 |

(continued)

| Query | Homolog | Identity (%) | Microorganisms of origin | Amino acid sequence | Nucleotide sequence |
|---|---|---|---|---|---|
| PatA | MaAT | 41.5 | *Methanosarcina acetivorans* C2A | SEQ ID NO. 107 | SEQ ID NO. 112 |

(6) Construction of AT homolog expressing strain

In order to obtain gene fragments of each AT homolog enzyme shown in Table 3, a PCR reaction was performed using the template and primers 1 and 2 shown in Table 4. Various chromosomal DNA fragments were prepared by a general method. In this case, the 5' end of the nucleotide sequence shown in SEQ ID NO. 9 contained a nucleotide sequence complementary to the 5' end of the nucleotide sequence shown in each primer 1 in Table 4, and the 5' end of the nucleotide sequence shown in SEQ ID NO. 10 contained a nucleotide sequence complementary to the 5' end of the nucleotide sequence shown in each primer 2 in Table 4.

[Table 4]

| Query | Homolog | Template | Primer 1 | Primer 2 |
|---|---|---|---|---|
| PpAT8 | PcAT | chromosomal DNA of *Pseudomonas chlororaphis subsp. aureofaciens* NBRC 3521 | SEQ ID NO. 113 | SEQ ID NO. 114 |
| | PaAT | chromosomal DNA of *Pseudomonas aeruginosa* PAO1 | SEQ ID NO. 115 | SEQ ID NO. 1116 |
| | HeAT1 | chromosomal DNA of *Halomonas elongata* DSM 2581 | SEQ ID NO. 117 | SEQ ID NO. 118 |
| | HeAT2 | chromosomal DNA of *Halomonas elongata* DSM 2581 | SEQ ID NO. 119 | SEQ ID NO. 120 |
| PatA | MaAT | chromosomal DNA of *Methanosarcina acetivorans* C2A | SEQ ID NO. 121 | SEQ ID NO. 122 |

The amplified DNA fragments obtained by the above PCR and the pQE80L vector fragments prepared in Example 1 were linked using an In-Fusion (trademark) HD Cloning Kit (commercially available from Takara Bio Inc.) to construct each AT-expressing plasmid. *Escherichia coli* BL21(DE3) was transformed using the obtained expression plasmids to construct recombinant *E. coli* having each AT-expressing plasmid.

(7) Evaluation of transamination activity of AT homolog with respect to 1,4-bis(aminomethyl)cyclohexane

Each His-tagged recombinant type AT purified enzyme was obtained in the same method as in (3) using the recombinant *E. coli* having each AT-expressing plasmid obtained in the above (6).
A 0.1 mL reaction solution (pH 7.8) containing 0.07 mg of each purified enzyme obtained, 50 mM MOPS (pH 7.8), 1 mM magnesium chloride, 1 mM DTT, 1 mM pyruvic acid, 0.1 mM PLP, and a 1 mM mixture of cis- and trans-1,4-bis(aminomethyl)cyclohexane (a trans proportion of 45.7%) was prepared and reacted under conditions of 30°C and 400 rpm for 24 hours. In addition, the reaction was performed in the same manner using a reaction solution to which an equal volume of a MOPS buffer solution was added instead of the purified enzyme as a negative control, and a reaction solution using the purified PatA enzyme as a positive control.
After the reaction was completed, the reaction product was analyzed in the same method as in (4). The results are shown in Table 5.

[Table 5]

| AT | Residual substrate (mM) | Amount of 4-(aminomethyl)cyclohexane-1-carbaldehyde produced (Area value) | |
|---|---|---|---|
| | | Trans-form | Cis-form |
| -(negative control) | 1.26 | N.D. | N.D. |
| PcAT | 1.01 | 1972463 | 4143361 |

(continued)

| AT | Residual substrate (mM) | Amount of 4-(aminomethyl)cyclohexane-1-carbaldehyde produced (Area value) | |
|---|---|---|---|
| | | Trans-form | Cis-form |
| PaAT | 0.76 | 2156060 | 8886741 |
| HeAT1 | 0.87 | 831663 | 3931875 |
| HeAT2 | 1.22 | N.D. | N.D. |
| MaAT | 1.29 | N.D. | N.D. |
| PatA (positive control) | 0.75 | 2085188 | 9881719 |

It was confirmed that PcAT, PaAT and HeAT1 had transamination activity for 1,4-bis(aminomethyl)cyclohexane and produced and accumulated 4-(aminomethyl)cyclohexane-1-carbaldehyde. Particularly, PaAT was found to have activity comparable to that of PatA. In addition, it was suggested that the PpAT8 homolog having 60% or more identity to PpAT8 had desired activity.

[Example 2] Influence of pH on step of producing 4-(aminomethyl)cyclohexane-1-carbaldehyde

(1) Evaluation of influence of solution pH on transamination reaction

The influence of a pH condition (pH 7.0 or pH 9.0) on the supply of 4-(aminomethyl)cyclohexane-1-carbaldehyde, which is an intermediate of 4-(aminomethyl)cyclohexane-1-carboxylic acid, was evaluated.

A 0.1 mL reaction solution containing 0.07 mg of the purified PatA enzyme obtained in Example 1(3), 50 mM MOPS (pH 7.0) or 50 mM CHES (pH 9.0), 1 mM magnesium chloride, 1 mM DTT, 1 mM pyruvic acid, 0.1 mM PLP, and 1 mM cis- or trans-1,4-bis(aminomethyl)cyclohexane as a reaction substrate was prepared and reacted under conditions of 30°C and 400 rpm for 24 hours.

After the reaction was completed, 4-(aminomethyl)cyclohexane-1-carbaldehyde contained in the supernatant of the reaction solution was analyzed using LCMS-8040 (commercially available from Shimadzu Corporation). The trans proportion of 4-(aminomethyl)cyclohexane-1-carbaldehyde (intermediate trans proportion) was calculated using a calculation formula shown in Math. 1 based on the Area value of the produced 4-(aminomethyl)cyclohexane-1-carbaldehyde. In the formula, the trans-form was trans-4-(aminomethyl)cyclohexane-1-carbaldehyde, and the cis-form was cis-4-(aminomethyl)cyclohexane-1-carbaldehyde. The results are shown in Table 6.

[Math. 1]

$$\text{Intermediate trans proportion (\%)} = \{\text{Area value of trans-form}/(\text{Area value of trans-form} + \text{Area value of cis-form})\} \times 100$$

[Table 6]

| Substrate | pH | Amount of 4-(aminomethyl)cyclohexane-1-carbaldehyde produced (Area value) | | Intermediate trans proportion (%) |
|---|---|---|---|---|
| | | trans | cis | |
| Cis-form | 7.0 | 240436 | 14223981 | 1.7 |
| | 9.0 | 2210679 | 3469878 | 38.9 |
| Trans-form | 7.0 | 3795074 | 207188 | 94.8 |
| | 9.0 | 3851428 | 1671628 | 69.7 |

It was found that, regardless of whether a cis-substrate or a trans-substrate was used, at pH 7.0, about 95% or more of the produced 4-(aminomethyl)cyclohexane-1-carbaldehyde had the same three-dimensional structure as the added substrate. On the other hand, at pH 9.0, 30% or more of 4-(aminomethyl)cyclohexane-1-carbaldehyde, which had an isomer structure of the added substrate, was produced.

These results suggest that, in the transamination reaction of 1,4-bis(aminomethyl)cyclohexane, the three-dimensional structure of 4-(aminomethyl)cyclohexane-1-carbaldehyde produced changed depending on the pH of the reaction solution.

The change in the above three-dimensional structure was thought to be due to (i) the action of aminotransferase or (ii) keto-enol tautomerism of the aldehyde group. Regarding (ii), 4-(aminomethyl)cyclohexane-1-carbaldehyde has an aldehyde group, and it is generally known that an aldehyde group can be interconverted between a keto form and an enol form in the presence of an acidic or basic catalyst (keto-enol tautomerism). It was thought that the aldehyde group of 4-(aminomethyl)cyclohexane-1-carbaldehyde was converted from a keto form to an enol form, a hydrogen atom at the $\alpha$-carbon atom was transferred, a carbon-carbon double bond was formed with cyclohexane, and thus it became not possible to distinguish between a cis-form and a trans-form. Therefore, in the presence of an acid or a base, independently of the aminotransferase reaction, the isomerization reaction of 4-(aminomethyl)cyclohexane-1-carbaldehyde was expected to occur. Therefore, the following test was performed in order to confirm whether the change in the three-dimensional structure depended on an aminotransferase.

(2) Evaluation of pH-dependent isomerization of 4-(aminomethyl)cyclohexane-1-carbaldehyde

According to the above (1), (i) a reaction solution in which trans proportion of the intermediate became 2.1% by reacting the purified PatA enzyme with the cis-substrate under a pH 7.0 condition and (ii) a reaction solution in which trans proportion of the intermediate became 95.3% by reacting the purified PatA enzyme and the trans-substrate under a pH 7.0 condition were prepared. The enzymes in the reaction solutions were removed using Amicon (trademark) Ultra 0.5 mL, 3K (commercially available from Millipore) and flow-through fractions were collected. 90 μL of any of a 50 mM sodium acetate buffer solution (pH 4.0 or pH 5.0), a 50 mM MES buffer solution (pH 6.0), a 50 mM sodium phosphate buffer solution (pH 6.0 or pH 7.0), a 50 mM MOPS buffer solution (pH 7.0), a 50 mM CHES buffer solution (pH 9.0 or 10.0), and a 50 mM sodium carbonate buffer solution (pH 9.0 or 10.0) was added to 10 μL of each flow-through fraction, and the mixture was reacted under conditions of 30°C and 400 rpm for 20 hours.

In addition, in order to evaluate the influence of adding a secondary amine on isomerization, the reaction was also performed in the same manner under the conditions in which 50 mM L-proline was added to the composition. After the reaction was completed, the reaction product was analyzed by the same method as in the above (1), and the intermediate trans proportion was calculated. The results are shown in Table 7.

[Table 7]

| Substrate | pH | Buffer solution | L-proline | Intermediate trans proportion (%) |
|---|---|---|---|---|
| Cis-form | 4 | sodium acetate | - | 1.7 |
| | 5 | sodium acetate | - | 3.1 |
| | 6 | MES | - | 3.2 |
| | | sodium phosphate | - | 7.3 |
| | | sodium phosphate | + | 10.0 |
| | 7 | MOPS | - | 3.6 |
| | | sodium phosphate | - | 16.3 |
| | | sodium phosphate | + | 24.0 |
| | 9 | CHES | - | 47.8 |
| | | sodium bicarbonate | - | 22.6 |
| | | sodium bicarbonate | + | 63.4 |
| | 10 | CHES | - | 63.4 |
| | | sodium bicarbonate | - | 63.1 |
| | | sodium bicarbonate | + | 66.5 |
| | 4 | sodium acetate | - | 100 |

(continued)

| Substrate | pH | Buffer solution | L-proline | Intermediate trans proportion (%) |
|---|---|---|---|---|
| Trans-form | 5 | sodium acetate | - | 100 |
| | 6 | MES | - | 91.1 |
| | | sodium phosphate | - | 87.2 |
| | | sodium phosphate | + | 88.8 |
| | 7 | MOPS | - | 94.5 |
| | | sodium phosphate | - | 85.0 |
| | | sodium phosphate | + | 75.7 |
| | 9 | CHES | - | 67.9 |
| | | sodium bicarbonate | - | 79.5 |
| | | sodium bicarbonate | + | 60.8 |
| | 10 | CHES | - | 63.9 |
| | | sodium bicarbonate | - | 64.7 |
| | | sodium bicarbonate | + | 65.1 |

As shown in Table 7, whether a cis-substrate or a trans-substrate was used, almost no isomerization of the intermediate occurred under acidic conditions of pH 4 to 6, but a change in the intermediate trans proportion was observed at pH 7 or more. Particularly, under basic conditions of pH 9 to 10, the isomerization of the intermediate significantly proceeded. In addition, it was found that the isomerization of 4-(aminomethyl)cyclohexane-1-carbaldehyde reached equilibrium at a trans proportion of about 60%.

In addition, it was found that, under the proline addition condition, almost no change was observed at pH 4 to 6, but at pH 7, the isomerization of the intermediate tended to proceed, and at pH 9, the isomerization of the intermediate was further promoted.

From the above, it was clearly understood that the isomerization of 4-(aminomethyl)cyclohexane-1-carbaldehyde occurred under neutral or basic conditions regardless of the presence of an enzyme. In addition, it was found that the isomerization reaction under neutral or basic conditions was promoted in the coexistence of a secondary amine such as L-proline.

The above results suggest the three-dimensional structure pattern of the reaction product could be controlled by changing the pH condition according to the three-dimensional structure of the substrate used.

[Example 3] Evaluation of activity of aldehyde dehydrogenase (ALDH)

(1) Selection of ALDH to be evaluated

So far, no enzyme having aldehyde dehydrogenase activity of oxidizing an aldehyde group of 4-(aminomethyl)cyclohexane-1-carbaldehyde to produce 4-(aminomethyl)cyclohexane carboxylic acid is known.

In the present invention, based on the results of Example 1, the NAD$^+$-dependent gamma-aminobutyraldehyde dehydrogenase PatD (FEBS Letters, 2005, 579:4107-4112) derived from *Escherichia coli* K12 MG1655, which is involved in the metabolism of putrescine and cadaverine like PatA, was selected as an evaluation target ALDH. In addition, based on the fact that the substrate 4-(aminomethyl)cyclohexane-1-carbaldehyde has a bulky and low polarity cyclohexane ring structure, NAD$^+$-dependent benzaldehyde dehydrogenase XylC derived from *Pseudomonas putida* CSV86 (Arch.Microbiol., 2011, 193:553-563), NAD$^+$-dependent phenylacetaldehyde dehydrogenase StyD derived from *Pseudomonas putida* S12 (Archives of Biochemistry and Biophysics, 2017, 616:47-58) and NADP$^+$-dependent 4-hydroxybenzaldehyde dehydrogenase PchA derived from *Pseudomonas putida* NCIMB 9866 (Appl. Microbiol. Biotechnol., 2014, 98:1349-1356), which have been reported to have ALDH activity for substrates having a bulky and low polarity benzene ring structure, although the three-dimensional structure was different, were selected as evaluation target ALDHs.

(2) Construction of ALDH-expressing strain

The amino acid sequences of the ALDHs (PatD, XylC, StyD and PchA) selected in (1) are shown in SEQ ID NOs. 19 to 22. The nucleotide sequences encoding these enzymes are shown in SEQ ID NOs. 23 to 26. Strains expressing these enzymes were constructed as follows.

PCR was performed using DNA shown in "Template" in Table 8 as a template, and DNA consisting of a nucleotide sequence shown in "Primer set" in Table 8 as a primer set, and DNA fragments were amplified.

[Table 8]

| Primer set (SEQ ID NO.) | Template | Amplified DNA fragment |
|---|---|---|
| 27 and 28 | chromosomal DNA of *Escherichia coli* K12 MG1655 | PatD (SEQ ID NO. 23) |
| 29 and 30 | DNA shown in SEQ ID NO. 24 | XylC (SEQ ID NO. 24) |
| 31 and 32 | DNA shown in SEQ ID NO. 25 | StyD (SEQ ID NO. 25) |
| 33 and 34 | DNA shown in SEQ ID NO. 26 | PchA (SEQ ID NO. 26) |

The chromosomal DNA of *Escherichia coli* K12 MG1655 was prepared by a general method. DNA shown in SEQ ID NO. 24 had a nucleotide sequence of the gene encoding XylC derived from *Pseudomonas putida* CSV86 shown in SEQ ID NO. 20 and prepared by artificial synthesis. DNA shown in SEQ ID NO. 25 had a nucleotide sequence of the gene encoding StyD derived from *Pseudomonas putida* S12 shown in SEQ ID NO. 21, and prepared by artificial synthesis. DNA shown in SEQ ID NO. 26 had a nucleotide sequence of the gene encoding PchA derived from *Pseudomonas putida* NCIMB 9866 shown in SEQ ID NO. 22, and prepared by artificial synthesis.

The 5' end of the nucleotide sequence shown in SEQ ID NO. 9 contained a sequence complementary to the 5' end of SEQ ID NOs. 27, 29, 31 and 33, and the 5' end of the nucleotide sequence shown in SEQ ID NO. 10 contained a sequence complementary to the 5' end of the nucleotide sequences shown in SEQ ID NOs. 28, 30, 32 and 34.

The various amplified DNA fragments obtained above and the pQE80L vector fragments prepared in Example 1 were linked using an In-Fusion HD Cloning Kit (commercially available from Takara Bio Inc.) to construct plasmids expressing various ALDHs: pQE80L-PatD, pQE80L-XylC, pQE80L-StyD and pQE80L-PchA.

*Escherichia coli* BL21(DE3) was transformed using the ALDH expressing plasmids pQE80L-PatD, pQE80L-XylC, pQE80L-StyD and pQE80L-PchA obtained above to construct recombinant *E. coli* strains, each containing a respective plasmid.

(3) Expression of ALDH and enzyme purification

Using the bacteria strain obtained in the above (2), wet bacterial cells were obtained in the same method as in Example 1, and enzyme purification was performed to obtain His-tagged recombinant type purified enzymes PatD, XylC, StyD and PchA.

(4) Evaluation of productivity of 4-(aminomethyl)cyclohexane-1-carboxylic acid by ALDH combination reaction with AT

<When NAD$^+$ is used as a coenzyme for ALDH>

A 0.1 mL reaction solution (pH 7.8) containing 0.07 mg of the purified enzyme (PatD, XylC, StyD and PchA) obtained in the above (3), 0.07 mg of PatA obtained in Example 1(3), 50 mM MOPS (pH 7.8), 1 mM magnesium chloride, 1 mM DTT, 1 mM pyruvic acid, 0.1 mM PLP, 2 mM nicotinamide adenine dinucleotide (NAD$^+$), and a 1 mM mixture of cis- and trans-1,4-bis(aminomethyl)cyclohexane (a trans proportion of 45.7%) was prepared and reacted under conditions of 30°C and 400 rpm for 24 hours. In addition, as a negative control, the reaction was performed in the same manner using a reaction solution to which an equal volume of a MOPS buffer solution was added instead of the purified enzyme.

After the reaction was completed, the reaction product was analyzed in the same method as in Example 1. In addition, in order to confirm whether tranexamic acid (TXA) or cis-TXA could be selectively produced from the produced 4-(aminomethyl)cyclohexane-1-carboxylic acid, the trans proportion (also referred to as an AMCHA trans proportion or an AMCHA trans percentage) of 4-(aminomethyl)cyclohexane-1-carboxylic acid was calculated using a calculation formula shown in the following Math. 2 based on the amount of TXA and cis-TXA produced. Here, the concentration (mM) of each compound was calculated by comparison with the Area value of a sample with a known concentration.

AMCHA trans percentage (%)={TXA (mM)/(TXA (mM) + cis-TXA (mM))}×100       [Math. 2]

First, Table 9 shows the results when a mixture of cis- and trans-1,4-bis(aminomethyl)cyclohexane (a trans proportion of 45.7%) was used as the substrate.

[Table 9]

| ALDH | Amount produced (mM) | | AMCHA trans proportion (%) |
|---|---|---|---|
| | TXA | cis-TXA | |
| -(negative control) | N.D. | N.D. | - |
| PatD | 0.38 | 0.70 | 35 |
| Xy1C | 0.46 | 0.10 | 82 |
| StyD | 0.37 | 0.49 | 43 |
| N.D.: qualitative limit or below (S/N<3) | | | |

Based on Table 9, it was found that, by combining PatA with PatD, XylC or StyD, 4-(aminomethyl)cyclohexane-1-carboxylic acid could be produced and accumulated using 1,4-bis(aminomethyl)cyclohexane as a substrate.

In addition, because a mixture of a cis-form and a trans-form was used as the substrate, it was expected that the product 4-(aminomethyl)cyclohexane-1-carboxylic acid would be a mixture of a cis-form and a trans-form. However, in reality, the trans proportion of 4-(aminomethyl)cyclohexane carboxylic acid produced differed depending on the type of ALDH in the results. These results suggest that the ALDH had substrate selectivity, and particularly, when XylC was used, the trans proportion of 4-(aminomethyl)cyclohexane carboxylic acid was high, indicating that the ALDH could selectively react with the trans-4-(aminomethyl)cyclohexane-1-carbaldehyde. Therefore, XylC can be said to be a useful ALDH for efficiently producing TXA from a mixture substrate of a cis-form and a trans-form.

Next, Table 10 shows the results when only trans-1,4-bis(aminomethyl)cyclohexane was used as the substrate.

[Table 10]

| ALDH | Amount produced (mM) | |
|---|---|---|
| | TXA | cis-TXA |
| - (negative control) | N.D. | N.D. |
| PatD | 0.88 | N.Q. |
| XylC | 0.72 | N.D. |
| StyD | 0.83 | N.D. |
| N.Q.: quantitative limit or below (S/N<10), N.D.: qualitative limit or below (S/N<3) | | |

Based on the results in Table 10, when the trans-form was used as the substrate, the production of TXA was confirmed for all ALDHs, but cis-TXA was hardly detected. Therefore, the results suggest that, when a trans-substrate was used, TXA could be produced at a high yield for all ALDHs found here, and thus it was desirable to use a trans-form as a substrate or a substrate containing a large amount of a trans-form in the TXA production. In addition, it was found that these ALDHs could accept trans-substrates.

Subsequently, the amount of 4-(aminomethyl)cyclohexane carboxylic acid produced when only cis-1,4-bis(aminomethyl)cyclohexane was used as the substrate was evaluated.

A 0.1 mL reaction solution containing 0.07 mg of each of the purified enzyme PatA and various ALDHs (PatD, XylC or StyD) obtained in the same manner as above, 50 mM MOPS (pH 7.8), 10 mM magnesium chloride, 1 mM DTT, 10 mM pyruvic acid, 0.1 mM PLP, 2 mM NAD$^+$, and 10 mM cis-1,4-bis(aminomethyl)cyclohexane was prepared and reacted under conditions of 30°C and 400 rpm for 24 hours. The results are shown in Table 11.

[Table 11]

| ALDH | Amount produced (mM) | |
|---|---|---|
| | TXA | cis-TXA |
| - (negative control) | N.D. | N.D. |
| PatD | 0.01 | 1.79 |
| XylC | 0.46 | 0.90 |

(continued)

| ALDH | Amount produced (mM) | |
|---|---|---|
| | TXA | cis-TXA |
| - (negative control) | N.D. | N.D. |
| StyD | 0.06 | 1.64 |
| N.D.: qualitative limit or below (S/N<3) | | |

As shown in Table 11, it was found that, when a cis-form was used as the substrate, the proportion of cis-TXA produced was high in all ALDHs. In addition, it was found that, since the amount of cis-TXA produced with XylC was small, XylC did not readily accept the cis-substrate.

Particularly, TXA was produced at a relatively high proportion only when XylC was used. It is speculated that, because this reaction was performed under weakly basic conditions at pH 7.8, the isomerization of the intermediate occurred under basic conditions shown in Example 2, and even when the cis-substrate was added, the trans-intermediate was produced, and as a result, a large amount of TXA accumulated. This suggests that, when the production of TXA was desired, by using XylC as ALDH, the trans-intermediate was selectively oxidized to produced TXA, and at the same time, the remaining cis-intermediate was dynamically isomerized to the trans-form under neutral or basic conditions, and even when a mixture substrate of a cis-form and a trans-form was used, it was theoretically possible to produce 100% TXA.

<When $NADP^+$ is used as coenzyme for ALDH>

PchA, for which ALDH activity was not confirmed in the results of Table 6, is known to use $NADP^+$ as a coenzyme (Appl. Microbiol. Biotechnol., 2014, 98:1349-1356). Therefore, the aldehyde oxidation activity of 4-(aminomethyl) cyclohexane-1-carbaldehyde when the purified enzyme PchA obtained in the above (3), XylC was used as a comparison target, and $NADP^+$ was used as a coenzyme was evaluated.

A reaction solution in which 1 mM $NADP^+$ was added instead of $NAD^+$ in the above reaction solution composition was prepared, and the same reaction as above was performed. As the substrate, a mixture of a cis-form and a trans-form (a trans proportion of 45.7%) was used. In addition, as a negative control, the reaction was performed in the same manner using a reaction solution to which an equal volume of a MOPS buffer solution was added instead of the purified enzyme.

After the reaction was completed, the reaction product was analyzed in in the same method as above. The results are shown in Table 12.

[Table 12]

| ALDH | Amount produced (mM) | | AMCH Atrans proportion (%) |
|---|---|---|---|
| | TXA | cis-TXA | |
| - (negative control) | N.D. | N.D. | - |
| PchA | 0.06 | 0.16 | 27 |
| XylC | 0.17 | 0.01 | 94 |
| N.D.: qualitative limit or below (S/N<3) | | | |

As shown in Table 12, it was confirmed that PchA could produce 4-(aminomethyl)cyclohexane-1-carboxylic acid when $NADP^+$ was added as a coenzyme. In addition, although XylC is known as an $NAD^+$-dependent enzyme, it was found that $NADP^+$ could also be used as a coenzyme in the production of 4-(aminomethyl)cyclohexane-1-carboxylic acid.

[Example 4] Production of 4-(aminomethyl)cyclohexane carboxylic acid using ALDH homolog

(1) Search for ALDH homolog enzyme in database

Using the amino acid sequences (SEQ ID Nos. 19 to 21) of PatD, XylC and StyD obtained in Example 3 as ALDH which enables 4-(aminomethyl)cyclohexane-1-carboxylic acid to be produced as queries, enzymes having 50 to 80% identity to each enzyme were searched for using the homology search function of the BLAST (https://blast.ncbi.nlm.nih.gov/Blast.cgi) protein sequence database from the National Center for Biotechnology Information. The amino acid sequences (SEQ ID Nos. 35 to 46) of the enzymes extracted by the above operation and the nucleotide sequences (SEQ ID Nos. 47 to 58) encoding the enzymes are shown in Table 13. In Table 13, CkpatD, SepatD, CspatD and PppatD were all proteins annotated as gamma-aminobutyraldehyde dehydrogenases. SsBD, HaBD and RrAD were proteins annotated as benzaldehyde dehydrogenases. PmsLAD was a protein annotated as a phenylacetaldehyde dehydrogenase. The others were proteins annotated as aldehyde dehydrogenase or their family proteins.

[Table 13]

| Query | Homolog | Identity (%) | Microorganism origin | Amino acid sequence | Nucleotide sequence |
|---|---|---|---|---|---|
| PatD | CkpatD | 88.4 | *Citrobacter koseri* ATCC BAA-895 | SEQ ID NO. 35 | SEQ ID NO. 47 |
| | SepatD | 83.3 | *Salmonella enterica subsp. Enterica serovar Choleraesuis str.* SC-B67 | SEQ ID NO. 36 | SEQ ID NO. 48 |
| | PapatD | 73.0 | *Pectobacterium atrosepticum* SCRI1043 | SEQ ID NO. 37 | SEQ ID NO. 49 |
| | CspatD | 83.3 | *Cronobacter sakazakii* ATCC 29544 | SEQ ID NO. 38 | SEQ ID NO. 50 |
| | PppatD | 71.1 | *Pseudomonas putida* KT2440 | SEQ ID NO. 39 | SEQ ID NO. 51 |
| XylC | HaBD | 84.2 | *Halioxenophilus aromaticivorans* | SEQ ID NO. 40 | SEQ ID NO. 52 |
| | SsBD | 72.4 | *Sphingomonas sp.* TF3 | SEQ ID NO. 41 | SEQ ID NO. 53 |
| | RiAD | 48.4 | *Rhodococcus imtechensis* | SEQ ID NO. 42 | SEQ ID NO. 54 |
| | RrAD | 49.7 | *Rhodococcus ruber* | SEQ ID NO. 43 | SEQ ID NO. 55 |
| StyD | PmsLAD | 86.9 | *Pseudomonas sp.* LQ26 | SEQ ID NO. 44 | SEQ ID NO. 56 |
| | BlAD | 69.9 | *Burkholderia lata* | SEQ ID NO. 45 | SEQ ID NO. 57 |
| | PbsAD | 63.3 | *Paraburkholderia sp.* 5N | SEQ ID NO. 46 | SEQ ID NO. 58 |

(2) Construction of ALDH homolog expressing strain

In order to obtain gene fragments of ALDH homolog enzymes shown in Table 13, a PCR reaction was performed using the templates and primers 1 and 2 shown in Table 14.

[Table 14]

| Query | Homolog | Template | Primer 1 | Primer 2 |
|---|---|---|---|---|
| PatD | CkpatD | SEQ ID NO. 47 | SEQ ID NO. 59 | SEQ ID NO. 60 |
| | SepatD | SEQ ID NO. 48 | SEQ ID NO. 61 | SEQ ID NO. 62 |
| | PapatD | SEQ ID NO. 49 | SEQ ID NO. 63 | SEQ ID NO. 64 |
| | CspatD | chromosomal DNAof *Cronobacter sakazakii* | SEQ ID NO. 65 | SEQ ID NO. 66 |
| | PppatD | chromosomal DNA of *Pseudomonas putida* KT2440 | SEQ ID NO. 67 | SEQ ID NO. 68 |
| XylC | HaBD | SEQ ID NO. 52 | SEQ ID NO. 69 | SEQ ID NO. 70 |
| | SsBD | SEQ ID NO. 53 | SEQ ID NO. 71 | SEQ ID NO. 72 |
| | RiAD | chromosomal DNA of *Rhodococcus imtechensis* | SEQ ID NO. 73 | SEQ ID NO. 74 |
| | RrAD | chromosomal DNA of *Rhodococcus ruber* | SEQ ID NO. 75 | SEQ ID NO. 76 |
| StyD | PmsLAD | SEQ ID NO. 56 | SEQ ID NO. 77 | SEQ ID NO. 78 |
| | BlAD | SEQ ID NO. 57 | SEQ ID NO. 79 | SEQ ID NO. 80 |
| | PbsAD | SEQ ID NO. 58 | SEQ ID NO. 81 | SEQ ID NO. 82 |

Various chromosomal DNA fragments were prepared by a general method. DNA shown in SEQ ID NO. 47 was DNA in which the nucleotide sequence of the gene encoding ALDH derived from *Citrobacter koseri* ATCC BAA-895 strain shown in SEQ ID NO. 35 was codon-optimized for expression in *E. coli,* and prepared by artificial synthesis. DNA shown in SEQ ID NO. 48 was DNA in which the nucleotide sequence of the gene encoding ALDH derived from *Salmonella enterica subsp.enterica serovar Choleraesuis str.* SC-B67 strain shown in SEQ ID NO. 36 was codon-optimized for expression in *E. coli,* and prepared by artificial synthesis. DNA shown in SEQ ID NO. 49 was DNA in which the nucleotide sequence of the gene encoding ALDH derived from *Pectobacterium atrosepticum* SCRI1043 strain shown in SEQ ID NO. 37 was codon-optimized for expression in *E. coli,* and prepared by artificial synthesis. DNA shown in SEQ ID NO. 52 was DNA in which the nucleotide sequence of the gene encoding ALDH derived from *Halioxenophilus aromaticivorans* strain shown in SEQ ID NO. 40 was codon-optimized for expression in *E. coli,* and prepared by artificial synthesis. DNA shown in SEQ ID NO. 53 was DNA in which the nucleotide sequence of the gene encoding ALDH derived from *Sphingomonas sp.* TF3 strain shown in SEQ ID NO. 41 was codon-optimized for expression in *E. coli,* and prepared by artificial synthesis. DNA shown in SEQ ID NO. 56 was DNA in which the nucleotide sequence of the gene encoding ALDH derived from *Pseudomonas* sp.LQ26 strain shown in SEQ ID NO. 44 was codon-optimized for expression in *E. coli,* and prepared by artificial synthesis. DNA shown in SEQ ID NO. 57 was DNA in which the nucleotide sequence of the gene encoding ALDH derived from *Burkholderia lata* strain shown in SEQ ID NO. 45 was codon-optimized for expression in *E. coli,* and prepared by artificial synthesis. DNA shown in SEQ ID NO. 58 was DNA in which the nucleotide sequence of the gene encoding ALDH derived from *Paraburkholderia sp.* 5N strain shown in SEQ ID NO. 46 was codon-optimized for expression in *E. coli,* and prepared by artificial synthesis.

In this case, the nucleotide sequences shown in SEQ ID NO. 9 and each primer 1 in Table 14, and the nucleotide sequences shown in SEQ ID NO. 10 and each primer 2 in Table 14 each contained a complementary nucleotide sequence at their 5' ends. That is, the 5' end of the nucleotide sequence shown in SEQ ID NO. 9 contained a nucleotide sequence complementary to the 5' end of the nucleotide sequence shown in each primer 1 in Table 14, and the 5' end of the nucleotide sequence shown in SEQ ID NO. 10 contained a nucleotide sequence complementary to the 5' end of the nucleotide sequence shown in each primer 2 in Table 14.

The amplified DNA fragments obtained by the above PCR and the pQE80L vector fragments prepared in Example 1 were linked using an In-Fusion (trademark) HD Cloning Kit (commercially available from Takara Bio Inc.) to construct each ALDH-expressing plasmid.

*Escherichia coli* BL21(DE3) was transformed using the expression plasmids obtained above to construct *E. coli* having each ALDH-expressing plasmid.

(3) Evaluation of productivity of 4-(aminomethyl)cyclohexane carboxylic acid of ALDH homolog

Each His-tagged recombinant type purified ALDH enzyme was obtained in the same method as in Example 1 using *E. coli* having each ALDH-expressing plasmid obtained in the above (2).

A 0.1 mL reaction solution (pH 7.8) containing 0.07 mg of the purified enzyme obtained, 0.07 mg of PatA obtained in Example 1, 50 mM MOPS (pH 7.8), 1 mM magnesium chloride, 1 mM DTT, 1 mM pyruvic acid, 0.1 mM PLP, 1 mM NAD$^+$, and a 1 mM mixture of cis- and trans-1,4-bis(aminomethyl)cyclohexane (a trans proportion of 45.7%) was prepared and reacted under conditions of 30°C and 400 rpm for 24 hours. In addition, the reaction was performed in the same manner using a reaction solution to which an equal volume of a MOPS buffer solution was added instead of the purified enzyme as a negative control, and a reaction solution using the purified enzyme XylC as a positive control.

After the reaction was completed, the reaction product was analyzed in the same method as in Example 1. The results are shown in Table 15.

[Table 15]

| Query | ALDH homolog | Identity (%) | Amount produced (mM) | | AMCHA trans proportion (%) |
| --- | --- | --- | --- | --- | --- |
| | | | TXA | cis-TXA | |
| - | - (negative control) | - | N.D. | N.D. | - |
| PatD | CkpatD | 88.4 | 0.22 | 0.35 | 39 |
| | SepatD | 83.3 | 0.23 | 0.39 | 37 |
| | CspatD | 83.3 | 0.29 | 0.28 | 51 |
| | PppatD | 71.1 | 0.24 | 0.32 | 43 |

(continued)

| Query | ALDH homolog | Identity (%) | Amount produced (mM) | | AMCHA trans proportion (%) |
| --- | --- | --- | --- | --- | --- |
| | | | TXA | cis-TXA | |
| - | - (negative control) | - | N.D. | N.D. | - |
| XylC | HaBD | 84.2 | 0.33 | 0.06 | 85 |
| | SsBD | 72.4 | 0.20 | 0.03 | 87 |
| | RiAD | 48.4 | 0.02 | 0.02 | 50 |
| | RrAD | 49.7 | 0.01 | 0.01 | 50 |
| StyD | PmsLAD | 86.9 | 0.22 | 0.38 | 37 |
| | BlAD | 69.9 | 0.20 | 0.34 | 37 |
| | PbsAD | 63.3 | 0.21 | 0.37 | 36 |
| - | XylC (positive control) | - | 0.30 | 0.05 | 87 |

As shown in Table 15, it was found that 4-(aminomethyl)cyclohexane-1-carboxylic acid could be produced even when ALDH having about 60% or more identity to PatD, XylC or StyD was used. In addition, it was found that, when CspatD, HaBD, or SsBD was used, the trans proportion of the produced 4-(aminomethyl)cyclohexane-1-carboxylic acid was larger than 50%, and these enzymes were enzymes with high trans-specificity. Particularly, it was found that, when HaBD or SsBD having 70% or more identity to XylC was used, the trans proportion of the produced 4-(aminomethyl) cyclohexane-1-carboxylic acid was 70% or more, and these enzymes reacted particularly trans-selectively, like XylC, that is, had a high trans-selectivity.

[Example 5] Evaluation of TXA productivity by introducing coenzyme regeneration system

(1) Construction of alanine dehydrogenase (AlaDH) expression strain

In order to obtain gene fragments of BsAlaDH encoding alanine dehydrogenase derived from *Bacillus subtilis* 168 shown in SEQ ID NO. 123, a PCR reaction was performed using chromosomal DNA of *Bacillus subtilis* 168 prepared by a general method as a template and oligonucleotides having nucleotide sequences shown in SEQ ID Nos. 83 and 84 as a primer set to obtain BsAlaDH gene fragments shown in SEQ ID NO. 124. The 5' end of the nucleotide sequence shown in SEQ ID NO. 9 contained a sequence complementary to the 5' end of the nucleotide sequence shown in SEQ ID NO. 83, and the 5' end of the nucleotide sequence shown in SEQ ID NO. 10 contained a sequence complementary to the 5' end of the nucleotide sequence shown in SEQ ID NO. 84.

The BsAlaDH fragments obtained by the above PCR and the pQE80L vector fragment prepared in Example 1 were linked using an In-Fusion (trademark) HD Cloning Kit (commercially available from Takara Bio Inc.) to obtain an expression plasmid pQE80L-BsAlaDH.

*Escherichia coli* BL21(DE3) was transformed using the expression plasmid obtained above to obtain BL21(DE3)/pQE80L-BsAlaDH.

(2) Construction of NADH oxidase (NOX) expression strain

In order to obtain gene fragments of BsNOX encoding NADH oxidase derived from *Bacillus subtilis* 168 shown in SEQ ID NO. 125, a PCR reaction was performed using chromosomal DNA of *Bacillus subtilis* 168 as a template and oligonucleotides having nucleotide sequences shown in SEQ ID Nos. 85 and 86 as a primer set to obtain BsNOX gene fragments shown in SEQ ID NO. 126. The 5' end of the nucleotide sequence shown in SEQ ID NO. 9 contained a sequence complementary to the 5' end of the nucleotide sequence shown in SEQ ID NO. 85, and the 5' end of the nucleotide sequence shown in SEQ ID NO. 10 contained a sequence complementary to the 5' end of the nucleotide sequence shown in SEQ ID NO. 86.

The BsNOX gene fragments obtained by the above PCR and the pQE80L vector fragment prepared in Example 1 were linked in 5 parts using an In-Fusion (trademark) HD Cloning Kit (commercially available from Takara Bio Inc.) to obtain an expression plasmid pQE80L-BsNOX.

*Escherichia coli* BL21(DE3) was transformed using the expression plasmid obtained above to obtain BL21(DE3)/pQE80L-BsNOX.

(3) Expression of BsAlaDH and BsNOX and enzyme purification

Using the BL21(DE3)/pQE80L-BsAlaDH and BL21(DE3)/pQE80L-BsNOX obtained above, His-tagged recombinant type enzyme-purified BsAlaDH and BsNOX were obtained in the same method as in Example 1.

(4) Evaluation of TXA production activity by introducing AlaDH and NOX

A 0.1 mL reaction solution (pH 7.8) containing 0.07 mg of PatA obtained in Example 1, 0.07 mg of XylC obtained in Example 2, 0.07 mg of BsAlaDH obtained in the above (3), 0.07 mg of BsNOX, 50 mM MOPS (pH 7.8), 1 mM magnesium chloride, 1 mM DTT, 1 mM pyruvic acid, 0.1 mM PLP, 2 mM NAD$^+$, and a 1 mM mixture of cis- and trans-1,4-bis(aminomethyl)cyclohexane (a trans proportion of 45.7%) was prepared and reacted under conditions of 30°C and 400 rpm for 24 hours.

After the reaction was completed, the reaction product and the residual substrate were analyzed in the same method as in Example 1. The results are shown in Table 16.

[Table 16]

| Regenerating enzyme | | Amount produced (mM) | | |
|---|---|---|---|---|
| BsAlaDH | BsNOX | Residual substrate | TXA | cis-TXA |
| - | - | 0.56 | 0.46 | 0.09 |
| + | + | 0.13 | 0.71 | 0.27 |

As shown in Table 16, when the coenzyme regeneration system BsAlaDH and BsNOX were introduced, substrate consumption proceeded and the productivity of 4-(aminomethyl)cyclohexane-1-carboxylic acid was improved.

[Example 6] Production of 4-(aminomethyl)cyclohexane-1-carboxylic acid according to resting bacterial cell reaction using AT-expressing strain and ALDH-expressing strain

For the BL21(DE3)/pQE80L-PatA strain and BL21(DE3)/pQE80L-XylC strain constructed in Example 1, wet bacterial cells were obtained in the same method as in Example 1(2). Xylene was added to the wet bacterial cells to a final concentration of 10 mL/L, and a membrane treatment was performed at 30°C and 850 rpm for 30 minutes.

A 0.4 mL reaction solution containing 50 g/L of each membrane-treated wet bacterial cells, 50 mM MOPS(pH 7.8), 350 mM glucose, a 50 mM mixture of cis- and trans-1,4-bis(aminomethyl)cyclohexane (a trans proportion of 45.7%), 50 mM magnesium chloride, and 50 mM NAD$^+$ was prepared and reacted under conditions of 30°C and 850 rpm for 24 hours.

After the reaction was completed, the reaction product was analyzed in the same method as in Example 1. As a result, it was confirmed that 3.9 mM TXA and 2.8 mM cis-TXA were produced and accumulated. Therefore, it was confirmed that 4-(aminomethyl)cyclohexane carboxylic acid could be produced according to a resting bacterial cell reaction using microorganism bacterial cells each expressing AT and ALDH.

[Example 7] Production of 4-(aminomethyl)cyclohexane-1-carboxylic acid according to resting bacterial cell reaction using AT and ALDH co-expressing strain

(1) Construction of AT and ALDH co-expressing strain

A) Construction of BL21(DE3)/pET28a-PatA-XylC strain

A PCR reaction was performed using chromosomal DNA of *Escherichia coli* K12 MG1655 as a template and oligonucleotides having nucleotide sequences shown in SEQ ID NOs. 89 and 90 as a primer set to obtain PatA fragments. Subsequently, a PCR reaction was performed using the nucleotide sequence shown in SEQ ID NO. 24 as a template and oligonucleotides having nucleotide sequences shown in SEQ ID NOs. 91 and 92 as a primer set to obtain XylC fragments. Here, the 5' end of the nucleotide sequence shown in SEQ ID NO. 90 contained a sequence complementary to the 5' end of the nucleotide sequence shown in SEQ ID NO. 91.

A PCR reaction was performed using a mixture of the PatA fragments and XylC fragments obtained above as a template and oligonucleotides having nucleotide sequences shown in SEQ ID NOs. 89 and 92 as a primer set to obtain DNA (hereinafter referred to as PatA-XylC) fragments in which two fragments were linked.

In addition, a PCR reaction was performed using DNA consisting of nucleotide sequences shown in SEQ ID NOs.

**EP 4 541 902 A1**

87 and 88 as a primer set and an expression vector pET28a (commercially available from Novagen) as a template to obtain vector fragments of about 5.2 kb. Here, the 5' end of the nucleotide sequence shown in SEQ ID NO. 87 contained a sequence complementary to the 5' end of the nucleotide sequence shown in SEQ ID NO. 89, and the 5' end of the nucleotide sequence shown in SEQ ID NO. 88 contained a sequence complementary to the 5' end of the nucleotide sequence shown in SEQ ID NO. 92.

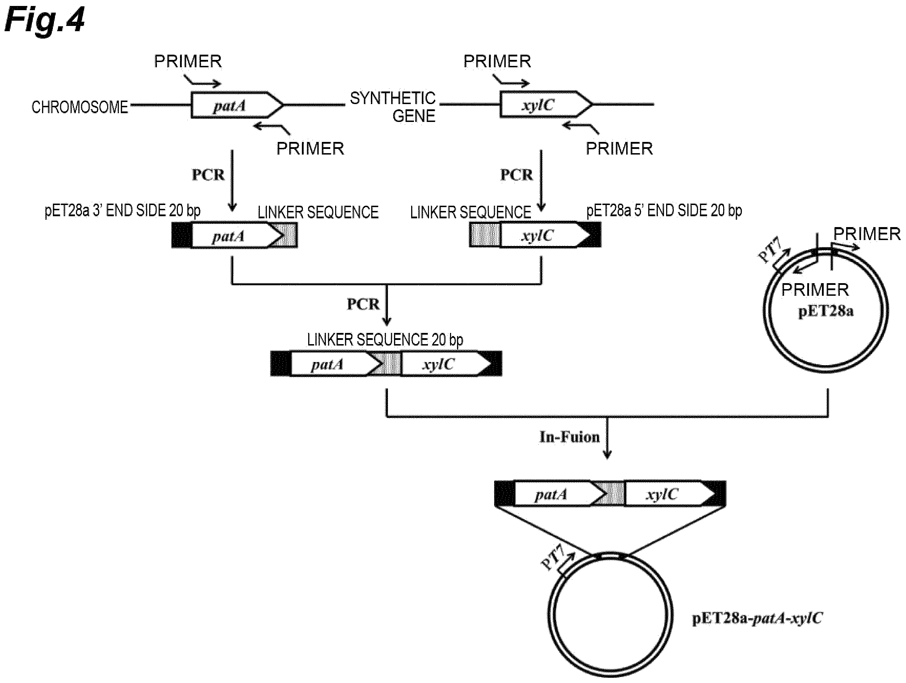

The PatA-XylC fragments and vector fragments obtained above were linked using an In-Fusion (trademark) HD Cloning Kit (commercially available from Takara Bio Inc.) to obtain an expression plasmid pET28a-PatA-XylC (FIG. 4).

*Escherichia coli* BL21(DE3) was transformed using the expression plasmid obtained above to obtain BL21(DE3)/pET28a-PatA-XylC strain.

A) Construction of BL21(DE3)/pET28a-XylC-PatA

A PCR reaction was performed using the nucleotide sequence shown in SEQ ID NO. 24 as a template and oligonucleotides having nucleotide sequences shown in SEQ ID NOs. 93 and 94 as a primer set to obtain XylC fragments. A PCR reaction was performed using chromosomal DNA of *Escherichia coli* K12 MG1655 as a template and oligonucleotides having nucleotide sequences shown in SEQ ID NOs. 95 and 96 as a primer set to obtain PatA fragments. Here, the 5' end of the nucleotide sequence shown in SEQ ID NO. 94 contained a sequence complementary to the 5' end of the nucleotide sequence shown in SEQ ID NO. 95.

A PCR reaction was performed using a mixture of the XylC fragments and PatA fragments obtained above as a template and oligonucleotides having nucleotide sequences shown in SEQ ID NOs. 93 and 96 as a primer set to obtain DNA (hereinafter referred to as XylC-PatA) fragments in which two fragments were linked. Here, the 5' end of the nucleotide sequence shown in SEQ ID NO. 87 contained a sequence complementary to the 5' end of SEQ ID NO. 93, and the 5' end of the nucleotide sequence shown in SEQ ID NO. 88 contained a sequence complementary to the 5' end of the nucleotide sequence shown in SEQ ID NO. 96.

The XylC-PatA fragments obtained above and the vector fragments obtained in the above A) were linked using an In-Fusion (trademark) HD Cloning Kit (commercially available from Takara Bio Inc.) to obtain an expression plasmid pET28a-XylC-PatA.

*Escherichia coli* BL21(DE3) was transformed using the expression plasmid obtained above to obtain a BL21(DE3)/pET28a-XylC-PatA strain.

C) Construction of BL21(DE3)/pUC19-PatA-XylC strain

A PCR reaction was performed using chromosomal DNA of *Escherichia coli* K12 MG1655 as a template and oligonucleotides having nucleotide sequences shown in SEQ ID NOs. 99 and 90 as a primer set to obtain PatA fragments. Subsequently, a PCR reaction was performed using the nucleotide sequence shown in SEQ ID NO. 24 as a template and oligonucleotides having nucleotide sequences shown in SEQ ID NOs. 91 and 100 as a primer set to obtain XylC fragments. Here, the 5' end of the nucleotide sequence shown in SEQ ID NO. 90 contained a sequence complementary to the 5' end of the nucleotide sequence shown in SEQ ID NO. 91.

A PCR reaction was performed using a mixture of the PatA fragments and XylC fragments obtained above as a template and DNA consisting of nucleotide sequences shown in SEQ ID NOs. 99 and 100 as a primer set to obtain DNA (hereinafter referred to as PatA-XylC) fragments in which two fragments were linked.

A PCR reaction was performed using DNA consisting of nucleotide sequences shown in SEQ ID NOs. 97 and 98 as a primer set and an expression vector pUC19 (commercially available from Nippon Gene Co., Ltd.) as a template to obtain vector fragments of about 2.6 kb. Here, the 5' end of the nucleotide sequence shown in SEQ ID NO. 97 contained a sequence complementary to the 5' end of the nucleotide sequence shown in SEQ ID NO. 99, and the 5' end of the nucleotide sequence shown in SEQ ID NO. 98 contained a sequence complementary to the 5' end of the nucleotide sequence shown in SEQ ID NO. 100.

The PatA-XylC fragments and vector fragments obtained above were linked using an In-Fusion (trademark) HD Cloning Kit (commercially available from Takara Bio Inc.) to obtain an expression plasmid pUC19-PatA-XylC.

*Escherichia coli* BL21(DE3) was transformed using the expression plasmid obtained above to obtain a BL21(DE3)/pUC19-PatA-XylC strain.

D) Construction of BL21(DE3)/pUC19-XylC-PatA strain

A PCR reaction was performed using DNA shown in SEQ ID NO. 24 as a template and oligonucleotides having nucleotide sequences shown in SEQ ID NOs. 101 and 94 as a primer set to obtain XylC fragments. Subsequently, a PCR reaction was performed using chromosomal DNA of *Escherichia coli* K12 MG1655 as a template and oligonucleotides having nucleotide sequences shown in SEQ ID NOs. 95 and 102 as a primer set to obtain PatA fragments. Here, the 5' end

32

of the nucleotide sequence shown in SEQ ID NO. 94 contained a sequence complementary to the 5' end of the nucleotide sequence shown in SEQ ID NO. 95.

A PCR reaction was performed using a mixture of the XylC fragments and PatA gene fragments obtained above as a template and oligonucleotides having nucleotide sequences shown in SEQ ID NOs. 101 and 102 as a primer set to obtain DNA (hereinafter referred to as XylC-PatA) fragments in which two fragments were linked. Here, the 5' end of the nucleotide sequence shown in SEQ ID NO. 97 contained a sequence complementary to the 5' end of the nucleotide sequence shown in SEQ ID NO. 101, and the 5' end of the nucleotide sequence shown in SEQ ID NO. 98 contained a sequence complementary to the 5' end of the nucleotide sequence shown in SEQ ID NO. 102.

The XylC-PatA fragments obtained above and the pUC19 vector fragments obtained in the above C) were linked using an In-Fusion (trademark) HD Cloning Kit (commercially available from Takara Bio Inc.) to obtain an expression plasmid pUC19-XylC-PatA.

*Escherichia coli* BL21(DE3) was transformed using the expression plasmid obtained above to obtain a BL21(DE3)/pUC19-XylC-PatA strain.

(2) Bacterial cell reaction using AT and ALDH co-expressing strain

The productivity of 4-(aminomethyl)cyclohexane-1-carboxylic acid was evaluated according to a resting bacterial cell reaction using the BL21(DE3)/pET28a-PatA-XylC strain, BL21(DE3)/pET28a-XylC-PatA strain, BL21(DE3)/pUC19-PatA-XylC strain and BL21(DE3)/pUC19-XylC-PatA strain constructed in the above (1) and the BL21(DE3) strain as a negative control.

Each bacteria strain was inoculated in a test tube containing a 2 mL LB medium containing 30 mg/L kanamycin or 100 mg/L ampicillin, and cultured with shaking at 30°C for 16 hours. The culture solution was inoculated in a 250 mL Erlenmeyer flask containing a 40 mL LB medium containing 30 mg/L kanamycin or 100 mg/L ampicillin and cultured with shaking at 30°C for 2 hours, IPTG was then added to a final concentration of 1 mM, and the mixture was additionally cultured with shaking at 37°C for 5 hours. In addition, as a control, BL21(DE3) was similarly cultured in a medium containing neither kanamycin nor ampicillin.

The culture solution after the culture was centrifuged to obtain wet bacterial cells. Xylene was added to the wet bacterial cells to a final concentration of 10 mL/L, and a membrane treatment was performed at 30°C and 850 rpm for 30 minutes.

A 0.4 mL reaction solution containing 100 g/L of the above membrane-treated wet bacterial cells, 50 mM MOPS (pH 7.8), 350 mM glucose, a 50 mM mixture of cis- and trans-1,4-bis(aminomethyl)cyclohexane (a trans proportion of 45.7%), 50 mM magnesium chloride, and 50 mM NAD$^+$ was prepared and reacted under conditions of 30°C and 850 rpm for 24 hours.

After the reaction was completed, the reaction product was analyzed in the same method as in Example 1. The results are shown in Table 17.

[Table 17]

| Microorganisms | Amount produced (mM) | |
|---|---|---|
| | TXA | cis-TXA |
| BL21(DE3) | N.D. | N.D. |
| BL21(DE3)/pET28a-PatA-XylC | 5.6 | 0.9 |
| BL21(DE3)/pET28a-XylC-PatA | 9.2 | 0.8 |
| BL21(DE3)/pUC19-PatA-XylC | 8.3 | 1.1 |
| BL21(DE3)/pUC19-XylC-PatA | 8.6 | 1.7 |
| N.D.: qualitative limit or below (S/N<3) | | |

As shown in Table 17, it was confirmed that 4-(aminomethyl)cyclohexane-1-carboxylic acid could be produced according to a resting bacterial cell reaction using the bacteria strain co-expressing PatA and XylC.

Here, the productivity of 4-(aminomethyl)cyclohexane-1-carboxylic acid when NAD$^+$ was removed from the reaction solution was evaluated. The reaction conditions were as described above. The results are shown in Table 18.

[Table 18]

| Microorganisms | Amount produced (mM) | |
|---|---|---|
| | TXA | cis-TXA |
| BL21(DE3) | N.D. | N.D. |
| BL21(DE3)/pET28a-PatA-XylC | 1.0 | 0.1 |
| BL21(DE3)/pET28a-XylC-PatA | 2.7 | 0.2 |
| BL21(DE3)/pUC19-PatA-XylC | 4.1 | 0.2 |
| BL21(DE3)/pUC19-XylC-PatA | 3.0 | 0.4 |
| N.D.: qualitative limit or below (S/N<3) | | |

When NAD$^+$ was removed from the reaction solution, the amount of 4-(aminomethyl)cyclohexane-1-carboxylic acid produced decreased. This suggests that supply of coenzymes was important in the production of 4-(aminomethyl) cyclohexane-1-carboxylic acid according to a resting bacterial cell reaction.

[Example 8] Production of 4-(aminomethyl)cyclohexane-1-carboxylic acid by additive culture using AT and ALDH co-expressing strain

The productivity of 4-(aminomethyl)cyclohexane-1-carboxylic acid was evaluated according to an additive culture method using the BL21(DE3)/pUC19-PatA-XylC strain, BL21(DE3)/pUC19-XylC-PatA strain constructed in Example 7 and the BL21(DE3) strain as a negative control.

Each bacteria strain was inoculated in a test tube containing a 2 mL LB medium containing 100 mg/L ampicillin and cultured with shaking at 30°C for 16 hours. The BL21(DE3) strain was similarly cultured in a medium containing no ampicillin. The culture solution was inoculated in a large test tube containing a 4 mL LB medium containing 30 mg/L 100 mg/L ampicillin and cultured at 30°C for 5 hours, IPTG was then added to a final concentration of 1 mM and a 1 g/L mixture of cis- and trans-1,4-bis(aminomethyl)cyclohexane (a trans proportion of 45.7%) was added, and the mixture was additionally cultured at 37°C for 24 hours.

After the culture was completed, the reaction product was analyzed in the same method as in Example 1. The results are shown in Table 19.

[Table 19]

| Microorganisms | Amount produced (μM) |
|---|---|
| | TXA |
| BL21(DE3) | 4 |
| BL21(DE3)/pUC19-PatA-XylC | 133 |
| BL21(DE3)/pUC19-XylC-PatA | 27 |

As shown in Table 19, it was confirmed that TXA could be produced by an additive culture method

[Example 9] Production of 4-(aminomethyl)cyclohexane carboxylic acid using ALDH homolog

(1) Search for ALDH homolog enzyme

Using the amino acid sequence (SEQ ID NO. 20) of XylC, indicating an ALDH having high selectivity for trans-substrates in Example 3 as a query, those having 50% or more identity to XylC and less than 90% identity with homolog enzymes in the found results were selected using the homology search function of the BLAST (https://blast.ncbi.nlm.nih.gov/Blast.cgi) protein sequence database from the National Center for Biotechnology Information. The amino acid sequences (SEQ ID NOs. 127 to 139) of the enzymes extracted by the above operation are shown in Table 20.

[Table 20]

| ALDH homolog | Identity (%) | | | Microorganism origin | Amino acid sequenc e (SEQ ID NO.) |
|---|---|---|---|---|---|
| | vs.Xyl C | vs.HaB D | vs.SsB D | | |
| 1 | 68.0 | 65.3 | 76.0 | *Hydrogenophaga aromaticivorans* | 127 |
| 2 | 99.0 | 84.0 | 70.8 | *Pseudomonas aeruginosa* | 128 |
| 3 | 87.1 | 80.1 | 69.2 | *Pseudomonas sp.* MAP12 | 129 |
| 4 | 82.6 | 92.2 | 67.8 | *Alteromonas* | 130 |
| 5 | 81.3 | 80.7 | 71.3 | *Tepidiphilus succinatimandens* | 131 |
| 6 | 51.8 | 52.2 | 57.9 | *Halomonas cupida* | 132 |
| 7 | 52.5 | 52.0 | 58.6 | *Pseudomonas fluorescens* | 133 |
| 8 | 57.3 | 55.9 | 63.9 | *Glaciimonas immobilis* | 134 |
| 9 | 58.7 | 57.1 | 64.5 | *Paraburkholderia unamae* | 135 |
| 10 | *55.1* | 54.7 | 57.8 | *Marinobacter salsuginis* | 136 |
| 11 | 57.6 | 56.4 | 63.0 | *Aromatoleum toluclasticum* | 137 |
| 12 | 58.9 | 57.2 | 64.9 | *Ideonella livida* | 138 |
| 13 | 58.7 | 56.5 | 62.2 | *Burkholderia_sp._ D7* | 139 |

In Table 20, ALDH homologs 1 to 11 and 13 are all proteins annotated as benzaldehyde dehydrogenases, and ALDH homolog 12 is a protein annotated as an aldehyde dehydrogenase family protein.

(2) Construction of ALDH homolog expressing strain

In order to obtain gene fragments of ALDH homolog enzymes shown in Table 20, a PCR reaction was performed using the templates and primers 1 and 2 shown in Table 21. In this case, the 5' end of the nucleotide sequence shown in SEQ ID NO. 9 contained a nucleotide sequence complementary to the 5' end of the nucleotide sequence shown in each primer 1 in Table 21, and the 5' end of the nucleotide sequence shown in SEQ ID NO. 10 contained a nucleotide sequence complementary to the 5' end of the nucleotide sequence shown in each primer 2 in Table 21.

[Table 21]

| ALDHhomolog | Microorganism origin | SEQ ID NO. | | |
|---|---|---|---|---|
| | | Template | Primer 1 | Primer 2 |
| 1 | *Hydrogenophaga aromaticivorans* | 140 | 153 | 154 |
| 2 | *Pseudomonas aeruginosa* | 141 | *155* | 156 |
| 3 | *Pseudomonas sp.* MAP12 | 142 | 157 | 158 |
| 4 | *Alteromonas* | 143 | 159 | 160 |
| 5 | *Tepidiphilus succinatimandens* | 144 | 161 | 162 |
| 6 | *Halomonas cupida* | 145 | 163 | 164 |
| 7 | *Pseudomonas fluorescens* | 146 | 165 | 166 |
| 8 | *Glaciimonas immobilis* | 147 | 167 | 168 |
| 9 | *Paraburkholderia unamae* | 148 | 169 | 170 |
| 10 | *Marinobacter salsuginis* | 149 | 171 | 172 |
| 11 | *Aromatoleum toluclasticum* | 150 | 173 | 174 |
| 12 | *Idconella livida* | 151 | 175 | 176 |
| 13 | *Burkholderia_sp._D7* | 152 | 177 | 178 |

The DNAs shown in SEQ ID NOs. 140 to 152 were DNAs in which the nucleotide sequences of genes encoding ALDH homolog enzymes shown in SEQ ID NOs. 127 to 139 were codon-optimized for expression in *E. coli,* and prepared by artificial synthesis.

The amplified DNA fragments obtained by the above PCR and the pQE80L vector fragments prepared in Example 1 were linked using an In-Fusion (trademark) HD Cloning Kit (commercially available from Takara Bio Inc.) to construct each ALDH-expressing plasmid. *Escherichia coli* BL21(DE3) was transformed using the expression plasmids obtained above to construct *E. coli* having each ALDH-expressing plasmid.

(3) Evaluation of productivity of 4-(aminomethyl)cyclohexane carboxylic acid of ALDH homolog

Each His-tagged recombinant type purified ALDH enzyme was obtained in the same method as in Example 1 using *E. coli* having each ALDH-expressing plasmid obtained in the above (2).

A 0.1 mL reaction solution (pH 7.8) containing 0.07 mg of the purified enzyme obtained, 0.07 mg of PatA obtained in Example 1, 50 mM CHES (pH 9.0), 10 mM magnesium chloride, 1 mM DTT, 1 mM pyruvic acid, 0.1 mM PLP, 1 mM NAD$^+$, and a 1 mM mixture of cis- and trans-1,4-bis(aminomethyl)cyclohexane (a trans proportion of 45.7%) was prepared and reacted under conditions of 30°C and 400 rpm for 24 hours. In addition, as a positive control, the reaction was performed in the same manner using a reaction solution using the purified enzyme XylC.

After the reaction was completed, the reaction product was analyzed in the same method as in Example 1. The results are shown in Table 22.

[Table 22]

| ALDH homolog | Identity (%) | | | Amount produced (mM) | | AMCHA trans proportion (%) |
|---|---|---|---|---|---|---|
| | vs. XylC | vs. HaBD | vs. SsBD | TXA | cis-TXA | |
| 1 | 68.0 | 65.3 | 76.0 | 0.25 | 0.04 | 85 |
| 2 | 99.0 | 84.0 | 70.8 | 0.45 | 0.17 | 72 |
| 3 | 87.1 | 80.1 | 69.2 | 0.50 | 0.35 | 59 |
| 4 | 82.6 | 92.2 | 67.8 | 0.59 | 0.06 | 90 |
| 5 | 81.3 | 80.7 | 71.3 | 0.55 | 0.23 | 71 |
| 6 | 51.8 | 52.2 | 57.9 | 0.02 | 0.04 | 39 |
| 7 | 52.5 | 52.0 | 58.6 | 0.11 | 0.03 | 78 |
| 8 | 57.3 | 55.9 | 63.9 | 0.22 | 0.46 | 32 |
| 9 | 58.7 | 57.1 | 64.5 | 0.19 | 0.21 | 48 |
| 10 | 55.1 | 54.7 | 57.8 | 0.01 | 0.01 | 54 |
| 11 | 57.6 | 56.4 | 63.0 | 0.03 | 0.02 | 59 |
| 12 | 58.9 | 57.2 | 64.9 | 0.04 | 0.06 | 41 |
| 13 | 58.7 | 56.5 | 62.2 | 0.18 | 0.16 | 53 |
| XylC | 100 | 84.2 | 74.2 | 0.57 | 0.18 | 76 |

As shown in Table 22, it was confirmed that, in all of the evaluated ALDH homologs 1 to 13, 4-(aminomethyl) cyclohexane-1-carboxylic acid was produced. Particularly, it was confirmed that 4-(aminomethyl)cyclohexane-1-carboxylic acid was significantly produced when ALDH homologs 1 to 5, 7 to 9, and 13 were used. In addition, when ALDH homologs 1 to 5, 7, and 13 were used, the trans proportion of the produced 4-(aminomethyl)cyclohexane-1-carboxylic acid was larger than 50%, indicating high specificity for the trans-substrate. Among these, particularly, ALDH homologs 1, 2, 4, 5, and 7 had a trans proportion of 70% or more of 4-(aminomethyl)cyclohexane-1-carboxylic acid, and were found to have particularly high selectivity for trans-substrates comparable to that of XylC. Among these, ALDH homologs 4 and 5 produced trans-4-(aminomethyl)cyclohexane-1-carboxylic acid in quantities equivalent to that of XylC.

[Example 10] Evaluation of TXA productivity by enhancing keto acid and coenzyme supply

(1) Construction of evaluation strain

A) Construction of MG1655/pQE80L-XylC-PatA

A PCR reaction was performed using the nucleotide sequence shown in SEQ ID NO. 24 as a template and oligonucleotides having nucleotide sequences shown in SEQ ID NOs. 179 and 94 as a primer set to obtain XylC fragments. Subsequently, a PCR reaction was performed using chromosomal DNA of *Escherichia coli* K12 MG1655 as a template and oligonucleotides having nucleotide sequences shown in SEQ ID NOs. 95 and 180 as a primer set to obtain PatA fragments. Here, the 5' end of the nucleotide sequence shown in SEQ ID NO. 94 contained a sequence complementary to the 5' end of the nucleotide sequence shown in SEQ ID NO. 95.

A PCR reaction was performed using a mixture of the XylC fragments and PatA fragments obtained above as a template and oligonucleotides having nucleotide sequences shown in SEQ ID NOs. 179 and 180 as a primer set to obtain DNA (hereinafter referred to as XylC-PatA) fragments in which two fragments were linked.

In addition, a PCR reaction was performed using DNA consisting of nucleotide sequences shown in SEQ ID NOs. 181 and 182 as a primer set and an expression vector pQE80L (commercially available from QIAGEN) as a template to obtain vector fragments of about 4.7 kb. Here, the 5' end of the nucleotide sequence shown in SEQ ID NO. 181 contained a sequence complementary to the 5' end of the nucleotide sequence shown in SEQ ID NO. 179, and the 5' end of the nucleotide sequence shown in SEQ ID NO. 182 contained a sequence complementary to the 5' end of the nucleotide sequence shown in SEQ ID NO. 180.

The XylC-PatA fragments and vector fragments obtained above were linked using an In-Fusion (trademark) HD Cloning Kit (commercially available from Takara Bio Inc.) to obtain an expression plasmid pQE80L-XylC-PatA. *Escherichia coli* MG1655 was transformed using the expression plasmid obtained above to obtain an MG1655/pQE80L-XylC-PatA strain.

B) Construction of MG1655/pQE80L-PatA-BsAlaDH-XylC-BsNOX

A PCR reaction was performed using chromosomal DNA of *Escherichia coli* K12 MG1655 as a template and oligonucleotides having nucleotide sequences shown in SEQ ID NOs. 183 and 90 as a primer set to obtain PatA fragments. Subsequently, a PCR reaction was performed using the nucleotide sequence shown in SEQ ID NO. 24 as a template and oligonucleotides having nucleotide sequences shown in SEQ ID NOs. 91 and 30 as a primer set to obtain XylC fragments. Here, the 5' end of the nucleotide sequence shown in SEQ ID NO. 90 contained a sequence complementary to the 5' end of the nucleotide sequence shown in SEQ ID NO. 91.

A PCR reaction was performed using a mixture of the PatA fragments and XylC fragments obtained above as a template and oligonucleotides having nucleotide sequences shown in SEQ ID NOs. 183 and 30 as a primer set to obtain a DNA (hereinafter referred to as PatA-XylC) fragment in which two fragments were linked.

In addition, a PCR reaction was performed using DNA consisting of nucleotide sequences shown in SEQ ID NOs. 181 and 182 as a primer set, and an expression vector pQE80L (commercially available from QIAGEN) as a template to obtain vector fragments of about 4.7 kb. Here, the 5' end of the nucleotide sequence shown in SEQ ID NO. 181 contained a sequence complementary to the 5' end of the nucleotide sequence shown in SEQ ID NO. 183, and the 5' end of the nucleotide sequence shown in SEQ ID NO. 182 contained a sequence complementary to the 5' end of the nucleotide sequence shown in SEQ ID NO. 30.

The PatA fragments and vector fragments obtained above were linked using an In-Fusion (trademark) HD Cloning Kit (commercially available from Takara Bio Inc.) to obtain a plasmid pQE80L-PatA-XylC. A PCR reaction was performed using the plasmid obtained above as a template and DNA consisting of nucleotide sequences shown in SEQ ID NOs. 184 and 182 as a primer set to obtain DNA (hereinafter referred to as pQE80L-PatA) fragments.

A PCR reaction was performed using chromosomal DNA of *Bacillus subtilis* 168 as a template and oligonucleotides having nucleotide sequences shown in SEQ ID NOs. 185 and 186 as a primer set to obtain BsAlaDH gene fragments. A PCR reaction was performed using chromosomal DNA of *Bacillus subtilis* 168 as a template and oligonucleotides having nucleotide sequences shown in SEQ ID NOs. 187 and 86 as a primer set to obtain BsNOX gene fragments. Here, the 5' end of the nucleotide sequence shown in SEQ ID NO. 186 contained a sequence complementary to the 5' end of the nucleotide sequence shown in SEQ ID NO. 187.

A PCR reaction was performed using a mixture of the BsAlaDH gene fragments and NOX gene fragments obtained above as a template and oligonucleotides having nucleotide sequences shown in SEQ ID NOs. 185 and 86 as a primer set to obtain DNA (hereinafter referred to as BsAlaDH-BsNOX) fragments in which two fragments were linked.

Here, the 5' end of the nucleotide sequence shown in SEQ ID NO. 185 contained a sequence complementary to the 5' end of the nucleotide sequence shown in SEQ ID NO. 184, and the 5' end of the nucleotide sequence shown in SEQ ID NO. 86 contained a sequence complementary to the 5' end of the nucleotide sequence shown in SEQ ID NO. 182.

The pQE80L-PatA fragments and BsAlaDH-BsNOX fragments obtained above were linked using an In-Fusion (trademark) HD Cloning Kit (commercially available from Takara Bio Inc.) to obtain a plasmid pQE80L-PatA-BsAlaDH-BsNOX.

A PCR reaction was performed using the plasmid obtained above as a template and DNA consisting of nucleotide sequences shown in SEQ ID NOs. 188 and 189 as a primer set to obtain DNA (hereinafter referred to as pQE80L-PatA-BsAlaDH-BsNOX) fragments.

A PCR reaction was performed using the nucleotide sequence shown in SEQ ID NO. 24 as a template and oligonucleotides having nucleotide sequences shown in SEQ ID NOs. 190 and 94 as a primer set to obtain XylC fragments.

Here, the 5' end of the nucleotide sequence shown in SEQ ID NO. 188 contained a sequence complementary to the 5' end of the nucleotide sequence shown in SEQ ID NO. 190, and the 5' end of the nucleotide sequence shown in SEQ ID NO. 189 contained a sequence complementary to the 5' end of the nucleotide sequence shown in SEQ ID NO. 94.

The pQE80L-PatA-BsAlaDH-BsNOX fragments and XylC fragments obtained above were linked using an In-Fusion (trademark) HD Cloning Kit (commercially available from Takara Bio Inc.) to obtain an expression plasmid pQE80L-PatA-BsAlaDH-XylC-BsNOX.

*Escherichia coli* MG1655 was transformed using the expression plasmid obtained above to obtain an MG1655/pQE80L-PatA-BsAlaDH-XylC-BsNOX strain.

C) Construction of MG1655ΔaceE/pQE80L-patA-xylC-ald-nox strain

<Obtaining DNA fragments to be used as markers during gene deletion>

PCR was performed using DNA consisting of nucleotide sequences shown in SEQ ID NOs. 191 and 192 as a primer set and pCatSac (Appl Environ Microbiol (2013) 79, 3033-3039) as a template to obtain cat-sacB fragments containing a chloramphenicol-resistant cat gene and a sucrose-susceptible sacB gene.

<Construction of *E. coli* lacking pyruvate dehydrogenase activity>

*E. coli* lacking DNA encoding a pyruvate dehydrogenase (hereinafter referred to as an aceE gene) was constructed by the following method. PCR was performed using genomic DNA of the *Escherichia coli* MG1655 strain prepared by a general method as a template and DNA consisting of a nucleotide sequence shown in "Primer set" in Table 23 as a primer set to amplify each DNA fragment.

[Table 23]

| Primer set (SEQ ID NO.) | Amplified DNA fragment | Remarks |
|---|---|---|
| 193 and 194 | aceE upstream 1 | The 5' end sequences of the nucleotide sequences shown in SEQ ID NO. 191 and SEQ ID NO. 194 are complementary. |
| 195 and 196 | aceE downstream 1 | The 5' end sequences of the nucleotide sequences shown in SEQ ID NO. 192 and SEQ ID NO. 195 are complementary. |
| 193 and 197 | aceE upstream 2 | The 5' end sequences of the nucleotide sequences shown in SEQ ID NO. 197 and SEQ ID NO. 198 are complementary. |
| 198 and 196 | aceE downstream 2 | |

The aceE upstream 1 and aceE upstream 2 included a region from the initiation codon of the aceE gene to about 1,000 bp upstream from the initiation codon. The aceE downstream 1 and aceE downstream 2 included a region from about 50 bp to about 1,000 bp downstream from the termination codon of the aceE gene.

PCR was performed using a mixture of the aceE upstream 1, aceE downstream 1, and cat-sacB fragments in an equal molar ratio as a template and DNA consisting of nucleotide sequences shown in SEQ ID NOs. 193 and 196 as a primer set to obtain DNA (hereinafter referred to as aceE::cat-sacB) fragments consisting of a sequence in which the cat-sacB fragment was inserted into a sequence of the region surrounding the aceE gene.

PCR was performed using a mixture of the aceE upstream 2 and aceE downstream 2 at an equal molar ratio as a template and DNA consisting of nucleotide sequences shown in SEQ ID NOs. 193 and 196 as a primer set to obtain DNA (hereinafter referred to as ΔaceE) fragments containing no aceE and consisting of a sequence in which the aceE upstream and the aceE downstream were directly linked.

The aceE::cat-sacB fragment was introduced by an electroporation method into the MG1655 strain carrying the plasmid pKD46 containing the gene encoding λ recombinase [Datsenko, K. A., Warner, B. L., Proc. Natl. Acad. Sci., USA, Vol. 97, 6640-6645 (2000)] to obtain a transformant exhibiting chloramphenicol resistance and sucrose susceptibility (transformant in which the aceE gene was substituted with aceE::cat-sacB).

The ΔaceE fragment was introduced into the transformant by an electroporation method to obtain a transformant exhibiting chloramphenicol susceptibility and sucrose resistance (transformant in which aceE::cat-sacB was substituted

with △aceE). From among these, a transformant exhibiting ampicillin susceptibility (a transformant from which pKD46 was removed) was obtained. This transformant was named MG1655△aceE.

<Obtaining expression plasmid>

PCR was performed using the plasmid pQE80L-PatA-XylC obtained in A) as a template and DNA consisting of nucleotide sequences shown in SEQ ID NOs. 199 and 182 as a primer set to obtain DNA (hereinafter referred to as pQE80L-PatA-XylC) fragments.

PCR was performed using the BsAlaDH-BsNOX fragment obtained in A) as a template and DNA consisting of nucleotide sequences shown in SEQ ID NOs. 200 and 86 as a primer set to obtain DNA (hereinafter referred to as BsAlaDH-BsNOX2) fragments.

Here, the 5' end of the nucleotide sequence shown in SEQ ID NO. 200 contained a sequence complementary to the 5' end of the nucleotide sequence shown in SEQ ID NO. 199, and the 5' end of the nucleotide sequence shown in SEQ ID NO. 86 contained a sequence complementary to the 5' end of the nucleotide sequence shown in SEQ ID NO. 182.

The pQE80L-PatA-XylC fragments and BsAlaDH-BsNOX2 fragments obtained above were linked using an In-Fusion (trademark) HD Cloning Kit (commercially available from Takara Bio Inc.) to obtain an expression plasmid pQE80L-PatA-XylC-BsAlaDH-BsNOX. *Escherichia coli* MG1655△aceE was transformed using the expression plasmid obtained above to obtain an MG1655△aceE/pQE80L-PatA-XylC-BsAlaDH-BsNOX strain.

(2) Resting bacterial cell reaction using strain with coenzyme regeneration system introduced

The productivity of 4-(aminomethyl)cyclohexane-1-carboxylic acid was evaluated according to a resting bacterial cell reaction using the MG1655/pQE80L-XylC-PatA strain, MG1655/pQE80L-PatA-BsAlaDH-XylC-BsNOX strain and MG1655△aceE/pQE80L-PatA-XylC-BsAlaDH-BsNOX strain constructed in the above (1).

Each bacteria strain was inoculated in a test tube containing a 2 mL LB medium containing 100 mg/L ampicillin and cultured with shaking at 30°C for 16 hours. The culture solution was inoculated in a 250 mL Erlenmeyer flask containing a 40 mL LB medium containing 100 mg/L ampicillin and cultured with shaking at 28°C for 2 hours, IPTG was then added to a final concentration of 1 mM, and the mixture was additionally cultured with shaking at 28°C for 24 hours. However, only in the case of the MG1655△aceE/pQE80L-PatA-XylC-BsAlaDH-BsNOX strain, the culture time until ITPG was added was set to 3.5 hours.

The culture solution after the culture was centrifuged to obtain wet bacterial cells. Xylene was added to the wet bacterial cells to a final concentration of 10 mL/L, and a membrane treatment was performed at 30°C and 850 rpm for 30 minutes.

A 0.2 mL reaction solution containing 100 g/L of the above membrane-treated wet bacterial cells, 140 mM glucose, a 50 mM mixture of cis- and trans-1,4-bis(aminomethyl)cyclohexane (a trans proportion of 45.7%), 50 mM magnesium chloride, 10 mM pyruvic acid, and 10 mM $NAD^+$ was prepared and reacted under conditions of 30°C and 850 rpm for 24 hours.

After the reaction was completed, the reaction product and the residual substrate were analyzed in the same method as in Example 1. The results are shown in Table 24.

[Table 24]

| Microorganisms | Residual substrate (mM) | Amount produced (mM) | |
| --- | --- | --- | --- |
| | | TXA | cis-TXA |
| MG1655/pQE80L-XylC-PatA | 24.2 | 9.9. | N.D. |
| MG1655/pQE80L-PatA-BsAlaDH-XylC-BsNOX | 12.9 | 21.9 | 0.8 |
| MG1655△aceE/pQE80L-PatA-XylC-BsAlaDH-BsNOX | 5.3 | 27.0 | 1.6 |
| N.D.: qualitative limit or below (S/N<3) | | | |

As shown in Table 24, when pyruvic acid and $NAD^+$ regeneration genes were introduced, the amount of 4-(aminomethyl)cyclohexane-1-carboxylic acid produced and the amount of the substrate 1,4-bis(aminomethyl)cyclohexane consumed were improved. In addition, deletion of the pyruvate degradation gene in the host *E. coli* also improved target production and substrate consumption, and an improvement in substrate consumption was particularly significant. Accordingly, it is speculated that deletion of the pyruvate degradation gene promoted the transamination reaction of the substrate.

From the above, it was found that, in the production of 4-(aminomethyl)cyclohexane-1-carboxylic acid according to a resting bacterial cell reaction, it was effective to enhance the supply of keto acids and coenzymes by introducing genes for enzymes that regenerate keto acids and coenzymes or by blocking the degradation system of keto acids in the host.

[Example 11] Extraction of amino acid residue common to ALDH having high specificity for trans-4-(aminomethyl)cyclohexane-1-carbaldehyde

In Example 3, Example 4 and Example 9, it can be said that ALDHs that allowed AMCHA with a high trans proportion to be produced according to a reaction using a cis-trans mixed substrate were useful for the production of AMCHA, particularly for the production of TXA. In order to find amino acid residues specific to these ALDHs, the amino acid sequences were compared.

In Example 3, Example 4 and Example 9, the ALDHs (SEQ ID NOs. 20, 40, 41, 127, 128, 130 and 131) that allowed AMCHA with a trans proportion of 70% or more to be produced according to a reaction using a cis-trans mixed substrate were extracted as ALDHs having particularly high specificity for trans-4-(aminomethyl)cyclohexane-1-carbaldehyde. As a result, all of the enzymes were annotated as benzaldehyde dehydrogenases. Here, among the ALDHs annotated as benzaldehyde dehydrogenases used in Example 3, Example 4 and Example 9, ALDHs (SEQ ID NOs. 134 and 135) that allowed AMCHA with a trans proportion of less than 50% to be produced according to a reaction using a cis-trans mixed substrate were extracted as ALDHs having no specificity for trans-4-(aminomethyl)cyclohexane-1-carbaldehyde.

Here, in order to eliminate the influence of the strength of the enzyme activity, ALDHs that allowed less than 0.2 mM AMCHA to be produced according to a reaction using a cis-trans mixed substrate in Example 3, Example 4 and Example 9 were excluded.

The amino acid sequence of ALDH extracted by the above operation was aligned using Clustal Omega (FIG. 5A and FIG. 5B). In addition, amino acid residues that were conserved in ALDHs having high specificity for trans-4-(aminomethyl)cyclohexane-1-carbaldehyde but were not conserved in ALDHs having no specificity for trans-4-(aminomethyl)cyclohexane-1-carbaldehyde were extracted. The extracted amino acid residues are shown with the amino acid residue numbers corresponding to XylC (SEQ ID NO. 20) in Table 25.

[Table 25]

| Amino acid residues conserved in ALDHs having high specificity for trans-4-(aminomethyl)cyclohexane-1-carbaldehyde | | | |
|---|---|---|---|
| G35 | A122 | D319 | P440 |
| A65 | L124 | D333 | C443 |
| L82 | V134 | Q365 | P445 |
| G104 | V144 | I390 | S466 |
| G109 | F160 | A420 | I467 |
| I110 | G271 | L423 | |
| Q117 | A290 | V434 | |

It was suggested that the amino acid residues shown in Table 25 were important for ALDHs to have particularly high specificity for trans-4-(aminomethyl)cyclohexane-1-carbaldehyde.

## Claims

1.  A protein having transamination activity of transferring an amino group of 1,4-bis(aminomethyl)cyclohexane to another compound to produce 4-(aminomethyl)cyclohexane-1-carbaldehyde and consisting of an amino acid sequence having 60% or more identity to an amino acid sequence shown in any one of SEQ ID NOs. 1 to 4 and 103 to 105.

2.  DNA encoding the protein according to claim 1.

3.  Recombinant DNA comprising the DNA according to claim 2.

4.  A recombinant cell comprising the DNA according to claim 2 or obtained by transforming a host cell with the recombinant DNA according to claim 3.

5. A protein having aldehyde dehydrogenase activity of oxidizing an aldehyde group of 4-(aminomethyl)cyclohexane-1-carbaldehyde to produce 4-(aminomethyl)cyclohexane-1-carboxylic acid and consisting of an amino acid sequence having 50% or more identity to an amino acid sequence shown in any one of SEQ ID NOs. 19 to 22, 35 to 46 and 127 to 139.

6. A protein having aldehyde dehydrogenase activity of oxidizing an aldehyde group of 4-(aminomethyl)cyclohexane-1-carbaldehyde to produce 4-(aminomethyl)cyclohexane-1-carboxylic acid and consisting of an amino acid sequence having 60% or more identity to an amino acid sequence shown in any one of SEQ ID NOs. 19 to 22 and 35 to 46.

7. DNA encoding the protein according to claim 6.

8. Recombinant DNA comprising the DNA according to claim 7.

9. A recombinant cell comprising the DNA according to claim 7 or obtained by transforming a host cell with the recombinant DNA according to claim 8.

10. A method for producing cis- and/or trans- 4-(aminomethyl)cyclohexane-1-carboxylic acid from cis- and/or trans- 1,4-bis(aminomethyl)cyclohexane, the production method comprising:

   (i) a step of producing 4-(aminomethyl)cyclohexane-1-carbaldehyde from 1,4-bis(aminomethyl)cyclohexane in the presence of an aminotransferase; and
   (ii) a step of producing 4-(aminomethyl)cyclohexane-1-carboxylic acid from 4-(aminomethyl)cyclohexane-1-carbaldehyde in the presence of an aldehyde dehydrogenase.

11. The production method according to claim 10,
   wherein the aminotransferase is the protein according to claim 1, and the aldehyde dehydrogenase is the protein according to claim 6.

12. The production method according to claim 10 or 11,
   wherein, in a part or all of the method, alanine dehydrogenase and/or NADH oxidase is allowed to coexist.

13. The production method according to claim 10 or 11,
   wherein trans-4-(aminomethyl)cyclohexane-1-carboxylic acid is produced from cis- and/or trans- 1,4-bis(aminomethyl)cyclohexane.

14. The production method according to claim 10 or 11,
   wherein cis-4-(aminomethyl)cyclohexane-1-carboxylic acid is produced from cis- and/or trans- 1,4-bis(aminomethyl)cyclohexane.

15. The production method according to claim 10 or 11,
   wherein a part or all of the method is performed under neutral or basic conditions.

16. The production method according to claim 15,
   wherein a part or all of the method is performed in the presence of a secondary amine.

17. A protein which is an aldehyde dehydrogenase and has aldehyde dehydrogenase activity of oxidizing an aldehyde group of 4-(aminomethyl)cyclohexane-1-carbaldehyde to produce 4-(aminomethyl)cyclohexane-1-carboxylic acid.

18. The protein according to claim 17, wherein the protein is a benzaldehyde dehydrogenase and has aldehyde dehydrogenase activity of oxidizing an aldehyde group of 4-(aminomethyl)cyclohexane-1-carbaldehyde to produce 4-(aminomethyl)cyclohexane-1-carboxylic acid.

19. The protein according to claim 17,
   wherein the aldehyde dehydrogenase is an aldehyde dehydrogenase derived from bacteria belonging to the genus *Pseudomonas*.

20. The protein according to claim 17, wherein the protein is consisting of an amino acid sequence having 50% or more identity to an amino acid sequence shown in any one of SEQ ID NOs. 19 to 22, 35 to 46 and 127 to 139.

21. A protein having aldehyde dehydrogenase activity of producing 4-(aminomethyl)cyclohexane-1-carboxylic acid from 4-(aminomethyl)cyclohexane-1-carbaldehyde and consisting of an amino acid sequence having 50% or more identity to an amino acid sequence shown in SEQ ID NO. 20, wherein the amino acid sequence contains at least one of the following amino acid residues [1] to [26] when aligned with the amino acid sequence shown in SEQ ID NO. 20:

[1] an amino acid residue at a position corresponding to position 35 which is a glycine residue (G)
[2] an amino acid residue at a position corresponding to position 65 which is an alanine residue (A)
[3] an amino acid residue at a position corresponding to position 82 which is a leucine residue (L)
[4] an amino acid residue at a position corresponding to position 104 which is a glycine residue (G)
[5] an amino acid residue at a position corresponding to position 109 which is a glycine residue (G)
[6] an amino acid residue at a position corresponding to position 110 which is an isoleucine residue (I)
[7] an amino acid residue at a position corresponding to position 117 which is a glutamine residue (Q)
[8] an amino acid residue at a position corresponding to position 122 which is an alanine residue (A)
[9] an amino acid residue at a position corresponding to position 124 which is a leucine residue (L)
[10] an amino acid residue at a position corresponding to position 134 which is a valine residue (V)
[11] an amino acid residue at a position corresponding to position 144 which is a valine residue (V)
[12] an amino acid residue at a position corresponding to position 160 which is a phenylalanine residue (F)
[13] an amino acid residue at a position corresponding to position 271 which is a glycine residue (G)
[14] an amino acid residue at a position corresponding to position 290 which is an alanine residue (A)
[15] an amino acid residue at a position corresponding to position 319 which is an aspartic acid residue (D)
[16] an amino acid residue at a position corresponding to position 333 which is an aspartic acid residue (D)
[17] an amino acid residue at a position corresponding to position 365 which is a glutamine residue (Q)
[18] an amino acid residue at a position corresponding to position 390 which is an isoleucine residue (I)
[19] an amino acid residue at a position corresponding to position 420 which is an alanine residue (A)
[20] an amino acid residue at a position corresponding to position 423 which is a leucine residue (L)
[21] an amino acid residue at a position corresponding to position 434 which is a valine residue (V)
[22] an amino acid residue at a position corresponding to position 440 which is a proline residue (P)
[23] an amino acid residue at a position corresponding to position 443 which is a cysteine residue (C)
[24] an amino acid residue at a position corresponding to position 445 which is a proline residue (P)
[25] an amino acid residue at a position corresponding to position 466 which is a serine residue (S)
[26] an amino acid residue at a position corresponding to position 467 which is an isoleucine residue (I)

22. DNA encoding the protein according to any one of claims 5, and 17 to 21.

23. Recombinant DNA comprising the DNA according to claim 22.

24. A recombinant cell comprising the DNA according to claim 22.

25. A recombinant cell obtained by transforming a host cell with the recombinant DNA according to claim 23.

26. The production method according to claim 10,
wherein the aminotransferase is the protein according to claim 1, and the aldehyde dehydrogenase is the protein according to any one of claims 5, and 17 to 21.

27. The production method according to claim 26,
wherein, in a part or all of the method, alanine dehydrogenase and/or NADH oxidase is allowed to coexist.

28. The production method according to claim 26,
wherein trans-4-(aminomethyl)cyclohexane-1-carboxylic acid is produced from cis- and/or trans- 1,4-bis(aminomethyl)cyclohexane.

29. The production method according to claim 26,
wherein cis-4-(aminomethyl)cyclohexane-1-carboxylic acid is produced from cis- and/or trans- 1,4-bis(aminomethyl)cyclohexane.

30. The production method according to claim 26,
wherein a part or all of the method is performed under neutral or basic conditions.

**31.** The production method according to claim 26,
wherein a part or all of the method is performed in the presence of a secondary amine.

# Fig.1

NADH, NH$_3$   NAD$^+$

PYRUVIC ACID REGENERATE | ALANINE DEHYDROGENASE

PYRUVIC ACID   ALANINE

H$_2$O OR H$_2$O$_2$   O$_2$

NAD(P)$^+$ REGENERATE | NAD(P)H OXIDASE

NAD(P)$^+$   NAD(P)H

trans-1,4-bis(aminomethyl)
cyclohexane

trans-4-(aminomethyl)cyclohexane
-1-carbaldehyde

trans-4-(aminomethyl)cyclohexane
-1-carboxylic acid

AMINOTRANSFERASE   ISOMERIZATION | BASE

AMINO GROUP TRANSFER

ALDEHYDE DEHYDROGENASE

ALDEHYDE OXIDATION

cis-1,4-bis(aminomethyl)
cyclohexane

cis-4-(aminomethyl)cyclohexane
-1-carbaldehyde

cis-4-(aminomethyl)cyclohexane
-1-carboxylic acid

SUBSTRATE

INTERMEDIATE

DESIRED PRODUCT

# *Fig.2*

*Fig.3*

1:142.10(+)

trans | cis

# Fig.4

CHROMOSOME

PRIMER

patA

SYNTHETIC GENE

PRIMER

xylC

PRIMER

PRIMER

PCR

PCR

pET28a 3' END SIDE 20 bp

LINKER SEQUENCE

patA

LINKER SEQUENCE

pET28a 5' END SIDE 20 bp

xylC

PCR

LINKER SEQUENCE 20 bp

patA

xylC

PRIMER

PT7

PRIMER

pET28a

In-Fuion

patA

xylC

PT7

pET28a-patA-xylC

EP 4 541 902 A1

# Fig.5A

```
20    1  -MRETK-EQPIWYGKVFSSNWVEARGGVANVVDPSNGDILGITGVANGEDVDAAVNAAKR  58
128   1  -MRETK-EQPIWYGKVFSSNWVEARGGVANVVDPSNGDILGITGVANGEDVDAAVNAAKR  58
40    1  MQQERR-EASIWSGKVFSSGWVEARGGVCDVTDPSNGDLLGVTGIANGEDVAAAQTARR   59
130   1  MQQERKKEVPIWSGKVFSSGWVEARGGASDVTDPSNGDLLGVVGIANGEDVAAAQSARR   60
131   1  MNWNGK--LPIWHGKVFSNGWVVPRGGVADVVDPSSGDVLGVTGIANSEDIATAASAAKE  58
41    1  MNTPIK--SNAWTGKVFSHGWSAGSGGVAEVIEPATGEVLGQIGIADVKDVDAAVASARS  58
127   1  MNTPVK--TERWSGKIFTGSWVAARGGVAPVLEAATGAALGEVGVASPEDVDAAARSAQA  58
134   1  MNTSTV--TNIWDSKIYSGGWIAAFGGTAAIIEPATEQVLGHIGIGATQDVDRAVQIASH  58
135   1  MTGTGS--EGQWTGKIFLGEWVKGTGATAPVMEPATDNQISTIALASREDVDTAIERAVV  58

                *                        *                     *    **      *
20   59  AQKEWAAIPFSERAAIVRKAAEKLKEREHEFADWNVRECGAIRPKGLWEAGIAYEQMHQA 118
128  59  AQKEWAAIPFSERAAIVRKAAEKLKEREHEFADWNVRECGAIRPKGLWEAGIAYEQMHQA 118
40   60  AQKGWAAIPFSERAEIVRKAAEKLKERENEFADWNVRECGAIRQKGLWEAGITYQQMHQA 119
130  61  AQKEWAAIPFSERAEIVRKAAEKLKERENEFADWNVRECGAIRQKGLWEAGITYQQMHQA 120
131  59  AQKKWAAMPFNDRAAILRKAAEKLREREAEFADWNVRECGAVRMKGVWEAGITYEQMHQA 118
41   59  AQEAWAATPFDQRAAIIRTAAEVLKRRAAEFVQWNTRECGSIPPKGEYEVGITYEQLQQA 118
127  59  AQKAWAATPFDQRAAIVREAARLLKERAAEFTQWNVRECGSIVPKGEWEVGITHEQMQQA 118
134  59  AQRSWARMPFDQRANIMREAARLVLERAAEFNVWNIRQCGSIPAKAEWELHATCEQILMA 118
135  59  AQRAWAETTFDKRAAVLHEAARLIKARAERFNYWNVRECGSIVPKAQWELDASYEQLLMA 118

              *  *          *            *                  *
20  119  AGLAYLSNGTLFPSAVPGRMNLCQRVPVGVVGVIAPWNFPLFLAMRSVAPALALGNAVIL 178
128 119  AGLAYLSNGTLFPSAVPGRMNLCQRVPVGVVGVIAPWNFPLFLAMRSVAPALALGNAVIL 178
40  120  ASLAFLPDGTSFPSAVAGRLNLCQRVPVGVVGVIAPWNFPLFLSMRSVAPALVLGNAVML 179
130 121  ASLAFLPDGTSLPSTVAGRLNLCHRVPVGVVGVIAPWNFPLFLAMRSVAPALVLGNAVIL 180
131 119  ASLAFLPNGTLFPSQVPGRMNFVHRVPVGVVGVIAPWNFPLFLAMRSVAPALVLGNAVIL 178
41  119  AALAALPNGSLFPSSVPGRTNLWQRVPLGVVGVIAPWNFPLFLAMRSVAPALSLGNAVML 178
127 119  AALAGLPNGMMFPSSVPGRTNLWQRVPLGVVGVIAPWNFPLFLAMRSVAPALALGNAVLL 178
134 119  AALPMQPEGLLFPSSMPGRTNLSRQLPIGVIGVITPWNFPLLLAMRSVAPALALGNAVIL 178
135 119  AALPMHPTGTLFPSSMPGRTNTWRRLPIGVVGVIAPWNFPLLLALRSVAPATALGNAVML 178

20  179  KPDLQTAVTGGALIAEIFSDAGMPDGVLHVLPGGADVGESMVANSGINMISFTGSTQVGR 238
128 179  KPDLQTAVTGGALIAEIFSDAGMPDGVLHVLPGGADVGESMVANSGINMISFTGSTQVGR 238
40  180  KPDPQTAITGGALIAEIFSEAGLPDGVLHVLPGGAEAGESMVASPEIDMISFTGSTQVGR 239
130 181  KPDPQTAVTGGALIAEIFSEAGIPDGVIHVLPGGAEAGESMVTNPGIDMISFTGSTEVGR 240
131 179  KPDVQTAVTGGALIAEIFAEAGIPEGVLHVLPGGADAGDAMVTNPEIDMISFTGSTAVGR 238
41  179  KPDLQSAVTGGVLIAEIFEEAGLPDGVLHVLPGGAETGEALVRHPDVAMISFTGSTAVGR 238
127 179  KPDLQSAVTGGALIARLFEDVGLPPGVLQVLPGGPATGDAVVRHPCVNMVSFTGSTAVGR 238
134 179  KPDQQSAICGGALIAQAFEDAGLPVGVLHVIPGGPATGDALVKHPEVGMISFTGSTAVGR 238
135 179  KPDQQSAVTGGALIAELFEDAGLPRGVLQVLPGGAQTGDAVVRHPAVGMISFTGSTAVGR 238

                                     *                          *
20  239  LIGEKCGRMLKKVALELGGNNVHIVLPDADLDGAVSCAAWGTFLHQGQVCMAAGRHLVHR 298
128 239  LIGEKCGRMLKKVALELGGNNVHIVLPDADLDGAVSCAAWGTFLHQGQVCMAAGRHLVHR 298
40  240  LIGEKCGGMLKKVALELGGNNVHIVLPDADLDGAASCAAWGTFLHQGQVCMAAGRHLVHR 299
130 241  LIGEKCGRMLKKVALELGGNNVHIILPDADLDGAASCAAWGTFLHQGQVCMAAGRHLVHR 300
131 239  QIGEKCGRMLKKVTLELGGNNVHIVLPDADLTGAASCAAWGTFLHQGQVCMAAGRHLVHR 298
41  239  SIAEICGRMLKKVALELGGNNVHIVLPDADLDGAASCAAWGSFLHQGQVCMAAGRHLVHR 298
127 239  QIGEFCGRALKKVALELGGNNAMIVLDDADLDGASSCAAWGGFLHQGQICMAAGRHLVHR 298
134 239  AIGQTCGYILKKVALELGGNNAIIVLDDADMDAASSNGAWGAFLHQGQICMQTGRHLVHH 298
135 239  QIGETCGRMLKKTSLELGGNPIIVLPDADLDKAASCAAWGAFLHQGQICMQAGRHLVHR 298
```

# Fig.5B

```
20  299 DVAQQYAEKLALRAKNLVVGDPNSDRVHLGPLINDKQVVRVHALVESAQSAGAKVLAGGT 358
128 299 DVAQQYAEKLALRAKNLVVGDPNSDRVHLGPLINDKQVVRVHALVESAQSAGAKVLAGGT 358
40  300 DIAQEYAEKLALRARNLVVGDPKTDQVHLGPLINDKQVAHVHALVESAQRAGAKILAGGA 359
130 301 DIAQEYAEKLALRARNLVVGDPKTDQVHLGPLVNDKQVAHVHALVESAQRAGAKILAGGT 360
131 299 DVAQQYAQELAARAKNLVVGDPKTDQVHLGPLINDKQVPRVHALVESAQKQGAKVLVGGT 358
41  299 SVVDEYAAKLATRAGALHVGDPNAGPCHLGPLINDRQVPRVHEVVTGSIEAGAEVLTGGV 358
127 299 SVADAYAAKLVERAKKLYVGDPHAGPAHLGPLINDRQTERVHRIVTESIAAGATLLAGGT 358
134 299 SVADQYAEKLAARAEKLHVGNPHTDQVHLGPLINRKQCDRIHGIVQETLRAGAHALTGAT 358
135 299 SVAESYVAKLAARARKLAVGNPATEPVHIGPLINARQAERVERIVRDSVEMGAVIAAGGQ 358

20  359 YQDRYYQATVIMDVKPEMEVFKSEIFGPVAPITVFDSIEEAIELANCSEYGLAASIHTRA 418
128 359 YQDRYYQATVIMDVKPEMEVFKSEIFGPVAPITVFDSIEEAIELANCSEYGLAASIHTRA 418
40  360 YQDRYYQATVLMDVTPKMDVFKLETFGPVAPITVVDSVEEAIELANSSEYGLAASIHTKS 419
130 361 YQDRYYQATVLMDVTPKMDIFELPTFGPVAPITLVDSVEEAIGLANSSEYGLAASIHTKD 420
131 359 HQGRFYQPTVLMDVRPDMDVFKSEIFGPVAPITVFDTIDEAIELANSSEYGLAASIHTSA 418
41  359 HEGRFYQPTVLKGVTPEMSAFKSEIFGPVAPITVFDTEDEAIALSNRSEYGLAASIHTAS 418
127 359 HEGRFYQPTVLSNVAVDMPAFAEEIFGPVAPITVFDTDEEAVALANSSEYGLAAAIHSKS 418
134 359 HERLFYRPTVLTGVTPDMAAFQEELFGPVAPVTVFHDDDEAVELVNRSAYGLCAAIHSRN 418
135 359 RRGRFFEPTVLADVTPHMPAFKDEIFGPVAPVTLFDSVDEAIELVHASEYGLAAAVHSGS 418

20  419 LATGLDIAKRLNTGMVHINDQPINCEPHVPFGGMGASGSGGRFGGPASIEFTQSQWISM 478
128 419 LATGLDIAKRLNTGMVHINDQPINCEPHVPFGGMGSSGSGGRFGGLASIDEFTQSQWISM 478
40  420 LATGLEIARNLKTGMVHINDQPINCEPHVPFGGMGASGSSGRFGGLASIDEFTQSQWISM 479
130 421 LAAGLEIARNLKTGMVHINDQPINCEPHVPFGGMGASGSSGRFGGLASIDAFTQSQWISM 480
131 419 IATALQIAKRLKTGMVHINDQPINCEPHVPFGGMGASGTSGRFGGLSSIDEFTQSQWISM 478
41  419 AARGLALAKRLKTGMVHINDQPINCEPXVPFGGMGASGSGGRFGGPASIDEFTQAQWISL 478
127 419 TARALRMASQLKSGMVHINDQEVNCEPQVPFGGMGASGSGGRFGGPASIDEFTQAQWVSV 478
134 419 IPRAVALSQRLNTGMIHINDQTVNNEFHVPFGGMGSSGNGGRFGGPANVHEFTQSQWLSI 478
135 419 MSAAMSVRRRLKSGMIHINDQTVNNEFQVPFGGMGASGNGGRFGGPANVEEFTQTQWISA 478

20  479 VEKPANYPF                                                   487
128 479 VEKSASYPF                                                   487
40  480 VEKPSIYPF                                                   488
130 481 VGKPATYPF                                                   489
131 479 VEKPTNYPF                                                   487
41  479 VEKPVIYPF                                                   487
127 479 SEKAAQYPF                                                   487
134 479 MEKPVIYPF                                                   487
135 479 MDAPIEYPF                                                   487
```

49

## INTERNATIONAL SEARCH REPORT

International application No.

**PCT/JP2023/024236**

**A. CLASSIFICATION OF SUBJECT MATTER**

*C12P 13/00*(2006.01)i; *C12N 5/10*(2006.01)i; *C12N 1/15*(2006.01)i; *C12N 1/19*(2006.01)i; *C12N 1/21*(2006.01)i;
*C12N 15/53*(2006.01)i; *C12N 15/54*(2006.01)i; *C12N 9/10*(2006.01)i
FI: C12N15/54; C12N15/53; C12N1/15; C12N1/19; C12N1/21; C12N5/10; C12P13/00; C12N9/10 ZNA

According to International Patent Classification (IPC) or to both national classification and IPC

**B. FIELDS SEARCHED**

Minimum documentation searched (classification system followed by classification symbols)

C12P13/00; C12N5/10; C12N1/15; C12N1/19; C12N1/21; C12N15/53; C12N15/54; C12N9/10

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

JSTPlus/JMEDPlus/JST7580 (JDreamIII); CAplus/MEDLINE/EMBASE/BIOSIS (STN); SwissProt/GeneSeq

**C. DOCUMENTS CONSIDERED TO BE RELEVANT**

| Category* | Citation of document, with indication, where appropriate, of the relevant passages | Relevant to claim No. |
|---|---|---|
| X | WO 2018/190398 A1 (KYOUWA HAKKO BIO CO., LTD.) 18 October 2018 (2018-10-18) claims, SEQ ID NO. 2 | 1-4 |
| A | | 5-31 |
| X | LI, S. et al, 'aminotransferase [Pseudomonas putida ND6]', GenBank [online], 30 January 2014, Accession No. AFK66983.1, [retrieved on 05 September 2023], Internet: <https://www.ncbi.nlm.nih.gov/protein/388557842> entire text | 1-4 |
| A | | 5-31 |
| X | UHRYNOWSKI, W. et al., 'Omega-amino acid--pyruvate aminotransferase [Aeromonas salmonicida]', GenBank [online], 08 June 2017, Accession No. ARW84612.1, [retrieved on 05 September 2023], Internet: <https://www.ncbi.nlm.nih.gov/protein/arw84612.1> entire text | 1-4 |
| A | | 5-31 |

☑ Further documents are listed in the continuation of Box C.  ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **08 September 2023** | **19 September 2023** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

| | INTERNATIONAL SEARCH REPORT | International application No. |
|---|---|---|
| | | **PCT/JP2023/024236** |

**C.      DOCUMENTS CONSIDERED TO BE RELEVANT**

| Category* | Citation of document, with indication, where appropriate, of the relevant passages | Relevant to claim No. |
|---|---|---|
| X | ASHTON, P. M. et al., 'putrescine aminotransferase [Shigella boydii]', GenBank [online], 02 September 2020, Accession No. EGE3747071.1, [retrieved on 05 September 2023], Internet: <https://www.ncbi.nlm.nih.gov/protein/ege3747071.1> entire text | 1-4 |
| A | | 5-31 |
| X | DURFEE, T. et al., 'medium chain aldehyde dehydrogenase [Escherichia coli str. K-12 substr. DH10B]', GenBank [online], 31 January 2014, Accession No. ACB02660.1, [retrieved on 05 September 2023], Internet: <https://www.ncbi.nlm.nih.gov/protein/acb02660.1> entire text | 5-9, 17-20, 22-25 |
| A | | 1-4, 10-16, 21, 26-31 |
| X | MARTINEZ, J. M. et al., 'Benzaldehyde dehydrogenase [NAD(+)] [Stutzerimonas stutzeri]', GenBank [online], 18 August 2020, Accession No. CAD2266443.1, [retrieved on 05 September 2023], Internet: <https://www.ncbi.nlm.nih.gov/protein/cad2266443.1> entire text | 5-9, 17-25 |
| A | | 1-4, 10-16, 26-31 |
| X | VELASCO, A. et al., 'phenylacetaldehyde dehydrogenase [Pseudomonas sp. Y2]', GenBank [online], 15 April 2005, Accession No. CAA04003.1, [retrieved on 05 September 2023], Internet: <https://www.ncbi.nlm.nih.gov/protein/caa04003.1> entire text | 5-9, 17-20, 22-25 |
| A | | 1-4, 10-16, 21, 26-31 |
| X | CRONIN, C. N., 'p-hydroxybenzaldehyde dehydrogenase (plasmid) [Pseudomonas putida]', GenBank [online], 26 July 2016, Accession No. AAA75634.2, [retrieved on 05 September 2023], Internet: <https://www.ncbi.nlm.nih.gov/protein/aaa75634.2> entire text | 5-9, 17-20, 22-25 |
| A | | 1-4, 10-16, 21, 26-31 |
| A | JP 63-152991 A (IDEMITSU KOSAN CO., LTD.) 25 June 1988 (1988-06-25) entire text | 1-31 |
| A | CN 103131649 A (ZHEJIANG UNIVERSITY OF TECHNOLOGY) 05 June 2013 (2013-06-05) entire text | 1-31 |
| A | CN 103114122 A (ZHEJIANG UNIVERSITY OF TECHNOLOGY) 22 May 2013 (2013-05-22) entire text | 1-31 |
| A | JP 55-076846 A (TAKEDA PHARMACEUTICAL CO., LTD.) 10 June 1980 (1980-06-10) entire text | 1-31 |

Form PCT/ISA/210 (second sheet) (January 2015)

## INTERNATIONAL SEARCH REPORT

International application No.

**PCT/JP2023/024236**

| Box No. I | Nucleotide and/or amino acid sequence(s) (Continuation of item 1.c of the first sheet) |
|---|---|

1. With regard to any nucleotide and/or amino acid sequence disclosed in the international application, the international search was carried out on the basis of a sequence listing:

    a. ☑ forming part of the international application as filed:

        ☑ in the form of an Annex C/ST.25 text file.

        ☐ on paper or in the form of an image file.

    b. ☐ furnished together with the international application under PCT Rule 13*ter*.1(a) for the purposes of international search only in the form of an Annex C/ST.25 text file.

    c. ☐ furnished subsequent to the international filing date for the purposes of international search only:

        ☐ in the form of an Annex C/ST.25 text file (Rule 13*ter*.1(a)).

        ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

    "In the form of an Annex C/ST.25 text file" above should be understood as "in ST.26 format".

Form PCT/ISA/210 (continuation of first sheet) (January 2015)

INTERNATIONAL SEARCH REPORT
Information on patent family members

International application No.

**PCT/JP2023/024236**

| Patent document cited in search report | | | Publication date (day/month/year) | Patent family member(s) | | | Publication date (day/month/year) |
|---|---|---|---|---|---|---|---|
| WO | 2018/190398 | A1 | 18 October 2018 | US | 2020/0131546 | A1 | |
| | | | | claims, SEQ ID NO. 2 | | | |
| | | | | EP | 3611253 | A1 | |
| JP | 63-152991 | A | 25 June 1988 | (Family: none) | | | |
| CN | 103131649 | A | 05 June 2013 | (Family: none) | | | |
| CN | 103114122 | A | 22 May 2013 | (Family: none) | | | |
| JP | 55-076846 | A | 10 June 1980 | (Family: none) | | | |

Form PCT/ISA/210 (patent family annex) (January 2015)

53

## REFERENCES CITED IN THE DESCRIPTION

**Patent documents cited in the description**

- CN 114014768 A
- JP S63152991 A
- JP S58110600 A
- WO 9812343 A
- JP 63233798 A
- JP 57134500 A
- JP S5835197 A
- JP S57183799 A
- JP S57134500 A
- JP S58105999 A
- JP S63248394 A

**Non-patent literature cited in the description**

- *Keio Journal of Medicine*, 1962, vol. 11 (8), 105-115
- *Keio Journal of Medicine*, 1964, vol. 18 (4), 177-185
- *European Journal of Haematology*, 2020, vol. 104 (2), 79-87
- *Journal of Trauma and Acute Care Surgery*, 2019, vol. 86 (1), 101-107
- *Pro. Nat. Acad. Sci. USA*, 1993, vol. 90, 5873
- *Methods Enzymol.*, 1990, vol. 183, 63
- **KARLIN** ; **ALTSCHUL**. Based on this algorithm BLAST, programs called BLASTN and BLASTX have been developed. *J. Mol. Biol.*, 1990, vol. 215, 403
- Molecular Cloning. Cold Spring Harbor Laboratory Press, 2012
- Methods for General and Molecular Bacteriology. ASM Press, 1994
- Immunology methods manual. Academic pres, 1997
- *Agric. Biol. Chem.*, 1984, vol. 48, 669
- *Agric. Biol. Chem.*, 1989, vol. 53, 277
- *Proc.Natl. Acad. Sci.*, 1985, vol. 82, 4306
- *APPLIED AND ENVIRONMENTAL MICROBIOL-OGY*, 2007, vol. 73 (20), 6378-6385
- *Appl. Environ. Microbiol.*, 2007, vol. 73, 6378-6385
- *Gene*, 1985, vol. 33, 103
- *Molecular and General Genetics*, 1984, vol. 196, 175
- *Appl. Microbiol. Biotechnol.*, 2000, vol. 53, 674-679
- *Proc. Natl. Acad. Sci.*, 1972, vol. 69, 2110
- *Nucleic Acids Res.*, 1988, vol. 16, 6127
- *Proc. Natl. Acad. Sci.*, 1984, vol. 81, 4889
- *J. Bacteriol.*, 1983, vol. 153, 163
- *Proc. Natl. Acad. Sci. USA*, 2000, vol. 97, 6641-6645
- *Mol. Microbiol.*, 2005, vol. 55, 137
- *Biosci. Biotechnol.Biochem.*, 2007, vol. 71, 2905
- *Applied Microbiology and Biotechnology*, 2020, vol. 104, 7745-7766
- *Journal of Bacteriology*, 2012, vol. 194 (15), 4080-4088
- *FEBS Letters*, 2005, vol. 579, 4107-4112
- *Arch.Microbiol.*, 2011, vol. 193, 553-563
- *Archives of Biochemistry and Biophysics*, 2017, vol. 616, 47-58
- *Appl. Microbiol. Biotechnol.*, 2014, vol. 98, 1349-1356
- *Appl. Microbiol. Biotechnol*, 2014, vol. 98, 1349-1356
- *Appl Environ Microbiol*, 2013, vol. 79, 3033-3039
- **DATSENKO, K. A.** ; **WARNER, B. L.** *Proc. Natl. Acad. Sci.*, 2000, vol. 97, 6640-6645